(19) Europäisches Patentamt
European Patent Office
Office européen des brevets

(11) EP 1 167 524 B1

(12) **EUROPEAN PATENT SPECIFICATION**

(45) Date of publication and mention of the grant of the patent:
**16.05.2007 Bulletin 2007/20**

(21) Application number: **00908056.5**

(22) Date of filing: **14.03.2000**

(51) Int Cl.:
***C12N 15/11*** *(2006.01)* ***C12Q 1/68*** *(2006.01)*

(86) International application number:
**PCT/JP2000/001534**

(87) International publication number:
**WO 2000/056877 (28.09.2000 Gazette 2000/39)**

(54) **Method for amplifying a nucleic acid sequence employing a chimeric primer**

Verfahren zur Amplifizierung einer Nukleinsäuresequenz unter Verwendung eines chimären Primers

Procédé d'amplification d'une acide nucléique utilisant une oligonucléotide chimère

(84) Designated Contracting States:
**AT BE CH CY DE DK ES FI FR GB GR IE IT LI LU MC NL PT SE**

(30) Priority: **19.03.1999 JP 7696699**
**27.12.1999 JP 37003599**

(43) Date of publication of application:
**02.01.2002 Bulletin 2002/01**

(73) Proprietor: **TAKARA BIO INC.**
**Otsu-shi,**
**Shiga (JP)**

(72) Inventors:
- **MUKAI, Hiroyuki**
  **Moriyama-shi, Shiga 524-0102 (JP)**
- **YAMAMOTO, Junko**
  **Moriyama-shi, Shiga 524-0044 (JP)**
- **TAKEDA, Osamu**
  **Hikone-shi, Shiga 521-1124 (JP)**
- **MIYAKE, Kazue**
  **Uji-shi, Kyoto 611-0011 (JP)**
- **UEMORI, Takashi**
  **Otsu-shi, Shiga 520-2141 (JP)**
- **SATO, Yoshimi**
  **Kurita-gun, Shiga 520-3031 (JP)**
- **MORIYAMA, Mariko**
  **Otsu-shi, Shiga 520-2133 (JP)**
- **SAWARAGI, Haruhisa**
  **Otsu-shi, Shiga 520-2133 (JP)**
- **HAGIYA, Michio**
  **Otsu-shi, Shiga 520-0226 (JP)**
- **ASADA, Kiyozo**
  **Koka-gun, Shiga 520-3333 (JP)**
- **KATO, Ikunoshin**
  **Uji-shi, Kyoto 611-0028 (JP)**

(74) Representative: **Schnappauf, Georg et al**
**Dr. Volker Vossius**
**Patent- und Rechtsanwaltskanzlei**
**Geibelstrasse 6**
**81679 München (DE)**

(56) References cited:
**EP-A- 0 747 479**
**WO-A-99/50542**
**WO-A1-99/09211**
**JP-A- 7 289 298**
**US-A- 5 824 517**

- **WALDER ROXANNE Y ET AL: "Use of PCR primers containing a 3'-terminal ribose residue to prevent cross-contamination of amplified sequences" NUCLEIC ACIDS RESEARCH, vol. 21, no. 18, 1993, pages 4339-4343, XP001204573 ISSN: 0305-1048**
- **CAIRNS M J ET AL: "Nucleic acid mutation analysis using catalytic DNA" NUCLEIC ACIDS RESEARCH, OXFORD UNIVERSITY PRESS, SURREY, GB, vol. 28, no. 3, 1 February 2000 (2000-02-01), pages E9-1, XP002233619 ISSN: 0305-1048**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 167 524 B1

SEQUENCE LISTING

<110> Takara Shuzo Co., Ltd.

<120> Method for amplifying nucleic acid sequence

<130> 38-39

<140> EP 00908056.5
<141> 2000-03-14

<150> JP 11/76966
<151> 1999-03-19

<150> JP 11/370035
<151> 1999-12-27

<160> 68

<210> 1
<211> 99
<212> DNA
<213> Artificial Sequence

<220>
<223> Synthetic DNA corresponding to a portion of human transferrin receptor-encoding sequence used as a template

<400> 1

**ggacagcaac tgggccagca aagttgagaa actcacttta gagaattctg ctttcccttt 60**
**ccttgcatat tctgagcagt ttctttctgt ttttgcgag 99**

<210> 2
<211> 22
<212> DNA
<213> Artificial Sequence

<220>
<223> Designed oligonucleotide primer to amplify a portion of human transferrin receptor-encoding sequence

<400> 2
cagcaactgg gccagcaaag tt 22

<210> 3
<211> 22
<212> DNA
<213> Artificial Sequence

<220>
<223> Designed oligonucleotide primer to amplify a portion of human transferrin receptor-encoding sequence

<400> 3
gcaaaaacag aaagaaactg ct 22

<210> 4
<211> 26
<212> DNA
<213> Artificial Sequence

<220>
<223> Designed oligonucleotide used as a probe for detecting an amplified portion of human transferrin receptor-encoding sequence

<400> 4
tgctttccct ttccttgcat attctg 26

<210> 5
<211> 25
<212> DNA
<213> Artificial Sequence

<220>
<223> Designed oligonucleotide primer designated as pUC19 upper(2)NN to amplify a portion of plasmid pUC19

<400> 5
attgcttaat cagtgaggca cctat 25

<210> 6
<211> 25
<212> DNA
<213> Artificial Sequence

<220>
<223> Designed oligonucleotide primer designated as pUC19 lower NN to amplify a portion of plasmid pUC19

<400> 6
gataacactg cggccaactt acttc 25

<210> 7
<211> 25
<212> DNA
<213> Artificial Sequence

<220>
<223> Designed oligonucleotide primer to amplify a portion of plasmid pUC19

<400> 7
actggcgaac tacttactct agctt 25

<210> 8
<211> 26

<212> DNA
<213> Artificial Sequence

<220>
<223> Designed oligonucleotide primer designated as pUC19 lower 542 to amplify a portion of plasmid pUC19

<400> 8
agtcaccaga aaagcatctt acggat 26

<210> 9
<211> 25
<212> DNA
<213> Artificial Sequence

<220>
<223> Designed oligonucleotide primer to amplify a portion of plasmid pUCl9

<400> 9
gctcatgaga caataaccct gataa 25

<210> 10
<211> 25
<212> DNA
<213> Artificial Sequence

<220>
<223> Designed oligonucleotide primer designated as pUC19 upper 150 to amplify a portion of plasmid pUC19

<400> 10
ggtgtcacgc tcgtcgtttg gtatg 25

<210> 11
<211> 25
<212> DNA
<213> Artificial Sequence

<220>
<223> Designed oligonucleotide primer designated as pUC19 upper 249 to amplify a portion of plasmid pUC19

<400> 11
cgcctccatc cagtctatta attgt 25

<210> 12
<211> 22
<212> DNA
<213> Artificial Sequence

<220>
<223> Designed oligonucleotide primer to amplify a portion of human transferrin receptor-encoding sequence

<400> 12
ctgattgaga ggattcctga gt 22

<210> 13
<211> 22
<212> DNA
<213> Artificial Sequence

<220>
<223> Designed oligonucleotide primer to amplify a portion of human transferrin receptor-encoding sequence

<400> 13
tagggagaga ggaagtgata ct 22

<210> 14
<211> 22
<212> DNA
<213> Artificial Sequence

<220>
<223> Designed oligonucleotide primer to amplify a portion of human transferrin receptor-encoding sequence

<400> 14
caacttcaag gtttctgcca gc 22

<210> 15
<211> 21
<212> DNA
<213> Artificial Sequence

<220>
<223> Designed oligonucleotide primer to amplify a portion of human transferrin receptor-encoding sequence

<400> 15
aatagtccaa gtagctagag c 21

<210> 16
<211> 24
<212> DNA
<213> Artificial Sequence

<220>
<223> Designed oligonucleotide primer designated as MF2N3(24) to amplify a portion of plasmid pUC19-249 or plasmid pUC19-911

<400> 16
gctgcaaggc gattaagttg ggta 24

<210> 17
<211> 24
<212> DNA
<213> Artificial Sequence

<220>
<223> Designed oligonucleotide primer designated as MR1N3(24) to amplify a portion of plasmid pUC19-249 or plasmid pUC19-911

<400> 17
ctttatgctt ccggctcgta tgtt 24

<210> 18
<211> 30
<212> DNA
<213> Artificial Sequence

<220>

<223> Designed oligonucleotide primer to amplify a portion of plasmid pUC19

<400> 18
ggatgtgctg caaggcgatt aagttgggta 30

<210> 19
<211> 30
<212> DNA
<213> Artificial Sequence

<220>
<223> Designed oligonucleotide primer designated as MR1N3 to amplify a portion of plasmid pUC19

<400> 19
tttacacttt atgcttccgg ctcgtatgtt 30

<210> 20
<211> 30
<212> RNA
<213> Artificial Sequence

<220>
<223> Synthetic RNA used as a probe for detecting an amplified portion of plasmid pUC19

<400> 20
ugauccccca uguugugcaa aaaagcgguu 30

<210> 21
<211> 18
<212> DNA
<213> Artificial Sequence

<220>
<223> Designed oligonucleotide primer to amplify a portion of vero toxin 1-encoding sequence from hemorrhagic Escherichia coli O-157

<400> 21
agttaatgtg gtggcgaa 18

<210> 22
<211> 17
<212> DNA
<213> Artificial Sequence

<220>
<223> Designed oligonucleotide primer to amplify a portion of vero toxin 1-encoding sequence from hemorrhagic Escherichia coli O-157

<400> 22
gactcttcca tctgcca 17

<210> 23
<211> 18
<212> DNA
<213> Artificial Sequence

<220>
<223> Designed oligonucleotide primer to amplify a portion of vero toxin 2-encoding sequence from hemorrhagic Escherichia coli O-157

<400> 23
ttcggtatcc tattcccg 18

<210> 24
<211> 18
<212> DNA
<213> Artificial Sequence

<220>
<223> Designed oligonucleotide primer to amplify a portion of vero toxin 2-encoding sequence from hemorrhagic Escherichia coli O-157

<400> 24
tctctggtca ttgtatta 18

<210> 25
<211> 22
<212> DNA
<213> Artificial Sequence

<220>
<223> Designed oligonucleotide primer designated as MCR-F to amplify a long DNA fragment

<400> 25
ccattcaggc tgcgcaactg tt 22

<210> 26
<211> 22
<212> DNA
<213> Artificial Sequence

<220>
<223> Designed oligonucleotide primer designated as MCR-R to amplify a long DNA fragment

<400> 26
tggcacgaca ggtttcccga ct 22

<210> 27
<211> 24
<212> DNA
<213> Artificial Sequence

<220>
<223> Designed oligonucleotide primer designated as MF2N3(24) to amplify a long DNA fragment

<400> 27
gctgcaaggc gattaagttg ggta 24

<210> 28
<211> 24
<212> DNA
<213> Artificial Sequence

<220>
<223> Designed oligonucleotide primer designated as MR1N3(24) to amplify a long DNA fragment

<400> 28
ctttatgctt ccggctcgta tgtt 24

<210> 29
<211> 20
<212> DNA
<213> Artificial Sequence

<220>
<223> Designed oligonucleotide primer to amplify a portion of bacteriophage lambda DNA

<400> 29
aacaacaaga aactggtttc 20

<210> 30
<211> 20
<212> DNA
<213> Artificial Sequence

<220>
<223> Designed oligonucleotide primer to amplify a portion of bacteriophage lambda DNA

<400> 30
gcaatgcatg acgactgggg 20

<210> 31
<211> 17
<212> DNA
<213> Artificial Sequence

<220>
<223> Designed oligonucleotide primer to amplify a portion of bacteriophage lambda DNA

<400> 31
gttttcccag tcacgac 17

<210> 32
<211> 17
<212> DNA
<213> Artificial Sequence

<220>
<223> Designed oligonucleotide primer to amplify a portion of bacteriophage lambda DNA

<400> 32
caggaaacag ctatgac 17

<210> 33
<211> 20
<212> DNA
<213> Artificial Sequence

<220>
<223> Designed oligonucleotide primer to amplify a portion of bacteriophage lambda DNA

<400> 33
gtacggtcat catctgacac 20

<210> 34
<211> 20
<212> DNA
<213> Artificial Sequence

<220>
<223> Designed oligonucleotide primer to amplify a portion of bacteriophage lambda DNA

<400> 34
gcaatcggca tgttaaacgc 20

<210> 35
<211> 20
<212> DNA
<213> Artificial Sequence

<220>
<223> Designed oligonucleotide primer to amplify a portion of bacteriophage lambda DNA

<400> 35
cgccatcctg ggaagactcc 20

<210> 36
<211> 44
<212> DNA
<213> Artificial Sequence

<220>
<223> Designed oligonucleotide primer designated as R1-S1 to amplify a portion of bacteriophage lambda DNA

<400> 36
tttcacacag gaaacagcta tgacaacaac aagaaactgg tttc 44

<210> 37
<211> 44
<212> DNA
<213> Artificial Sequence

<220>
<223> Designed oligonucleotide primer designated as R1-A3 to amplify a portion of bacteriophage lambda DNA

<400> 37
tttcacacag gaaacagcta tgacgcaatg catgacgact gggg 44

<210> 38
<211> 62
<212> DNA
<213> Artificial Sequence

<220>
<223> Designed oligonucleotide primer designated as R2-S1 to amplify a portion of bacteriophage lambda DNA

<400> 38

**attgtgagcg gataacaatt tcacacagga aacagctatg acaacaacaa gaaactggtt 60**
**tc 62**

<210> 39
<211> 62
<212> DNA
<213> Artificial Sequence

<220>
<223> Designed oligonucleotide primer designated as R2-A3 to amplify a portion of bacteriophage lambda DNA

<400> 39

**attgtgagcg gataacaatt tcacacagga aacagctatg acgcaatgca tgacgactgg 60**
**gg 62**

<210> 40
<211> 95
<212> DNA
<213> Artificial Sequence

<220>
<223> Designed oligonucleotide primer designated as R3-S1 to amplify a portion of bacteriophage lambda DNA

<400> 40

**cactttatgc ttccggctcg tatgttgtgt ggaattgtga gcggataaca atttcacaca 60**
**ggaaacagct atgacaacaa caagaaactg gtttc 95**

<210> 41
<211> 95
<212> DNA
<213> Artificial Sequence

<220>
<223> Designed oligonucleotide primer designated as R3-A3 to amplify a portion of bacteriophage lambda DNA

<400> 41

**cactttatgc ttccggctcg tatgttgtgt ggaattgtga gcggataaca atttcacaca 60**
**ggaaacagct atgacgcaat gcatgacgac tgggg 95**

<210> 42
<211> 17
<212> DNA
<213> Artificial Sequence

<220>
<223> Designed oligonucleotide primer designated as M13RV-2N 17mer

<400> 42
caggaaacag ctatgac 17

<210> 43
<211> 20
<212> DNA
<213> Artificial Sequence

<220>
<223> Designed oligonucleotide primer designated as M13RV-2N 20mer

<400> 43
acacaggaaa cagctatgac 20

<210> 44
<211> 70
<212> DNA
<213> Artificial Sequence

<220>
<223> Designed oligonucleotide primer to amplify a portion of CDC2-related protein kinase PISSLRE gene

<400> 44

**gagttcgtgt ccgtacaact atttcacaca ggaaacagct atgacccaac aagagcctat 60**
**agcttcgctc 70**

<210> 45
<211> 67
<212> DNA
<213> Artificial Sequence

<220>
<223> Designed oligonucleotide primer to amplify a portion of CDC2-related protein kinase PISSLRE gene

<400> 45

**tcgaaatcag ccacagcgcc atttcacaca ggaaacagct atgacccgct gtctttgagt 60**
**tgtggtg 67**

<210> 46
<211> 70
<212> DNA
<213> Artificial Sequence

<220>
<223> Designed oligonucleotide primer to amplify a portion of Type II cytoskeltal 11 keratin gene

<400> 46

**gagttcgtgt ccgtacaact atttcacaca ggaaacagct atgacgctat tctgacatca 60**
**ctttccagac 70**

<210> 47
<211> 66
<212> DNA
<213> Artificial Sequence

<220>
<223> Designed oligonucleotide primer to amplify a portion of Type II cytoskeltal 11 keratin gene

<400> 47

tcgaaatcag ccacagcgcc atttcacaca ggaaacagct atgacgaatt ccactggtgg 60
cagtag 66

<210> 48
<211> 62
<212> DNA
<213> Artificial Sequence

<220>
<223> Designed oligonucleotide primer to amplify a portion of bacteriophage lambda DNA

<400> 48

attgtgagcg gataacaatt tcacacagga aacagctatg acgtacggtc atcatctgac 60
ac 62

<210> 49
<211> 62
<212> DNA
<213> Artificial Sequence

<220>
<223> Designed oligonucleotide primer to amplify a portion of bacteriophage lambda DNA

<400> 49

attgtgagcg gataacaatt tcacacagga aacagctatg acatgcgccg cctgaaccac 60
ca 62

<210> 50
<211> 62
<212> DNA
<213> Artificial Sequence

<220>
<223> Designed oligonucleotide primer to amplify a portion of bacteriophage lambda DNA

<400> 50

attgtgagcg gataacaatt tcacacagga aacagctatg acctgctctg ccgcttcacg 60
ca 62

<210> 51
<211> 62
<212> DNA
<213> Artificial Sequence

<220>
<223> Designed oligonucleotide primer to amplify a portion of bacteriophage lambda DNA

<400> 51

```
attgtgagcg gataacaatt tcacacagga aacagctatg acgcaatcgg catgttaaac      60
gg                                                                     62
```

<210> 52
<211> 69
<212> DNA
<213> Artificial Sequence

<220>
<223> Designed oligonucleotide primer designated as 5'ID to amplify a portion of cyclin A DNA

<400> 52

```
tcgaaatcag ccacagcgcc atttcacaca ggaaacagct atgacatgtt ttgggagaat      60
taagtctga                                                              69
```

<210> 53
<211> 69
<212> DNA
<213> Artificial Sequence

<220>
<223> Designed oligonucleotide primer designated as 3'ID to amplify a portion of cyclin A DNA

<400> 53

```
gagttcgtgc cgtacaacta tttcacacag gaaacagcta tgacttacag atttagtgtc      60
tctggtggg                                                              69
```

<210> 54
<211> 16
<212> DNA
<213> Artificial Sequence

<220>
<223> Designed oligonucleotide primer designated as M13RV-2N 16mer

<400> 54
aggaaacagc tatgac 16

<210> 55
<211> 20
<212> DNA
<213> Artificial Sequence

<220>
<223> Designed oligonucleotide primer designated as M13M4-3N 16mer

<400> 55
agggttttcc cagtcacgac 20

<210> 56
<211> 24
<212> DNA
<213> Artificial Sequence

<220>
<223> Designed oligonucleotide primer designated as M13M4-3N 24mer

<400> 56
cgccagggtt ttcccagtca cgac 24

<210> 57
<211> 24
<212> DNA
<213> Artificial Sequence

<220>
<223> Designed oligonucleotide primer designated as M13RV-3N 24mer

<400> 57
tttcacacag gaaacagcta tgac 24

<210> 58
<211> 17
<212> DNA
<213> Artificial Sequence

<220>
<223> Designed oligonucleotide primer designated as M13M4

<400> 58
gttttcccag tcacgac 17

<210> 59
<211> 27
<212> DNA
<213> Artificial Sequence

<220>
<223> Designed oligonucleotide primer to amplify a portion of human transferrin receptor-encoding sequence

<400> 59
cagcaactgg gccagcaaag ttgagaa 27

<210> 60
<211> 27
<212> DNA
<213> Artificial Sequence

<220>
<223> Designed oligonucleotide primer to amplify a portion of human transferrin receptor-encoding sequence

<400> 60
gcaaaaacag aaagaaactg ctcagaa 27

<210> 61
<211> 26
<212> DNA
<213> Artificial Sequence

<220>
<223> Designed oligonucleotide primer to amplify a portion of human transferrin receptor-encoding sequence

<400> 61
cagcaactgg gccagcaaag ttgaga 26

<210> 62
<211> 26
<212> DNA
<213> Artificial Sequence

<220>
<223> Designed oligonucleotide primer to amplify a portion of human transferrin receptor-encoding sequence

<400> 62
gcaaaaacag aaagaaactg ctcaga 26

<210> 63
<211> 25
<212> DNA
<213> Artificial Sequence

<220>
<223> Designed oligonucleotide primer to amplify a portion of human transferrin receptor-encoding sequence

<400> 63
cagcaactgg gccagcaaag ttgag 25

<210> 64
<211> 25
<212> DNA
<213> Artificial Sequence

<220>
<223> Designed oligonucleotide primer to amplify a portion of human transferrin receptor-encoding sequence

<400> 64
gcaaaaacag aaagaaactg ctcag 25

<210> 65
<211> 24
<212> DNA
<213> Artificial Sequence

<220>
<223> Designed oligonucleotide primer to amplify a portion of human transferrin receptor-encoding sequence

<400> 65
cagcaactgg gccagcaaag ttga 24

<210> 66
<211> 24
<212> DNA
<213> Artificial Sequence

<220>
<223> Designed oligonucleotide primer to amplify a portion of human transferrin receptor-encoding sequence

<400> 66
gcaaaaacag aaagaaactg ctca 24

<210> 67
<211> 23
<212> DNA
<213> Artificial Sequence

<220>
<223> Designed oligonucleotide primer to amplify a portion of human transferrin receptor-encoding sequence

<400> 67
cagcaactgg gccagcaaag ttg 23

<210> 68
<211> 23
<212> DNA
<213> Artificial Sequence

<220>
<223> Designed oligonucleotide primer to amplify a portion of human transferrin receptor-encoding sequence

<400> 68
gcaaaaacag aaagaaactg ctc 23

SEQUENCE LISTING

<110> Takara Shuzo Co., Ltd.

<120> Method for amplifying nucleic acid sequence

<130> 38-39

<140> EP 00908056.5
<141> 2000-03-14

<150> JP 11/76966
<151> 1999-03-19

<150> JP 11/370035
<151> 1999-12-27

<160> 68

<210> 1
<211> 99
<212> DNA
<213> Artificial Sequence

<220>
<223> Synthetic DNA corresponding to a portion of human transferrin receptor-encoding sequence used as a template
<400> 1

```
ggacagcaac tgggccagca aagttgagaa actcacttta gagaattctg ctttcccttt     60
ccttgcatat tctgagcagt ttctttctgt ttttgcgag                            99
```

<210> 2

<211> 22
<212> DNA
<213> Artificial Sequence

<220>
<223> Designed oligonucleotide primer to amplify a portion of human transferrin receptor-encoding sequence

<400> 2
cagcaactgg gccagcaaag tt 22

<210> 3
<211> 22
<212> DNA
<213> Artificial Sequence

<220>
<223> Designed oligonucleotide primer to amplify a portion of human transferrin receptor-encoding sequence

<400> 3
gcaaaaacag aaagaaactg ct 22

<210> 4
<211> 26
<212> DNA
<213> Artificial Sequence

<220>
<223> Designed oligonucleotide used as a probe for detecting an amplified portion of human transferrin receptor-encoding sequence

<400> 4
tgctttccct ttccttgcat attctg 26

<210> 5
<211> 25
<212> DNA
<213> Artificial Sequence

<220>
<223> Designed oligonucleotide primer designated as pUC19 upper(2)NN to amplify a portion of plasmid pUC19

<400> 5
attgcttaat cagtgaggca cctat 25

<210> 6
<211> 25
<212> DNA
<213> Artificial Sequence

<220>
<223> Designed oligonucleotide primer designated as pUC19 lower NN to amplify a portion of plasmid pUC19

<400> 6
gataacactg cggccaactt acttc 25

<210> 7
<211> 25
<212> DNA

<213> Artificial Sequence

<220>
<223> Designed oligonucleotide primer to amplify a portion of plasmid pUC19

<400> 7
actggcgaac tacttactct agctt 25

<210> 8
<211> 26
<212> DNA
<213> Artificial Sequence

<220>
<223> Designed oligonucleotide primer designated as pUC19 lower 542 to amplify a portion of plasmid pUC19

<400> 8
agtcaccaga aaagcatctt acggat 26

<210> 9
<211> 25
<212> DNA
<213> Artificial Sequence

<220>
<223> Designed oligonucleotide primer to amplify a portion of plasmid pUC19

<400> 9
gctcatgaga caataaccct gataa 25

<210> 10
<211> 25
<212> DNA
<213> Artificial Sequence

<220>
<223> Designed oligonucleotide primer designated as pUC19 upper 150 to amplify a portion of plasmid pUC19

<400> 10
ggtgtcacgc tcgtcgtttg gtatg 25

<210> 11
<211> 25
<212> DNA
<213> Artificial Sequence

<220>
<223> Designed oligonucleotide primer designated as pUC19 upper 249 to amplify a portion of plasmid pUC19

<400> 11
cgcctccatc cagtctatta attgt 25

<210> 12
<211> 22
<212> DNA
<213> Artificial Sequence

<220>

<223> Designed oligonucleotide primer to amplify a portion of human transferrin receptor-encoding sequence

<400> 12
ctgattgaga ggattcctga gt 22

<210> 13
<211> 22
<212> DNA
<213> Artificial Sequence

<220>
<223> Designed oligonucleotide primer to amplify a portion of human transferrin receptor-encoding sequence

<400> 13
tagggagaga ggaagtgata ct 22

<210> 14
<211> 22
<212> DNA
<213> Artificial Sequence

<220>
<223> Designed oligonucleotide primer to amplify a portion of human transferrin receptor-encoding sequence

<400> 14
caacttcaag gtttctgcca gc 22

<210> 15
<211> 21
<212> DNA
<213> Artificial Sequence

<220>
<223> Designed oligonucleotide primer to amplify a portion of human transferrin receptor-encoding sequence

<400> 15
aatagtccaa gtagctagag c 21

<210> 16
<211> 24
<212> DNA
<213> Artificial Sequence

<220>
<223> Designed oligonucleotide primer designated as MF2N3(24) to amplify a portion of plasmid pUC19-249 or plasmid pUC19-911

<400> 16
gctgcaaggc gattaagttg ggta 24

<210> 17
<211> 24
<212> DNA
<213> Artificial Sequence

<220>
<223> Designed oligonucleotide primer designated as MR1N3(24) to amplify a portion of plasmid pUC19-249 or plasmid pUC19-911

<400> 17
ctttatgctt ccggctcgta tgtt 24

<210> 18
<211> 30
<212> DNA
<213> Artificial Sequence

<220>
<223> Designed oligonucleotide primer to amplify a portion of plasmid pUC19

<400> 18
ggatgtgctg caaggcgatt aagttgggta 30

<210> 19
<211> 30
<212> DNA
<213> Artificial Sequence

<220>
<223> Designed oligonucleotide primer designated as MR1N3 to amplify a portion of plasmid pUC19

<400> 19
tttacacttt atgcttccgg ctcgtatgtt 30

<210> 20
<211> 30
<212> RNA
<213> Artificial Sequence

<220>
<223> Synthetic RNA used as a probe for detecting an amplified portion of plasmid pUC19

<400> 20
ugaucccccca uguugugcaa aaaagcgguu 30

<210> 21
<211> 18
<212> DNA
<213> Artificial Sequence

<220>
<223> Designed oligonucleotide primer to amplify a portion of vero toxin 1-encoding sequence from hemorrhagic Escherichia coli O-157

<400> 21
agttaatgtg gtggcgaa 18

<210> 22
<211> 17
<212> DNA
<213> Artificial Sequence

<220>
<223> Designed oligonucleotide primer to amplify a portion of vero toxin 1-encoding sequence from hemorrhagic Escherichia coli O-157

<400> 22

gactcttcca tctgcca 17

<210> 23
<211> 18
<212> DNA
<213> Artificial Sequence

<220>
<223> Designed oligonucleotide primer to amplify a portion of vero toxin 2-encoding sequence from hemorrhagic Escherichia coli O-157

<400> 23
ttcggtatcc tattcccg 18

<210> 24
<211> 18
<212> DNA
<213> Artificial Sequence

<220>
<223> Designed oligonucleotide primer to amplify a portion of vero toxin 2-encoding sequence from hemorrhagic Escherichia coli O-157

<400> 24
tctctggtca ttgtatta 18

<210> 25
<211> 22
<212> DNA
<213> Artificial Sequence

<220>
<223> Designed oligonucleotide primer designated as MCR-F to amplify a long DNA fragment

<400> 25
ccattcaggc tgcgcaactg tt 22

<210> 26
<211> 22
<212> DNA
<213> Artificial Sequence

<220>
<223> Designed oligonucleotide primer designated as MCR-R to amplify a long DNA fragment

<400> 26
tggcacgaca ggtttcccga ct 22

<210> 27
<211> 24
<212> DNA
<213> Artificial Sequence

<220>
<223> Designed oligonucleotide primer designated as MF2N3(24) to amplify a long DNA fragment

<400> 27
gctgcaaggc gattaagttg ggta 24

<210> 28
<211> 24
<212> DNA
<213> Artificial Sequence

<220>
<223> Designed oligonucleotide primer designated as MR1N3(24) to amplify a long DNA fragment

<400> 28
ctttatgctt ccggctcgta tgtt 24

<210> 29
<211> 20
<212> DNA
<213> Artificial Sequence

<220>
<223> Designed oligonucleotide primer to amplify a portion of bacteriophage lambda DNA

<400> 29
aacaacaaga aactggtttc 20

<210> 30
<211> 20
<212> DNA
<213> Artificial Sequence

<220>
<223> Designed oligonucleotide primer to amplify a portion of bacteriophage lambda DNA

<400> 30
gcaatgcatg acgactgggg 20

<210> 31
<211> 17
<212> DNA
<213> Artificial Sequence

<220>
<223> Designed oligonucleotide primer to amplify a portion of bacteriophage lambda DNA

<400> 31
gttttcccag tcacgac 17

<210> 32
<211> 17
<212> DNA
<213> Artificial Sequence

<220>
<223> Designed oligonucleotide primer to amplify a portion of bacteriophage lambda DNA

<400> 32
caggaaacag ctatgac 17

<210> 33
<211> 20
<212> DNA

<213> Artificial Sequence

<220>
<223> Designed oligonucleotide primer to amplify a portion of bacteriophage lambda DNA

<400> 33
gtacggtcat catctgacac 20

<210> 34
<211> 20
<212> DNA
<213> Artificial Sequence

<220>
<223> Designed oligonucleotide primer to amplify a portion of bacteriophage lambda DNA

<400> 34
gcaatcggca tgttaaacgc 20

<210> 35
<211> 20
<212> DNA
<213> Artificial Sequence

<220>
<223> Designed oligonucleotide primer to amplify a portion of bacteriophage lambda DNA

<400> 35
cgccatcctg ggaagactcc 20

<210> 36
<211> 44
<212> DNA
<213> Artificial Sequence

<220>
<223> Designed oligonucleotide primer designated as R1-S1 to amplify a portion of bacteriophage lambda DNA

<400> 36
tttcacacag gaaacagcta tgacaacaac aagaaactgg tttc 44

<210> 37
<211> 44
<212> DNA
<213> Artificial Sequence

<220>
<223> Designed oligonucleotide primer designated as R1-A3 to amplify a portion of bacteriophage lambda DNA

<400> 37
tttcacacag gaaacagcta tgacgcaatg catgacgact gggg 44

<210> 38
<211> 62
<212> DNA
<213> Artificial Sequence

<220>

<223> Designed oligonucleotide primer designated as R2-S1 to amplify a portion of bacteriophage lambda DNA

<400> 38

```
attgtgagcg gataacaatt tcacacagga aacagctatg acaacaacaa gaaactggtt     60
tc                                                                    62
```

<210> 39
<211> 62
<212> DNA
<213> Artificial Sequence

<220>
<223> Designed oligonucleotide primer designated as R2-A3 to amplify a portion of bacteriophage lambda DNA

<400> 39

```
attgtgagcg gataacaatt tcacacagga aacagctatg acgcaatgca tgacgactgg     60
gg                                                                    62
```

<210> 40
<211> 95
<212> DNA
<213> Artificial Sequence

<220>
<223> Designed oligonucleotide primer designated as R3-S1 to amplify a portion of bacteriophage lambda DNA

<400> 40

```
cactttatgc ttccggctcg tatgttgtgt ggaattgtga gcggataaca atttcacaca     60
ggaaacagct atgacaacaa caagaaactg gtttc                                95
```

<210> 41
<211> 95
<212> DNA
<213> Artificial Sequence

<220>
<223> Designed oligonucleotide primer designated as R3-A3 to amplify a portion of bacteriophage lambda DNA

<400> 41

```
cactttatgc ttccggctcg tatgttgtgt ggaattgtga gcggataaca atttcacaca     60
ggaaacagct atgacgcaat gcatgacgac tgggg                                95
```

<210> 42
<211> 17
<212> DNA
<213> Artificial Sequence

<220>
<223> Designed oligonucleotide primer designated as M13RV-2N 17mer

<400> 42
caggaaacag ctatgac 17

<210> 43
<211> 20
<212> DNA
<213> Artificial Sequence

<220>
<223> Designed oligonucleotide primer designated as M13RV-2N 20mer

<400> 43
acacaggaaa cagctatgac 20

<210> 44
<211> 70
<212> DNA
<213> Artificial Sequence

<220>
<223> Designed oligonucleotide primer to amplify a portion of CDC2-related protein kinase PISSLRE gene

<400> 44

gagttcgtgt ccgtacaact atttcacaca ggaaacagct atgacccaac aagagcctat 60
agcttcgctc 70

<210> 45
<211> 67
<212> DNA
<213> Artificial Sequence

<220>
<223> Designed oligonucleotide primer to amplify a portion of CDC2-related protein kinase PISSLRE gene

<400> 45

tcgaaatcag ccacagcgcc atttcacaca ggaaacagct atgacccgct gtctttgagt 60
tgtggtg 67

<210> 46
<211> 70
<212> DNA
<213> Artificial Sequence

<220>
<223> Designed oligonucleotide primer to amplify a portion of Type II cytoskeltal 11 keratin gene

<400> 46

gagttcgtgt ccgtacaact atttcacaca ggaaacagct atgacgctat tctgacatca 60
ctttccagac 70

<210> 47
<211> 66

<212> DNA
<213> Artificial Sequence

<220>
<223> Designed oligonucleotide primer to amplify a portion of Type II cytoskeltal 11 keratin gene

<400> 47

```
tcgaaatcag ccacagcgcc atttcacaca ggaaacagct atgacgaatt ccactggtgg     60
cagtag                                                                66
```

<210> 48
<211> 62
<212> DNA
<213> Artificial Sequence

<220>
<223> Designed oligonucleotide primer to amplify a portion of bacteriophage lambda DNA

<400> 48

```
attgtgagcg gataacaatt tcacacagga aacagctatg acgtacggtc atcatctgac     60
ac                                                                    62
```

<210> 49
<211> 62
<212> DNA
<213> Artificial Sequence

<220>
<223> Designed oligonucleotide primer to amplify a portion of bacteriophage lambda DNA

<400> 49

```
attgtgagcg gataacaatt tcacacagga aacagctatg acatgcgccg cctgaaccac     60
ca                                                                    62
```

<210> 50
<211> 62
<212> DNA
<213> Artificial Sequence

<220>
<223> Designed oligonucleotide primer to amplify a portion of bacteriophage lambda DNA

<400> 50

```
attgtgagcg gataacaatt tcacacagga aacagctatg acctgctctg ccgcttcacg     60
ca                                                                    62
```

<210> 51

<211> 62
<212> DNA
<213> Artificial Sequence

<220>
<223> Designed oligonucleotide primer to amplify a portion of bacteriophage lambda DNA

<400> 51

```
attgtgagcg gataacaatt tcacacagga aacagctatg acgcaatcgg catgttaaac     60
gg                                                                    62
```

<210> 52
<211> 69
<212> DNA
<213> Artificial Sequence

<220>
<223> Designed oligonucleotide primer designated as 5'ID to amplify a portion of cyclin A DNA

<400> 52

```
tcgaaatcag ccacagcgcc atttcacaca ggaaacagct atgacatgtt ttgggagaat     60
taagtctga                                                             69
```

<210> 53
<211> 69
<212> DNA
<213> Artificial Sequence

<220>
<223> Designed oligonucleotide primer designated as 3'ID to amplify a portion of cyclin A DNA

<400> 53

```
gagttcgtgc cgtacaacta tttcacacag gaaacagcta tgacttacag atttagtgtc     60
tctggtggg                                                             69
```

<210> 54
<211> 16
<212> DNA
<213> Artificial Sequence

<220>
<223> Designed oligonucleotide primer designated as M13RV-2N 16mer

<400> 54
aggaaacagc tatgac 16

<210> 55
<211> 20
<212> DNA
<213> Artificial Sequence

<220>

<223> Designed oligonucleotide primer designated as M13M4-3N 16mer

<400> 55
agggttttcc cagtcacgac 20

<210> 56
<211> 24
<212> DNA
<213> Artificial Sequence

<220>
<223> Designed oligonucleotide primer designated as M13M4-3N 24mer

<400> 56
cgccagggtt ttcccagtca cgac 24

<210> 57
<211> 24
<212> DNA
<213> Artificial Sequence

<220>
<223> Designed oligonucleotide primer designated as M13RV-3N 24mer

<400> 57
tttcacacag gaaacagcta tgac 24

<210> 58
<211> 17
<212> DNA
<213> Artificial Sequence

<220>
<223> Designed oligonucleotide primer designated as M13M4

<400> 58
gttttcccag tcacgac 17

<210> 59
<211> 27
<212> DNA
<213> Artificial Sequence

<220>
<223> Designed oligonucleotide primer to amplify a portion of human transferrin receptor-encoding sequence

<400> 59
cagcaactgg gccagcaaag ttgagaa 27

<210> 60
<211> 27
<212> DNA
<213> Artificial Sequence

<220>
<223> Designed oligonucleotide primer to amplify a portion of human transferrin receptor-encoding sequence

<400> 60

gcaaaaacag aaagaaactg ctcagaa 27

<210> 61
<211> 26
<212> DNA
<213> Artificial Sequence

<220>
<223> Designed oligonucleotide primer to amplify a portion of human transferrin receptor-encoding sequence

<400> 61
cagcaactgg gccagcaaag ttgaga 26

<210> 62
<211> 26
<212> DNA
<213> Artificial Sequence

<220>
<223> Designed oligonucleotide primer to amplify a portion of human transferrin receptor-encoding sequence

<400> 62
gcaaaaacag aaagaaactg ctcaga 26

<210> 63
<211> 25
<212> DNA
<213> Artificial Sequence

<220>
<223> Designed oligonucleotide primer to amplify a portion of human transferrin receptor-encoding sequence

<400> 63
cagcaactgg gccagcaaag ttgag 25

<210> 64
<211> 25
<212> DNA
<213> Artificial Sequence

<220>
<223> Designed oligonucleotide primer to amplify a portion of human transferrin receptor-encoding sequence

<400> 64
gcaaaaacag aaagaaactg ctcag 25

<210> 65
<211> 24
<212> DNA
<213> Artificial Sequence

<220>
<223> Designed oligonucleotide primer to amplify a portion of human transferrin receptor-encoding sequence

<400> 65
cagcaactgg gccagcaaag ttga 24

<210> 66

<211> 24
<212> DNA
<213> Artificial Sequence

<220>
<223> Designed oligonucleotide primer to amplify a portion of human transferrin receptor-encoding sequence

<400> 66
gcaaaaacag aaagaaactg ctca 24

<210> 67
<211> 23
<212> DNA
<213> Artificial Sequence

<220>
<223> Designed oligonucleotide primer to amplify a portion of human transferrin receptor-encoding sequence

<400> 67
cagcaactgg gccagcaaag ttg 23

<210> 68
<211> 23
<212> DNA
<213> Artificial Sequence

<220>
<223> Designed oligonucleotide primer to amplify a portion of human transferrin receptor-encoding sequence

<400> 68
gcaaaaacag aaagaaactg ctc 23

**Description**

Technical Field

The present invention relates to a method for synthesizing a DNA, which is useful in a field of genetic engineering. It relates to a method for amplifying a nucleotide sequence as a template and a method for detecting a nucleic acid amplified by said method.

Background Art

DNA synthesis is used for various purposes in studies in a field of genetic engineering. Most of the DNA synthesis with the exception of that of a short-chain DNA such as an oligonucleotide is carried out by enzymatic methods in which a DNA polymerase is utilized. An example of the methods is the polymerase chain reaction (PCR) method as described in United States Patent Nos. 4,683,195, 4,683,202 and 4,800,159 in detail. Another example is the reverse transcription-PCR (RT-PCR) method, which is a combination of the PCR method and a reverse transcriptase reaction, as described in Trends in Biotechnology, 10:146-152 (1992). The development of the above-mentioned methods has enabled the amplification of a region of interest from a DNA or an RNA.

The above-mentioned DNA synthesis methods are conducted, for example, using a reaction that consists of three steps. The three steps are a step of dissociating (denaturing) a double-stranded DNA into single-stranded DNAs, a step of annealing a primer to the single-stranded DNA and a step of synthesizing (extending) a complementary strand from the primer in order to amplify a region of a DNA of interest. Alternatively, they are conducted using a reaction designated as "the shuttle PCR" ("PCR ho saizensen" (Recent advances in PCR methodology: Basic methodology and it's application), Tanpakushitsu Kakusan Kouso, Bessatsu, (Protein, Nucleic Acid and Enzyme, Supplement), 41(5): 425-428 (1996)) in which two of the three steps, that is, the step of annealing the primer and the step of extending are carried out at the same temperature.

Alternatively, the ligase chain reaction (LCR) method as described in EP 320,308 published on June 14, 1989 or the transcription-based amplification system (TAS) method as described in PCR Protocols, Academic Press Inc., 1990, pp. 245-252 may be used. The four methods as mentioned above require repeating a reaction at a high temperature and that at a low temperature several times in order to regenerate a single-stranded target molecule for the next amplification cycle. The reaction system should be conducted using discontinuous phases or cycles because the reaction is restricted by temperature as described above.

Thus, the methods require the use of an expensive thermal cycler that can strictly adjust a wide range of temperature over time. Furthermore, the reaction requires time for adjusting the temperature to the two or three predetermined ones. The loss of time increases in proportion to the cycle number.

Nucleic acid amplification methods that can be carried out isothermally have been developed in order to solve the problems. Examples thereof include the strand displacement amplification (SDA) method as described in JP-B 7-114718, the self-sustained sequence replication (3SR) method, the nucleic acid sequence based amplification (NASBA) method as described in Japanese Patent No. 2650159, the transcription-mediated amplification (TMA) method, the Qβ replicase method as described in Japanese Patent No. 2710159 and the various modified SDA methods as described in United States Patent No. 5,824,517, WO 99/09211, WO 95/25180 and WO 99/49081. A method of isothermal enzymatic synthesis of an oligonucleotide is described in United States Patent No. 5,916,777. Extension from a primer and/or annealing of a primer to a single-stranded extension product (or to an original target sequence) followed by extension from the primer take place in parallel in a reaction mixture incubated at a constant temperature in the reaction of these methods of isothermal nucleic acid amplification or synthesis of an oligonucleotide.

Among the isothermal nucleic acid amplification methods, the SDA method is an example of systems in which a DNA is finally amplified. The SDA method is a method for amplifying a target nucleic acid sequence (and a complementary strand thereof) in a sample by displacement of double strands using a DNA polymerase and a restriction endonuclease. The method requires four primers used for the amplification, two of which should be designed to contain a recognition site for the restriction endonuclease. The method requires the use of a modified deoxyribonucleotide triphosphate as a substrate for DNA synthesis in large quantities. An example of the modified deoxyribonucleotide triphosphates is an (α-S) deoxyribonucleotide triphosphate in which the oxygen atom of the phosphate group at the α-position is replaced by a sulfur atom (S). The problem of running cost associated with the use of the modified deoxyribonucleotide triphosphate becomes serious if the reaction is routinely conducted, for example, for genetic test. Furthermore, the incorporation of the modified nucleotide such as the (α-S) deoxyribonucleotide into the amplified DNA fragment in the method may abolish the cleavability of the amplified DNA fragment with a restriction enzyme, for example, when it is subjected to a restriction enzyme fragment length polymorphism (RFLP) analysis.

The modified SDA method as described in United States Patent No. 5,824,517 is a DNA amplification method that uses a chimeric primer that is composed of an RNA and a DNA and has as an essential element a structure in which DNA is positioned at least at the 3'-terminus. The modified SDA method as described in WO 99/09211 requires the use of a restriction enzyme that generates a 5'-protruding end. The modified SDA method as described in WO 95/25180 requires the use of at least two pairs of primers. The modified SDA method as described in WO 99/49081 requires the use of at least two pairs of primers and at least one modified deoxyribonucleotide triphosphate. On the other hand, the method for synthesizing an oligonucleotide as described in United States Patent No. 5,916,777 comprises synthesizing a DNA using a primer having a ribonucleotide at the 3'-terminus, completing a reaction using the primer, introducing a nick between the primer and an extended strand in an primer-extended strand with an endonuclease to separate them, digesting a template and recovering the primer to reuse it. It is required to isolate the primer from the reaction system and then anneal it to the template again in order to reuse the primer in the method.

As described above, the conventional isothermal nucleic acid amplification methods still have various problems. Thus, a method for amplifying a nucleotide sequence at low running cost by which a DNA fragment that can be further genetically engineered is obtained has been desired.

Objects of Invention

The main object of the present invention is to provide a convenient and efficient method for amplifying a nucleotide sequence characterized in that a DNA synthesis reaction is carried out in the presence of an oligonucleotide primer and a method for producing an amplified DNA fragment in large quantities for supplying it.

Summary of Invention

As a result of intensive studies, the present inventors have constructed an excellent system for gene amplification reaction. The construction was accomplished by developing a method in which a region of a DNA of interest is amplified in the presence of a chimeric oligonucleotide primer having a ribonucleotide positioned at the 3'-terminus or on the 3'-terminal side, an endonuclease and a DNA polymerase as defined in the claims. Thus, the present invention has been completed. The method of the present invention is designated as the isothermally chimeric primer used amplification of nucleic acid (ICAN) method, which is a method for amplifying a nucleotide sequence in which a chimeric oligonucleotide primer is used under isothermal conditions.

In a first aspect, the present invention relates to a method for amplifying a nucleotide sequence or for producing a nucleic acid in large quantities, characterized in that the method comprises:

(a) preparing a reaction mixture by mixing a nucleic acid as a template, a deoxyribonucleotide triphosphate, a DNA polymerase having a strand displacement activity, at least one primer and an endonuclease that cleaves an extended strand generated from the primer, wherein the primer is a chimeric oligonucleotide primer that is substantially complementary to the base sequence of the nucleic acid as the template and contains deoxyribonucleotides and one or more ribonucleotide(s), the ribonucleotide(s) being positioned at the 3'-terminus or on the 3'-terminal side of the primer wherein the endonuclease cleaves at a site that contains the ribonucleotide, and

(b) incubating the reaction mixture for a sufficient time to generate a reaction product under conditions where specific annealing of the primer to the nucleic acid as a template, an extended strand synthesis reaction and a strand displacement reaction by the DNA polymerase, and a reaction of cleaving the extended strand by the endonuclease take place.

In a preferred embodiment, the method of the first aspect comprises:

(a) treating a nucleic acid as a template with at least one primer that is substantially complementary to the nucleotide sequence of the nucleic acid and a DNA polymerase to synthesize a primer-extended strand that is complementary to the template, wherein the primer is a chimeric oligonucleotide primer containing a deoxyribonucleotide and a ribonucleotide, the ribonucleotide being positioned at the 3'-terminus or on the 3'-terminal side of the primer for cleavage with an endonuclease;

(b) cleaving the primer-extended strand of a double-stranded nucleic acid obtained in step (a) with the endonuclease at a site that contains the ribonucleotide; and

(c) extending a nucleotide sequence that is complementary to the template using a DNA polymerase having a strand displacement activity from the 3'-terminus of the primer portion of the double-stranded nucleic acid in which the primer-extended strand is cleaved obtained in step (b) to effect a strand displacement.

In a preferred embodiment, the method of the first aspect of the present invention relates to a method for amplifying a nucleotide sequence using at least two primers, characterized in that the method comprises:

(a) treating a nucleic acid as a template with at least one primer that is substantially complementary to the nucleotide sequence of the nucleic acid and a DNA polymerase to synthesize a primer-extended strand that is complementary to the template, wherein the primer is a chimeric oligonucleotide primer containing a deoxyribonucleotide and a ribonucleotide, the ribonucleotide being positioned at the 3'-terminus or on the 3'-terminal side of the primer for cleavage with an endonuclease;
(b) cleaving the primer-extended strand of a double-stranded nucleic acid obtained in step (a) with the endonuclease at a site that contains the ribonucleotide;
(c) extending a nucleotide sequence that is complementary to the template using a DNA polymerase having a strand displacement activity from the 3'-terminus of the primer portion of the double-stranded nucleic acid in which the primer-extended strand is cleaved obtained in step (b) to effect a strand displacement, wherein a double-stranded nucleic acid containing a regenerated primer-extended strand is reused in step (b);
(d) treating a released displaced strand obtained in step (c) as a template with at least one primer that is different from that used in step (a) and a DNA polymerase to synthesize a primer-extended strand that is complementary to the displaced strand, wherein the primer that is different from that used in step (a) is a chimeric oligonucleotide primer that is substantially complementary to the nucleotide sequence of the displaced strand and contains a deoxyribonucleotide and a ribonucleotide, the ribonucleotide being positioned at the 3'-terminus or on the 3'-terminal side of the primer for cleavage with an endonuclease;
(e) cleaving the primer-extended strand of a double-stranded nucleic acid obtained in step (d) with the endonuclease at a site that contains the ribonucleotide; and
(f) extending a nucleotide sequence that is complementary to the template using a DNA polymerase having a strand displacement activity from the 3'-terminus of the primer portion of the double-stranded nucleic acid in which the primer-extended strand is cleaved obtained in step (e) to effect a strand displacement, wherein a double-stranded nucleic acid containing a regenerated primer-extended strand is reused in step (e).

The method of the first aspect of the present invention may be conducted isothermally. The nucleotide sequence as the template may be a DNA sequence. A step of preparing a single-stranded cDNA by a reverse transcription reaction using reverse transcriptase and an RNA as a template may be comprised prior to step (a). The single-stranded cDNA may be used as the nucleotide sequence as the template. Both of a single-stranded DNA and a double-stranded DNA can be preferably used as the DNA as the template in the method of the first aspect of the present invention. If a double-stranded DNA is used as the template, the method of the first aspect of the present invention may be conducted after a pretreatment step of denaturing the double-stranded DNA into single-stranded DNAs.

In the above-mentioned method, the extension from the primer is conducted using a DNA polymerase having a strand displacement activity. A DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax can be preferably used in the present invention. Additionally, an endoribonuclease can be preferably used as the endonuclease. The endoribonuclease that can be used include, but are not limited to, RNase H, for example.

The primer used in the method of the first aspect of the present invention is a chimeric oligonucleotide primer. For example, a chimeric oligonucleotide having a. structure in which at least one, preferably two or more successive ribonucleotide residues are attached at the 3'-terminus or on the 3'-terminal side of the primer can be used.

The template used in the method of the first aspect of the present invention may be a nucleic acid that is amplified beforehand by a nucleic acid amplification method. For example, the TAS method, the 3SR method, the NASBA method, the TMA method, the Qβ replicase method, the PCR method, the LCR method and the SDA method can be utilized as the nucleic acid amplification method although any methods for amplifying a nucleic acid can be used without limitation. The method of the present invention can be used in combination with these nucleic acid amplification methods.

A random primer or a degenerate primer can be used in the nucleic acid amplification reaction. For example, without limitation, a primer having a random sequence or a degenerate sequence at least at the 3'-terminus or on the 3'-terminal side can be preferably used.

In a second aspect, the present invention relates to a method for detecting a target nucleic acid, characterized in that the method comprises amplifying a target nucleic acid by the above method for amplifying a nucleotide sequence of the first aspect of the present invention and then detecting the nucleic acid. The methods of detection include a method in which the target nucleic acid is detected using a ribonucleotide (RNA) probe labeled with two or more fluorescent dyes positioned at a distance that results in a quenching state.

In a third aspect, the present invention relates to a kit containing a DNA polymerase having a strand displacement activity, an endonuclease and the chimeric primer used in the method for detecting a target nucleic acid of the second aspect of the present invention.

In a fourth aspect, the present invention relates to a method for producing a material having an immobilized nucleic acid in which the nucleic acid is arrayed in a predefined region, characterized in that the method comprises arraying and immobilizing the nucleic acid amplified by the method for amplifying a nucleotide sequence of the first aspect of the present invention in a predefined region on a substrate. A method in which a single-stranded nucleic acid substantially free of a complementary strand thereto is amplified, and arrayed and immobilized in the predefined region is particularly preferable.

In the present invention, a nucleic acid containing a sequence to be amplified is amplified beforehand by a nucleic acid amplification reaction. The amplification product is then used as a nucleic acid as a template in the method of the first aspect of the present invention. For example, the TAS method, the 3SR method, the NASBA method, the TMA method, the Qβ replicase method, the PCR method, the LCR method and the SDA method can be used as the nucleic acid amplification method used in the invention although any methods for amplifying a nucleic acid can be used without limitation.

A random primer or a degenerate primer can be used in the nucleic acid amplification reaction. For example, without limitation, a primer having a random sequence or a degenerate sequence at least at the 3'-terminus or on the 3'-terminal side can be preferably used.

In a fifth aspect, the present invention relates to a method for determining a nucleotide sequence of a nucleic acid, characterized in that the method comprises amplifying a nucleotide sequence according to the method of the first aspect of the present invention.

In a sixth aspect, the present invention relates to a method for detecting a mutation of a target nucleic acid, characterized in that the method comprises amplifying a nucleotide sequence according to the method according to the first aspect of the present invention.

Brief Description of Drawings

Figure 1 is a flow chart that illustrates an example of the method of the present invention in which a single-stranded DNA is used. In the figure, the released DNA strand marked with the closed circle serves as a template DNA in (6).

Figure 2 shows the results of agarose gel electrophoresis of DNA fragments amplified by the method of the present invention using varying reaction time.

Detailed Description of the Invention

As used herein, a deoxyribonucleotide (also referred to as a dN) refers to a nucleotide of which the sugar portion is composed of D-2-deoxyribose. The deoxyribonucleotides include, for example, ones having adenine, cytosine, guanine or thymine as the base portion.

As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which the oxygen atom of the phosphate group at the α-position is replaced by a sulfur atom (also referred to as an (α-S) ribonucleotide or an (α-S) N) or other derivatives.

As used herein, a chimeric oligonucleotide primer refers to a primer that contains a deoxyribonucleotide and a ribonucleotide. The primer may contain an unmodified deoxyribonucleotide and/or a modified deoxyribonucleotide. Alternatively, it may contain an unmodified ribonucleotide and/or a modified ribonucleotide.

The chimeric oligonucleotide primers used in the present invention include any chimeric oligonucleotide primer that has a ribonucleotide being positioned at the 3'-terminus or on the 3'-terminal side of the primer, can be used to extend a nucleic acid strand in the method of the present invention, can be cleaved with an endonuclease, and can be used to effect a strand displacement reaction.

As used herein, a 3'-terminal side refers to a portion from the center to the 3'-terminus of a nucleic acid such as a primer. Likewise, the 5'-terminal side refers to a portion from the center to the 5'-terminus of a nucleic acid.

As used herein, an endonuclease may be any one that acts on a double-stranded DNA generated by extending a DNA from the chimeric oligonucleotide primer which have been annealed to a nucleic acid as a template, and specifically cleaves it at a portion of the primer that contains a ribonucleotide.

As used herein, a DNA polymerase refers to an enzyme that synthesizes a DNA strand de novo using a DNA strand as a template. The DNA polymerases include, but are not limited to, pol I-type DNA polymerases (e.g., Escherichia coli DNA polymerase I, Klenow fragment and Taq DNA polymerase), α-type DNA polymerases (e.g., a DNA polymerase from Pyrococcus furiosus (Stratagene), VENT DNA polymerase (New England Biolabs), KOD DNA polymerase (Toyobo) and DEEP VENT DNA polymerase (New England Biolabs)) and non-α-, non-pol 1-type DNA polymerases (e.g., a DNA polymerase as described in WO 97/24444). DNA polymerases having a strand displacement activity include DNA polymerases from thermophilic bacteria of genus Bacillus such as Bacillus caldotenax (hereinafter referred to as B. ca) and Bacillus stearothermophilus (hereinafter referred to as B. st) as well as variants of these DNA polymerases lacking their 5'→3' exonuclease activities. Furthermore, the DNA polymerases of strand displacement type include DNA polymerases, e.g., Klenow fragment, that have a strand displacement activity and do not have a 5'→3' exonuclease activity. In addition, the DNA polymerase may be a mixture of plural DNA polymerases such as, without limitation, a mixture of a DNA polymerase having a strand displacement activity and a DNA polymerase that does not have a strand displacement activity.

As used herein, "a strand displacement activity" refers to an activity that can effect a strand displacement, that is, that can proceed DNA duplication on the basis of the nucleotide sequence as the template while displacing the DNA strand to release the complementary strand that has been annealed to the template strand. In addition, a DNA strand released from a nucleotide sequence as a template as a result of a strand displacement is referred to as "a displaced strand" herein.

Hereinafter, the present invention will be described in detail.

(1) Chimeric oligonucleotide primer used in the present invention.

The primer used in the method of the present invention is a chimeric oligonucleotide primer that contains a deoxyribonucleotide and a ribonucleotide. Such primers include an oligoribonucleotide primer that contains an unmodified ribonucleotide and/or a modified ribonucleotide.

A chimeric oligonucleotide primer used in the method of the present invention may be any chimeric oligonucleotide primer that has a nucleotide sequence substantially complementary to a part of the nucleotide sequence of a nucleic acid as a template, that can be used to extend a DNA strand from its 3'-terminus, and that has a site at the 3'-terminus or on the 3'-terminal side at which an endonuclease cleaves during a DNA synthesis reaction. For example, a chimeric oligonucleotide primer having a ribonucleotide being positioned at the 3'-terminus or on the 3'-terminal side can be used. The primer is usually designed such that it is complementary to a portion upstream of the region to be amplified, that is, a portion 3' to the nucleotide sequence corresponding to a region to be amplified in a nucleic acid as a template. As used herein, "a substantially complementary nucleotide sequence" means a nucleotide sequence that can anneal to a DNA as a template under reaction conditions used. Such a chimeric oligonucleotide primer or an oligonucleotide primer can be designed by those skilled in the art according to known procedures, for example, with reference to Labo Manual PCR (Takara Shuzo, pp. 13-16, 1996). A commercially available software for designing a primer such as OLIGO™ Primer Analysis software (Takara Shuzo) can be used.

The chimeric oligonucleotide primer used in the method of the present invention may contain one or more modified ribonucleotide. As used herein, a ribonucleotide may be either an unmodified ribonucleotide or a modified ribonucleotide that can be positioned at the 3'-terminus or on the 3'-terminal side of a chimeric oligonucleotide primer and that is recognized by or cleaved with an endonuclease. The ribonucleotides include both of the unmodified ribonucleotide and the modified ribonucleotide as described above. An unmodified ribonucleotide, a modified ribonucleotide or a combination thereof can be used for the chimeric oligonucleotide primer of the present invention as long as it does not abolish the function of the primer. Examples of the modified ribonucleotides include, but are not limited to, an ($\alpha$-S) ribonucleotide in which the oxygen atom bound to the phosphate group is replaced by a sulfur atom, and a ribonucleotide in which the hydroxy group at the 2-position of the ribose is replaced by a methoxy group. Such a chimeric oligonucleotide primer containing a modified ribonucleotide can be produced by using, for example, an ($\alpha$-S) ribonucleotide triphosphate, which is prepared by a method as described in United States Patent No. 5,003,097 using a sulfuration reaction reagent (Glen Research), or a 2-OMe-RNA-CE phosphoramidite reagent (Glen Research).

A chimeric oligonucleotide primer that can be used in the amplification method of the present invention may be designed to contain a modified ribonucleotide that confers resistance to the cleavage with an endonuclease. Such a primer is useful in that one can control the cleavage site with an endonuclease during amplification reaction steps.

One or two chimeric oligonucleotide primer may be used in the method of the present invention depending on the desired form of a DNA fragment after amplification (single-stranded or double-stranded). Specifically, one chimeric oligonucleotide primer is used when a single-stranded DNA is desired, whereas two primers are used when a double-stranded DNA is desired.

The length of the chimeric oligonucleotide primer used in the method of the present invention is not specifically limited, but is preferably about 12 nucleotides to about 100 nucleotides, more preferably about 15 nucleotides to about 40 nucleotides. It is preferable that the nucleotide sequence of the chimeric oligonucleotide is substantially complementary to a nucleic acid as a template such that it anneals to the nucleic acid as the template under reaction conditions used. The primer contains a sequence recognized by an endonuclease, which is utilized in a step as described below, at the 3'-terminus or on the 3'-terminal side.

For example, an oligonucleotide having a structure represented by the following general formula can be used in the DNA synthesis method of the present invention as a primer, although it is not intended to limit the present invention:

General formula: 5'-$dN_a$-$N_b$-$dN_c$3'

(a: an integer of 11 or more; b: an integer of 1 or more; c: 0 or an integer of 1 or more, dN: deoxyribonucleotide; N: unmodified ribonucleotide and/or modified ribonucleotide).

For example, a chimeric oligonucleotide primer represented by the general formula in which a = an integer of 11 or more; and b = 1 and c =0, b = 2 and c = 0, b = 3-5 and c = 0, or b = 2 and c = 0-5 can be preferably used in the present invention. The length of the ribonucleotides at the 3'-terminus or on the 3'-termunal side of the chimeric oligonucleotide primer used in the method of the present invention is preferably 1-mer to 15-mer, more preferably 1-mer to 10-mer, most preferably 1-mer to 5-mer. The number of c in the general formula is not specifically limited, but any number that can be used in the method of the present invention may be selected. Usually, 5 or less is preferably. Better results are obtained in a reaction by selecting 3 rather than 4, 2 rather than 3, and 1 rather than 2 for c. In particular, the most efficient reaction can be accomplished in case of c = 0.

The chimeric oligonucleotide primer used in the present invention has a structure in which an endonuclease cleaves a DNA strand extended from the primer using a DNA polymerase (a primer-extended strand) at a site that contains a ribonucleotide. In other words, a ribonucleotide is positioned at the 3'-terminus or on the 3'-terminal side of the chimeric oligonucleotide primer for cleavage with an endonuclease. For example, when RNase H acts on a double-stranded DNA generated by extending a DNA from a chimeric oligonucleotide primer represented by the general formula that has been annealed to a nucleic acid as a template, the chimeric oligonucleotide primer is cleaved at the ribonucleotide portion. A double-stranded DNA in which a nick is introduced between the oligonucleotide primer and a DNA strand synthesized by the extension is then generated. Then, a strand displacement reaction with a DNA polymerase proceeds from the nicked site. Thus, any chimeric oligonucleotide primer that can be used to extend a nucleic acid strand from the 3'-terminus of the primer, that can be cleaved with an endonuclease, and with which a DNA polymerase can effect a strand displacement reaction can be used in the method of the present invention.

The chimeric oligonucleotide primer can be synthesized to have any nucleotide sequence using, for example, the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the chimeric oligonucleotide primer.

(2) Endonuclease used in the present invention.

Any endonuclease that acts on a double-stranded DNA generated by DNA extension from the chimeric oligonucleotide primer as described above in (1) that has been annealed to a nucleic acid as a template and cleaves the extended strand at the site containing the ribonucleotide to effect a strand displacement reaction can be used in the present invention. That is, the endonuclease is an enzyme that generates a nick in the chimeric oligonucleotide primer portion of the double-stranded DNA. Examples of endonucleases that can be used in the present invention include, but are not limited to, ribonucleases. Among these, endoribonuclease H (RNase H) that acts on an RNA portion of a double-stranded nucleic acid composed of a DNA and an RNA can be preferably used. Any ribonuclease that has the above-mentioned activities can be preferably used in the present invention, including mesophilic and heat-resistant ones. For example, RNase H from E. coli can be used for a reaction at about 50°C to about 70°C in the method of the present invention as described below in Examples. A commercially available heat-resistant ribonuclease, Hybridase™ Thermostable RNase H (Epicenter Technologies) can also be preferably used. Furthermore, the ribonuclease may be a naturally occurring one or a variant. The enzymatic unit of RNase H indicated herein is a value expressed according to a method of measuring an enzymatic unit as described in Referential Examples.

The efficiency of the cleavage reaction with an endonuclease such as RNase H used in the method of the present invention may depend on the nucleotide sequence around the 3'-terminus of the primer and influence the amplification efficiency of the desired DNA. Therefore, it is natural to design the optimal primer for the RNase H used.

As used herein, the term "introducing a nick" or "nicking" means internally cleaving one of the two strands of a double-stranded nucleic acid. For example, RNase H acts on a hybrid double-stranded nucleic acid composed of a DNA and a ribonucleotide-containing DNA to selectively cleave the ribonucleotide-containing strand among the two strands at the ribonucleotide portion, thereby introducing a nick into the hybrid double-stranded nucleic acid.

(3) DNA polymerase used in the present invention.

A DNA polymerase having a strand displacement activity on a DNA can be used in the present invention. Particularly, a DNA polymerase substantially lacking a lacking 5'→3' exonuclease activity can be preferably used.

As used herein, "a strand displacement activity" refers to an activity that can effect a strand displacement, that is, that can proceed DNA duplication on the basis of a nucleotide sequence as a template while displacing a DNA strand to release a complementary strand that has been annealed to the template strand. Additionally, a DNA strand released from a nucleotide sequence as a template as a result of a strand displacement is referred to as "a displaced strand" herein.

Any DNA polymerases having the strand displacement activity can be used in the present invention. Examples thereof include variants of DNA polymerases lacking their 5'→3' exonuclease activities derived from thermophilic bacteria of genus Bacillus such as Bacillus caldotenax (hereinafter referred to as B. ca) and Bacillus stearothermophilus (hereinafter referred to as B. st), as well as large fragment (Klenow fragment) of DNA polymerase I from Escherichia coli (E. coli). Both of mesophilic and heat-resistant DNA polymerases can be preferably used in the present invention.

B. ca is a thermophilic bacterium having an optimal growth temperature of about 70°C. Bca DNA polymerase from this bacterium is known to have a DNA-dependent DNA polymerase activity, an RNA-dependent DNA polymerase activity (a reverse transcription activity), a 5'→3' exonuclease activity and a 3'→5' exonuclease activity.

The enzyme may be either an enzyme purified from its original source or a recombinant protein produced by using genetic engineering techniques. The enzyme may be subjected to modification such as substitution, deletion, addition or insertion by using genetic engineering techniques or other means. Examples of modified enzymes include Bca BEST DNA polymerase (Takara Shuzo), which is Bca DNA polymerase lacking its 5'→3' exonuclease activity.

(4) Composition of the reaction buffer used in the present invention.

A reaction buffer that contains a buffering component, a magnesium salt and dNTPs is used in the present invention. Examples of the buffering components that can be preferably used include, but are not limited to, Tricine, tris-hydrochloride and a phosphate (such as sodium phosphate and potassium phosphate). Among these, a buffer that contains Tricine or a phosphate as a buffering component is preferable for the present invention. The final concentration of the buffering component ranges 5-100 mM, preferably 20-50 mM. The pH ranges 6.0-9.5, preferably 7.0-9.2. For example, a buffer containing 22-46 mM Tricine at pH 7.5-9.2 or a buffer containing 25-50 mM potassium phosphate at pH 7.0-8.0 is preferably used. Examples of magnesium salts that can be preferably used include, but are not limited to, magnesium chloride, magnesium acetate or magnesium sulfate. The final concentration of the magnesium salt ranges 1-20 mM, preferably 2-10 mM. The final concentrations of dNTPs (dATP, dCTP, dGTP and dTTP) in a mixture as substrates for a DNA extension reaction range 0.1-3.0 mM, preferably 0.2-1.2 mM. The amount of the primers used in a reaction volume of 50 μl ranges 1-1000 pmol, preferably 10-100 pmol. Additionally, the reaction mixture may contain an additive in order to stabilize the amplification reaction, for example. BSA at a final concentration of 0.1% or less, dimethylsulfoxide at a final concentration of 10% or less, putrescine dihydrochloride at a final concentration of 4 mM or less, or propylenediamine at a concentration of 0.01% or less may be added. Alternatively, NMP (1-methyl-2-pyrrolidinone), glycerol, poly(ethylene glycol), dimethylsulfoxide and/or formamide may be contained. It is expected that the addition of such an organic solvent reduces the non-specific annealing of oligonucleotide primers.

The amount of RNase H from E. coli as an example of endonucleases in a reaction volume of 50 μl ranges preferably 3-200 U, more preferably 15-60 U. The amount of Bca BEST DNA polymerase as an example of DNA polymerases in a reaction volume of 50 μl ranges preferably 0.5-100 U, more preferably 1-22 U. The preferable number of units of the endonuclease, among these, may presumably vary depending on the type thereof. In such case, the composition of the buffer and the amount of the enzyme to be added are adjusted such that the amount of amplification product becomes the maximum. In any case, it is natural to optimize the composition of the reaction buffer and the like depending on the type of the enzyme used.

(5) Method for amplifying nucleotide sequence of the present invention.

The method of the present invention can be conducted by using at least one oligonucleotide primer as described above in (1) in combination with the endonuclease as described above in (2) and the DNA polymerase as described above in (3). dNTPs used for the PCR method or the like (a mixture of dATP, dCTP, dGTP and dTTP) can be preferably used as the nucleotide triphosphates as substrates in an extension reaction in the method. The dNTPs may contain a dNTP analog such as 7-deaza-dGTP as long as it serves as a substrate for the DNA polymerase used. A chimeric oligonucleotide primer is used in the method. The primer can be prepared, for example, using a DNA synthesizer according to a conventional synthesis method. A combination of the chimeric oligonucleotide primer and a normal oligonucleotide primer can be used in the method of the present invention.

The nucleic acid (DNA or RNA) used as a template in the method of the present invention may be prepared or isolated from any sample that may contain a nucleic acid. Examples of the samples that may contain the nucleic acid include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. The sample may be a preparation containing a nucleic acid obtained by processing the samples as described above according to a known method. Examples of the preparations that can be used in the present invention include a cell destruction product or a sample obtained by fractionating the product, the nucleic acid in the sample, or specific nucleic acid molecules such as a sample in which mRNAs are enriched. Furthermore, a nucleic acid such as a DNA or an RNA obtained amplifying a nucleic acid contained in the sample by a known method can be preferably used.

The preparation containing a nucleic acid can be prepared from the above-mentioned materials by using, for example, lysis with a detergent, sonication, shaking/stirring using glass beads or a French press without limitation. In some cases, it is advantageous to further process the preparation to purify the nucleic acid (e.g., in case where an endogenous nuclease exists). In such cases, the nucleic acid is purified by a know method such as phenol extraction, chromatography, ion exchange, gel electrophoresis or density-gradient centrifugation.

When it is desired to amplify a nucleic acid having a sequence derived from an RNA, the method of the present invention may be conducted using as a template a cDNA synthesized by a reverse transcription reaction that uses the RNA as a template. Any RNA for which one can make a primer for a reverse transcription reaction can be applied to the method of the present invention, including RNA molecules such as total RNA, mRNA, tRNA and rRNA in a sample as well as specific RNA species.

Any primer that anneals to an RNA as a template under reaction conditions used can be used in the reverse transcription reaction. The primer may be a primer having a nucleotide sequence that is complementary to a specific RNA as a template (a specific primer), an oligo-dT (deoxythymine) primer and a primer having a random sequence (a random primer). In view of specific annealing, the length of the primer for reverse transcription is preferably 6 nucleotides or more, more preferably 9 nucleotides or more. In view of oligonucleotide synthesis, the length is preferably 100 nucleotides or less, more preferably 30 nucleotides or less.

Additionally, a chimeric oligonucleotide primer can be used as a primer for reverse transcription. The chimeric oligonucleotide primer can also be utilized as a primer for a strand displacement reaction in the method for amplifying a nucleotide sequence of the present invention using a cDNA obtained after reverse transcription as a template. Such primer may be any one that has the properties as described above in (1) and that can be used in a reverse transcription reaction from an RNA.

Any enzyme that has an activity of synthesizing a cDNA using an RNA as a template can be used in the reverse transcription reaction. Examples thereof include reverse transcriptases originating from various sources such as avian myeloblostosis virus-derived reverse transcriptase (AMV RTase), Molony murine leukemia virus-derived reverse transcriptase (MMLV RTase) and Rous-associated virus 2 reverse transcriptase (RAV-2 RTase). In addition, a DNA polymerase that also has a reverse transcription activity can be used. An enzyme having a reverse transcription activity at a high temperature such as a DNA polymerase from a bacterium of genus Thermus (e.g., Tth DNA polymerase) and a DNA polymerase from a thermophilic bacterium of genus Bacillus is preferable for the present invention. For example, DNA polymerases from thermophilic bacteria of genus Bacillus such as a DNA polymerase from B. st (Bst DNA polymerase) and a DNA polymerase from B. ca (Bca DNA polymerase) are preferable, although it is not intended to limit the present invention. For example, Bca DNA polymerase does not require a manganese ion for the reverse transcription reaction. Furthermore, it can synthesize a cDNA while suppressing the formation of a secondary structure of an RNA as a template under high-temperature conditions. Both a naturally occurring one and a variant of the enzyme having a reverse transcriptase activity can be used as long as they have the activity.

In the amplification of a nucleic acid by the method of the present invention, a nucleic acid of interest may be amplified more efficiently by amplifying the nucleic acid as a template beforehand. For example, when a nucleotide sequence present in a trace amount of a genomic DNA is amplified, a DNA fragment containing the nucleotide sequence of interest is first amplified by an appropriate nucleic acid amplification method. The thus obtained amplified DNA fragment is then used as a template to conduct the amplification method of the present invention. The first amplification step may be conducted by the method of the present invention. Alternatively, it may be conducted by a known nucleic acid amplification method such as the PCR method. Furthermore, a specific nucleotide sequence may be added on the 5'-terminal side of the primer to be used in the amplification step. When a fragment amplified using such a primer is used as a template, the amplification method of the present invention can be conducted using a chimeric oligonucleotide primer having the specific nucleotide sequence added to the above-mentioned primer. In other words, it is possible to conduct the nucleic acid amplification step in the method of the present invention, in which a DNA fragment amplified by the PCR is used as a template, by using a common chimeric oligonucleotide prime regardless of the nucleotide sequence of the region to be amplified. This is accomplished by combining the method of the present invention and the PCR using a primer having a specific nucleotide sequence added on the 5'-terminal side as described above.

Usually, one should produce a pair of primers specific to a nucleotide sequence of interest in order to amplify the sequence in the first nucleic acid amplification step specifically. However, a nucleic acid as a template can be amplified without the use of a primer specific to the nucleotide sequence of interest by utilizing a random primer, which non-specifically amplifies nucleic acid fragments, or a pair of primers selected from a set of ready-made degenerate primers. The number of primer pairs required to amplify plural nucleic acids as templates can be decreased. The decrease is accomplished by utilizing, for example, the PCR method that uses a random primer having a tag sequence (Nucleic Acids Research, 24(19):3778-3783 (1996)) or the degenerate oligonucleotide-primed PCR method (DOP-PCR; Genomics, 13:718-725 (1992), which uses a degenerate primer having a tag sequence. Each of such -primers has a random sequence or a degenerate sequence at the 3'-terminus. If the amplification method of the present invention is conducted using a nucleic acid amplified with a primer having a tag sequence as a template, the method of the present invention can be conducted by using one chimeric oligonucleotide primer. Such chimeric oligonucleotide primer has the nucleotide sequence identical with that of the tag sequence. By using such a primer, all of the nucleic acids that have been amplified using a primer having the same tag sequence are used as templates. Thus, a variety of nucleotide sequences can be supplied at very low cost and in large quantities by combining the method of the present invention with a nucleic acid amplification method that uses a random primer or a degenerate primer.

Any DNA polymerase that synthesizes a DNA strand de novo using a DNA strand as a template can be used in the nucleic acid amplification method. Such DNA polymerases include pol I-type DNA polymerases (e.g., E. coli DNA polymerase I, Klenow fragment and Taq DNA polymerase), α-type DNA polymerases (e.g., a DNA polymerase from Pyrococcus furiosus, VENT DNA polymerase, KOD DNA polymerase and DEEP VENT DNA polymerase) and non-α-, non-pol I-type DNA polymerases (e.g., a DNA polymerase as described in WO 97/24444). In addition, a mixture of at least two DNA polymerases such as TaKaRa Ex Taq DNA polymerase (Takara Shuzo) or KOD dash DNA polymerase (Toyobo) can be preferably used. Furthermore, DNA polymerases such as a DNA polymerase from B. ca, a DNA polymerase from B. st, variants of these DNA polymerases lacking their 5'→3' exonuclease activities, 9°N DNA polymerase, Pfu (exo-) DNA polymerase (Stratagene), Tth DNA polymerase (Toyobo) and Tfl DNA polymerase (Promega) can be preferably used.

If a linear DNA fragment (e.g., a PCR-amplified fragment) is used in the amplification method of the present invention as a template, introduction of a sequence designated as a spacer portion may increase the amplification efficiency. The spacer portion is located between the 3'-terminus of the linear DNA fragment as the template and the annealing site at the 5'-terminus of the primer used for the method of the present invention. For example, it is preferable to design the primer for the amplification method of the present invention such that the length of the spacer portion is, without limitation, 1 to about 70 bases, more preferably about 5 to about 60 bases. The preferable number of bases of the spacer portion may vary depending on the sequence of the primer for the amplification method of the present invention. The optimal spacer portion can be determined with reference to the disclosure in Examples herein. A fragment amplified beforehand, for example, by PCR such that the spacer portion is added 3' to the annealing region of the amplification primer of the present invention can be used as a template in the amplification method of the present invention. In one embodiment, a nucleic acid as a template is amplified beforehand by using a primer. Such primer has, in 5'→3' direction, a region of the spacer portion, a region of the amplification primer of the present invention, and a region of another primer for amplifying a nucleic acid. The thus amplified fragment can be then used as a template in the amplification method of the present invention. The region of another primer for amplifying a nucleic acid may be any region of a primer for a nucleic acid amplification method such as the PCR method. Alternatively, the region of another primer for amplifying a nucleic acid may be a region of another amplification primer of the present invention.

Both of a double-stranded DNA such as an isolated genomic DNA or a PCR fragment and a single-stranded DNA such as a cDNA prepared by a reverse transcription reaction from a total RNA or an mRNA can be preferably used as a template DNA in the present invention. The double-stranded DNA is preferably used after denaturing it into single-stranded DNAs.

The denaturing step may be eliminated in the amplification method of the present invention if a linear double-stranded DNA such as a PCR amplification product is used as a template. The elimination may be accomplished by locating the annealing site for the primer of the present invention about 50 bases inside from the terminus of the DNA. If a nucleic acid having a sequence from an RNA is to be amplified, a reverse transcription reaction using an RNA as a template and a DNA amplification reaction using a cDNA generated by the reverse transcription reaction as a template can be conducted with one DNA polymerase in the DNA synthesis method of the present invention. Such DNA polymerase has a reverse transcriptase activity and a strand displacement activity.

The suitable length of the template is one that provides a sufficient binding of the primer sequence due to the presence of the whole target sequence or at least a sufficient part of the target sequence in the fragment.

If a DNA as a template is a double-stranded DNA, the DNA is denatured into single-stranded DNAs to allow a primer to bind to the DNA strand as the template in the method of the present invention. Maintaining the double-stranded DNA at a temperature at which it is denatured (e.g., about 95°C) is preferable for the denaturation. Other processes include one in which an elevated pH is used. In this case, the pH should be lowered in order to allow an oligonucleotide primer to bind to a target in an amplification reaction. A nucleotide sequence is successively amplified under isothermal conditions after denaturing a double-stranded DNA into single-stranded DNAs or, if an RNA is used as a template, preparing a cDNA (a single-stranded DNA) by a reverse transcription reaction.

"Successively" means that a reaction proceeds without a change in the reaction temperature or the composition of the reaction mixture. As used herein, "isothermal" means conditions of a substantially constant temperature under which an enzyme and a nucleic acid strand function in each step.

Without being restricted by a theory, it is considered that the following steps successively and repeatedly proceeds in parallel (e.g., under isothermal conditions) in the method for amplifying a nucleotide sequence of the present invention:

[1] a step of annealing a DNA as a template to at least one oligonucleotide primer;
[2] a step of effecting a reaction of extending a DNA that is complementary to the DNA as the template from the 3'-terminus of the primer;
[3] a step of cleaving the DNA strand extended in step [2] with an endonuclease;
[4] a step effecting a reaction of extending a DNA from the 3'-terminus of the site cleaved in step [3] while releasing the DNA strand extended in step [2] without degrading it from the DNA as the template; and
[5] a step of repeating step [3] and step [4] using a double-stranded polynucleotide obtained in step [4].

The above-mentioned reaction may be conducted at a normal temperature (e.g., 37°C) by using a mesophilic DNA polymerase such as Klenow fragment. It can also be conducted at a high temperature (e.g., 50°C or higher, or 60°C or higher) by using heat-resistant enzymes (an endonuclease and a DNA polymerase). In this case, non-specific annealing of a primer is suppressed, resulting in increase in the specificity of DNA amplification. Furthermore, the problem of forming secondary structure of a DNA as a template is solved, resulting in increase in the ability of extension of a DNA polymerase. In one embodiment, a reverse transcription reaction and the nucleotide sequence amplification can be successively conducted in the method. In this case, a DNA having a sequence derived from an RNA can be amplified by combining the use of a reverse transcriptase with the above-mentioned reaction or by using a DNA polymerase having a reverse transcription activity.

As used herein, "a regenerated primer-extended strand" refers to a DNA strand complementary to a nucleotide sequence as a template which is extended from an oligonucleotide primer newly utilized for duplication as a result of a strand displacement.

As used herein, "reuse" means that a double-stranded DNA composed of a nucleotide sequence as a template and a regenerated primer-extended strand is utilized again in a step of strand displacement.

In each of the above-mentioned aspects of the present invention, a chimeric oligonucleotide primer that is complementary to a single-stranded DNA as a template is first annealed to the DNA. A DNA that is complementary to the DNA as the template (a primer-extended strand) is then extended along the remaining sequence of the DNA as the template from the 3'-terminus of the primer by the action of a DNA polymerase to synthesize a double-stranded DNA. An endonuclease acts on the double-stranded DNA to cleave it at a site on the 3'-terminal side of the ribonucleotide portion in the chimeric oligonucleotide primer. The endonuclease does not cleave the DNA at other sites. Thus, the endonuclease acts as a nicking enzyme that introduces a nick into the double-stranded DNA. The endonuclease may alter the structure of the double-stranded DNA composed of the chimeric oligonucleotide primer and the DNA as the template although the present invention is not restricted by a theory. A DNA polymerase having a strand displacement activity re-extends a DNA strand from the 3'-terminus of the nick introduced in the double-stranded DNA to generate a new primer-extended strand while releasing the DNA downstream of the 3'-terminus of the nick. Thus, the new primer-extended strand replaces the previously synthesized primer-extended strand.

The method for amplifying a nucleotide sequence of the present invention can be carried out using two primers, i.e., a chimeric oligonucleotide primer that is complementary to a nucleic acid as a template and another chimeric oligonucleotide primer that is complementary to a displaced strand. In this case, one primer binds to a DNA strand as a template to cause a strand displacement reaction, whereas another primer binds to a displaced strand released as a result of the strand displacement reaction to initiate another strand displacement reaction. It is clear that a reaction product with one primer can function as a template for another primer if this aspect is used. Thus, the amount of amplification product increases in a non-linear manner as the amount of the template increases.

When the method for amplifying a nucleotide sequence of the present invention is conducted using a double-stranded DNA as a template, a chimeric oligonucleotide primer, four deoxyribonucleotide triphosphates (dNTPs), a DNA polymerase and an endonuclease are added to a reaction mixture before or after the double-stranded DNA is denatured. If heat treatment is used for denaturing the double-stranded DNA and a heat-resistant enzyme is not used, it is preferable to add the enzyme after the denaturation.

As described above in (2), an endonuclease used in the method should be selected such that it cleaves a strand at a ribonucleotide portion of a primer. Preferably, the strand is cleaved at a site 3' to the ribonucleotide. One should select a DNA polymerase such that it dissociates a nicked DNA strand at a reasonable rate.

The DNA polymerase used in the present invention should synthesize an extended strand from the nicked site towards the downstream while displacing a previously extended DNA strand. It is important that the DNA polymerase should not exhibit a 5'→3' exonuclease activity that may degrade the displaced strand. For example, Klenow fragment, which is an exonuclease-deficient variant of DNA polymerase I from E. coli, a similar fragment derived from Bst DNA polymerase (New England Biolabs), and Bca BEST DNA polymerase from B. ca (Takara Shuzo) are useful as such a DNA polymerase. Sequenase 1.0 and Sequenase 2.0 (United States Biochemical), and T5 DNA polymerase and φ29 DNA polymerase as described in Gene, 97:13-19 (1991) can also be used. A polymerase that normally has an exonuclease activity can be used in the DNA synthesis method of the present invention if addition of an appropriate inhibitor can inhibit the activity.

The method for amplifying a nucleotide sequence of the present invention may be conducted at varying temperatures or it may be conducted isothermally. Varying temperatures mean that the reaction temperatures are changed for respective steps such that the change does not interfere with the reactions in the steps. Specifically, varying temperatures refer to change in temperature to that suitable for, for example, each of annealing of a primer, synthesis reaction of a complementary strand, nicking of a complementary strand and a displacement reaction.

On the other hand, isothermal means that the reaction temperature for each step is unchanged and each step is conducted at a substantially constant temperature. It is natural to select the temperature to optimize the reaction conditions in both cases.

One feature of the method for amplifying a nucleotide sequence of the present invention is that the method does not require adjusting the temperature up and down during the nucleic acid synthesis. Thus, the present invention provides a method for isothermally synthesizing a nucleotide sequence. Many of conventional nucleic acid amplification methods require adjusting the temperature up and down to dissociate a target from a synthesized strand. These methods require a special reaction equipment such as a thermal cycler for this purpose. However, the method of the present invention can be conducted only using an equipment that can keep a constant temperature.

As described above, the method of the present invention can be conducted at a single temperature. Preferably, it is conducted by selecting the reaction temperature and the stringency level such that non-specific annealing of a primer is decreased and such that the primer specifically anneals to a nucleotide sequence as a template. Although it is not intended to limit the present invention, the method of the present invention can be conducted under high-temperature conditions by using a heat-resistant enzyme as described above. In addition, it is preferable to conduct the method of the present invention at an appropriate temperature for sufficiently retaining the activity of the enzyme used in order to maintain the reaction efficiency at high level. Thus, the reaction temperature is preferably about 20°C to about 80°C, more preferably about 30°C to about 75°C, most preferably about 50°C to about 70°C although it varies depending on the enzyme used. When the reaction is conducted under high-temperature conditions in particular, it is preferable to use a longer primer than that for a reaction at a normal temperature. The sequence and the length of the primer appropriate for the reaction temperature may be determined, for example, with reference to its Tm value. Alternatively, a commercially available software for designing a primer such as OLIGO™ Primer Analysis software (Takara Shuzo) may be used. For example, when a reaction temperature of 55°C to 60°C or 65°C is used, the primer used for the method of the present invention may be, for example, without limitations, 12-100 nucleotides in length, preferably 14-50 nucleotides in length, more preferably 15-40 nucleotides in length. An example of effects brought by the elevated reaction temperature is the solution of a problem of forming secondary structure of a DNA as a template. The elevated reaction temperature enables amplification of a desired nucleic acid even if a nucleic acid having a high GC content is used as a template. Furthermore, it is similarly effective in amplifying a region of a long chain length. Such effect is observed in a range between about 100 bp and about 20 kbp, specifically between about 200 bp and about 4.3 kbp, more specifically about 250 bp and about 1500 bp.

Use of a DNA polymerase having a reverse transcriptase activity (e.g., Bca BEST DNA polymerase) in the method of the present invention makes the amplification of a nucleotide sequence from an RNA, which comprises a step of preparing a cDNA from an RNA (a reverse transcription reaction), be conveniently conducted. Alternatively, a product obtained by independently conducting a step of preparing a cDNA from an RNA, i.e., a cDNA, can be used in the method of the present invention as the DNA as a template.

In each case, the reaction in the method of the present invention is repeated until it is terminated by appropriate means, for example, by inactivating the enzyme or by lowering the reaction temperature, or until the reaction is deprived of one of the reagents.

Figure 1 illustrates one embodiment in which a single-stranded DNA as a template and two primers are used. Respective steps, which are successively conducted in parallel, are described below:

(1) a step of annealing a single-stranded DNA as a template to a chimeric oligonucleotide primer;
(2) a step of effecting a DNA extension reaction from the 3'-terminus of the primer to form a primer-extended strand;
(3) a step of cleaving at a site that contains a ribonucleotide in the primer with an endonuclease;
(4) a step of effecting a strand displacement using a DNA polymerase from the cleavage site in step (3);
(5) a step of reusing a double-stranded DNA, which is composed of a template obtained in step (4) and a regenerated primer-extended strand, in step (3), while utilizing a released displaced strand in a reaction of step (6) and the following steps;
(6) a step annealing an oligonucleotide primer that is different from that in step (1) to the released displaced strand in step (5) as a template;

(7) a step of effecting a DNA extension reaction from the 3'-terminus of the primer to form a primer-extended strand;

(8) a step of cleaving at a site that contains a ribonucleotide in the primer with an endonuclease;

(9) a step of effecting a strand displacement using a DNA polymerase from the cleavage site in step (8); and

(10) a step of reusing a template obtained in step (9) and a regenerated primer-extended strand in step (8) .

When a double-stranded DNA is used as a template, each of the single-stranded DNAs obtained after denaturing the double-stranded DNA serves as the template in step (1). Therefore, the amount of amplification product is more than that obtained with a single-stranded DNA as a template. In addition, detection of the amplification product can be conducted in a shorter time than that required when a single-stranded DNA is used as a template.

The method for amplifying a nucleotide sequence of the present invention can be used for various experimental procedures that utilize amplification of a nucleotide sequence including detection, labeling and sequencing of a nucleic acid.

Furthermore, the method for amplifying a nucleotide sequence of the present invention can be used for an in situ nucleic acid amplification method, a method for amplifying a nucleic acid on a solid substrate such as a DNA chip, or a multiplex nucleic acid amplification method in which plural regions are simultaneously amplified.

One of the features of the method for amplifying a nucleotide sequence of the present invention is its ability to prepare a single-stranded DNA. One or two chimeric oligonucleotide primer can be used in the method for this purpose. For example, if two oligonucleotide primers are used, the method of the present invention can be conducted applying a primer ratio as used for the so-called asymmetric PCR method in which an amplification reaction is carried out using an excess amount of one oligonucleotide primer relative to another. As a result, the amount of the replacement product from one strand becomes excessive relative to that from another.

According to the method for amplifying a nucleotide sequence of the present invention, a single-stranded DNA substantially free of a complementary strand thereto can be prepared. For example, a single-stranded DNA for producing a material having an immobilized nucleic acid such as a DNA chip, a single-stranded DNA probe for detecting a target nucleic acid, or a mega-primer for the long-chain PCR method can be readily produced in a short time. Only a sense sequence or an antisense sequence can be selectively amplified by using the method of the present invention. Thus, the present invention is useful as a method for producing a nucleic acid having a sense sequence or a antisense sequence.

Furthermore, the methods for amplifying a nucleotide sequence of the preset invention does not require the use of a reaction equipment that can adjust a temperature over time. Therefore, an amplification reaction can be conducted in a large volume of reaction mixture. Thus, a nucleic acid (e.g., for medical use) can be industrially produced in large quantities.

The utilization efficiency of the primer used in the method for amplifying a nucleotide sequence of the present invention is about 100%, which may be 5- to 10-folds higher that that in a conventional method such as the PCR method.

(6) Kit for the method for amplifying nucleotide sequence of the present invention.

The present invention provides a kit used for the method for amplifying a nucleotide sequence of the first to sixth aspects as described above. In one embodiment, the kit is in a packaged form and contains instructions regarding the use of a DNA polymerase and an endonuclease in a strand displacement reaction. Also, a kit that contains a DNA polymerase having a strand displacement activity, an endonuclease, and a buffer for a strand displacement reaction is preferably used for the method of the present invention. Alternatively, a commercially available DNA polymerase having a strand displacement activity and/or endonuclease may be selected according to the instructions and used. Additionally, the kit may contain a reagent for a reverse transcription reaction that is used when an RNA is used as a template. The DNA polymerase can be selected from the DNA polymerases to be used in the present invention as described above in (3). The endonuclease can be selected from the endonucleases as described above in (2). One having the reaction buffer composition as described above in (4) can be preferably used as the buffer for the strand displacement reaction.

"Instructions" are printed matter describing a method of using the kit, for example, a method for preparing a reagent solution for a strand displacement reaction, recommended reaction conditions and the like. The instructions include an instruction manual in a form of a pamphlet or a leaflet, a label stuck to the kit, and description on the surface of the package containing the kit. The instructions also include information disclosed or provided through electronic media such as the Internet.

(7) Method for detecting nucleotide sequence of the present invention and kit for the method.

A target nucleic acid in a sample can be detected by using the method for amplifying a nucleotide sequence of the present invention. The detection method comprises:

(a) amplifying a target nucleic acid by the method for amplifying a nucleotide sequence of the present invention as described above; and

(b) detecting the target nucleic acid amplified in the step above.

The method can be utilized to detect or quantify a specific gene in a sample. In other words, a specific gene can be detected or quantified form all samples suspected to contain a nucleic acid such as a DNA or an RNA. Examples of the samples from which a specific gene can be detected or quantified include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a micoorganism such as a virus or a bacterium (e.g., a food or a biological formulation), and samples that may contain an organism such as a soil and a waste water. For example, a viroid, a virus, a fungi, a bacterium or other microorganisms in a sample can be detected or quantified on the basis of the presence or the content of a specific gene derived from the viroid, the virus, the fungi, the bacterium or the other microorganisms as a target. Furthermore, the method of the present invention can be used to distinguish a genotype of an organism or to determine the expression level of a gene. Both of an RNA and a DNA can be preferably used as the nucleic acid as the template in the detection method.

Known methods for detecting a nucleic acid can be used for step (b). Examples of such methods include detection of a reaction product having a specific size by electrophoresis, and detection by hybridization with a probe. A fluorescent substance such as ethidium bromide is used in the detection by electrophoresis. The hybridization with a probe may be combined with the detection by electrophoresis. The probe may be labeled with a radioisotope or with a non-radioactive substance such as biotin or a fluorescent substance. Additionally, use of a labeled nucleotide in step (a) may facilitate the detection of amplification product. A fluorescence polarization method, a fluorescence energy transition or the like can also be utilized for the detection. The target nucleic acid can be detected automatically or quantified by constructing a suitable detection system.

A ribonucleotide (RNA) probe labeled with two or more fluorescent substances positioned at a distance that results in a quenching state can be used in the detection method of the present invention. The probe does not emit fluorescence. When it is annealed to a DNA amplified from a target nucleic acid that is complementary to the probe, RNase H digests the probe. The distance between the fluorescent substances on the probe then increases, resulting in the emission of fluorescence. Thus, the emission reveals the presence of the target nucleic acid. If RNase H is used in the method for amplifying a nucleotide sequence of the present invention, a target nucleic acid can be detected only by adding a probe to the reaction mixture. For example, a combination of fluorescent substances, 6-carboxyfluorescein (6-FAM) and N,N,N',N'-tetramethyl-6-carboxyrhodamine (TAMRA), can be preferably used for labeling the probe.

The method for amplifying a nucleotide sequence under isothermal conditions of the present invention does not require the use of an equipment such as a thermal cycler. The number of primers used in the amplification method of the present invention can be one or two, which is less than that used in a conventional method. Since reagents such as dNTPs used for PCR and the like can be applied to the method of the present invention, the running cost can be lowered as compared with a conventional method. Therefore, the method of the present invention can be preferably used in a field including genetic test in which the detection is routinely conducted. The method of the present invention provides a larger amount of an amplification product in a shorter time than the PCR method. Therefore, the method of the present invention can be utilized as a convenient, rapid and sensitive method for detecting a gene.

The present invention further provides a kit used for the method for detecting a target nucleic acid. The kit for the method for amplifying a nucleotide sequence of the present invention as described above can be used as this kit. The kit may further contain a chimeric oligonucleotide primer for amplifying the target nucleic acid and a reagent for detecting the amplified target nucleic acid such as a probe.

(8) Material having immobilized nucleic acid arrayed in predefined region of the present invention and method for producing the same.

A DNA chip (also referred to as a DNA microarray or a DNA array) is a material having an immobilized nucleic acid in which various fragments of genes or DNAs are arrayed and immobilized in a predefined region or at a predefined position on a solid substrate such as a slide glass. The DNA chip is used for examining the presence of a nucleic acid in a nucleic acid sample that has a sequence complementary to an arrayed and immobilized DNA in a predefined region on the DNA chip. The examination is carried out by contacting the DNA chip with the nucleic acid sample prepared from a sample, preferably a labeled nucleic acid sample, for hybridization. Since the DNA chip can be used to detect or quantify a number of nucleic acids in a sample in one procedure, it is a very useful means that dramatically promotes the analysis of gene expression, or the analysis of a mutation or polymorphism. A DNA chip in which a double-stranded nucleic acid is arrayed and immobilized in a predefined region is used for hybridization after it is subjected to appropriate denaturation. A DNA chip in which a single-stranded DNA complementary to a target nucleic acid to be detected is arrayed and immobilized in a predefined region is particularly preferable for the detection of a target nucleic acid.

As described above, a desired DNA can be amplified in a single-stranded form by the method of the present invention. Although any method for purifying an amplification product can be used, purification using isopropanol precipitation is preferable. The thus obtained DNA, preferably a single-stranded DNA substantially free of a complementary strand thereto, can be preferably used as a DNA fragment to be immobilized onto a DNA chip. Thus, the method of the present invention is preferably used as a method for preparing a DNA to be arrayed and immobilized in a predefined region for producing a DNA chip. Any insoluble substrate can be used as a substrate onto which the thus obtained DNA is arrayed and immobilized in a predefined region, but a plate-shaped substrate made from glass or plastic, and a membrane-shaped substrate made from nitrocellulose or nylon are preferably used. A known method for immobilizing a nucleic acid can be used for the immobilization. The DNA may be immobilized onto a substrate as it is. Alternatively, the DNA may be immobilized through a suitable linker or after ligating plural DNA molecules.

A target nucleic acid that hybridizes with a nucleic acid on a material having an immobilized nucleic acid in which a DNA amplified by the method of the present invention is arrayed and immobilized in a predefined region (e.g., a DNA chip) can be detected or quantified. Such detection or quantification can be accomplished by contacting the material with a nucleic acid sample prepared from a sample suspected to contain the target nucleic acid for hybridization. Among these, a DNA chip in which a single-stranded DNA amplified by the method of the present invention is arrayed and immobilized in a predefined region allows the detection of a target nucleic acid with more convenient operation, higher sensitivity and higher reproducibility as compared with a conventional material.

(9) Method for producing nucleic acid in large quantities of the present invention.

As described above, one aspect of the present invention provides a method for amplifying a nucleotide sequence that can be carried out under isothermal conditions. A desired nucleic acid can be produced in the method by mixing a nucleic acid as a template for the nucleic acid to be amplified and various components required for a reaction and reacting the mixture under isothermal conditions. Since the PCR method requires changing the temperature of the reaction mixture over time, the reaction volume is limited to one in which the temperature can be controlled (usually, 200 μl or less). Therefore, it is difficult to scale up the volume. On the other hand, there is no such limitation in the method of the present invention. A large amount of nucleic acid can be produced by increasing the volume of the reaction mixture. In the method of the present invention, a number of complementary strand molecules are synthesized from one template molecule. Furthermore, nucleic acids can be synthesized using these complementary strand molecules as templates. Thus, a desired nucleic acid can be efficiently produced in large quantities by suitably selecting the template and the primer. Additionally, the fact that, unlike the PCR method, the method of the present invention does not require a special equipment or a complicated temperature change makes it advantageous in view of the cost of equipment and energy. Therefore, the method is an excellent industrial method for producing a nucleic acid in large quantities.

Furthermore, the method of the present invention is useful as a method for supplying a variety of DNA fragments in large quantities, such as those to be immobilized onto the DNA chip. Specifically, DNA fragments can be obtained in large quantities in simple reaction steps in one embodiment. In another embodiment, a limited number of primers can be used to obtain a variety DNA fragments. A step of amplifying the nucleic acid that serves as the template in the method of the present invention beforehand by a known nucleic acid amplification method such as the PCR method can be incorporated in the latter embodiment. All kinds of nucleic acids as templates can be amplified using a limited number of primers, for example, based on the method for amplifying a nucleic acid using a random primer having a tag sequence (Nucleic Acids Research, 24(19):3778-3783 (1996)) or the degenerate oligonucleotide-primed PCR (DOP-PCR; Genomics, 13:718-725 (1992)), which uses a degenerate primer. The amplification method of the present invention can be conducted using one or several primers for all of the nucleic acids as templates produced in the above-mentioned step. This can be accomplished by designing the primer used in the amplification method of the present invention such that it corresponds to the tag sequence added to the random or degenerate primer. Thus, a combination of a suitable step for preparing a nucleic acid as a template and the method of the present invention can supply a variety of DNA fragments in larger quantities and at a lower cost as compared with a conventional method.

A pharmaceutical composition containing a nucleic acid may contain a double-stranded DNA for expressing a useful polypeptide in a cell or a single-stranded antisense DNA for suppressing the expression of a gene of interest. Such a nucleic acid is administered into an organism using a suitable means, for example, a carrier for gene transfer such as liposome. The method for producing a nucleic acid of the present invention is preferable for producing a single-stranded or double-stranded nucleic acid for medical use in large quantities. Additionally, a nucleic acid containing a dNTP analog that, for example, suppresses the degradation of the nucleic acid in vivo can be readily produced by the method of the present invention.

Since the DNA fragment amplified in the present invention is composed of normal nucleotides, the amplified DNA can be subcloned into a suitable vector utilizing a restriction enzyme site in the DNA. Furthermore, the DNA can be treated with a restriction enzyme for RFLP without a problem, for example. Therefore, the DNA can be widely utilized in a field of genetic test. Since the DNA fragment amplified in the present invention is composed of normal nucleotides, a promoter sequence for an RNA polymerase can be incorporated into the amplified fragment. The amplified fragment can be used as a template to synthesize an RNA, which can be used as a probe, for example. Of course, a fluorescence-labeled DNA probe can be produced by conducting the method for amplifying a nucleotide sequence of the present invention using a fluorescence-labeled dNTP instead of a normal dNTP.

Since the finally amplified fragment in the method of the present invention does not have a nucleotide sequence complementary to a primer for amplification on both ends, contamination due to the carry-over of an amplification product can be reduced. Therefore, the method of the present invention is useful in genetic test and the like in which the same region is amplified routinely.

Features of the method for amplifying a nucleotide sequence of the present invention are listed below.

1. It can amplify a large amount of a nucleic acid from a small amount of a template. The amplification product increases quadratically when two primers are used.
2. It can be conducted isothermally. In this case, it does not require the use of an equipment such as a thermal cycler. Therefore, the reaction volume can be readily scaled up.
3. Usually, the amplification reaction is conducted using one or two chimeric oligonucleotide primer and two enzymes (a DNA polymerase and an endonuclease).
4. Since a number of DNA strands are synthesized from one molecule of a primer, the amount of the primer does not restrict the amount of the amplification product. Furthermore, the primer utilization efficiency is about 100%, which is very higher than that of the PCR method.
5. A single-stranded or double-stranded DNA can be selectively amplified depending on the purpose.
6. Since it does not require a dNTP analog such as an (α-S) dNTP for the amplification reaction, the cost of reagents is low. Furthermore, a nucleic acid in a natural form without a dNTP analog can be obtained.
7. It can supply an amplified DNA fragment at low cost and in large quantities by combining the method of the present invention with another nucleic acid amplification method.

As described above, the method of the present invention is suitable for producing a nucleic acid on an industrial scale.

Examples

The following Examples illustrate the present invention in more detail, but are not to be construed to limit the scope thereof.

Referential Example

Unit value of RNase H used in the method of the present invention was measured according to the following method.

(1) Preparation of reagent solutions used

Reaction mixture for determining activity: The following substances at the indicated final concentrations were contained in sterile water: 40 mM tris-hydrochloride (pH 7.7 at 37°C), 4 mM magnesium chloride, 1 mM DTT, 0.003% BSA, 4% glycerol and 24 μM poly(dT).

Poly[8-$^3$H]adenylic acid solution: 370 kBq of a poly[8-$^3$H]adenylic acid solution was dissolved in 200 μl of sterile water.

Polyadenylic acid solution: Polyadenylic acid was diluted to a concentration of 3 mM with sterile ultrapure water.

Enzyme dilution solution: The following substances at the indicated final concentrations were contained in sterile water: 25 mM tris-hydrochloride (pH 7.5 at 37°C), 5 mM 2-mercaptoethanol, 0.5 mM EDTA (pH 7.5 at 37°C), 30 mM sodium chloride and 50% glycerol.

Preparation of heat-denatured calf thymus DNA: 200 mg of calf thymus DNA was suspended and swelled in 100 ml of TE buffer. The solution was diluted to a concentration of 1 mg/ml with sterile ultrapure water based on the absorbance measured at UV 260 nm. The diluted solution was heated at 100°C for 10 minutes and then rapidly cooled in an ice bath.

(2) Method for measuring activity

7 μl of the poly[8-$^3$H]adenylic acid solution was added to 985 μl of the reaction mixture for determining activity prepared in (1) above. The mixture was incubated at 37°C for 10 minutes. 8 μl of polyadenylic acid was added to the mixture to make the final concentration to 24 μM. The mixture was further incubated at 37°C for 5 minutes. Thus, 1000 μl of a poly [8-$^3$H]rA-poly-dT reaction mixture was prepared. 200 μl of the reaction mixture was then incubated at 30°C for 5 minutes. 1 μl of an appropriate serial dilution of an enzyme solution was added thereto. 50 μl each of samples was taken from the reaction mixture over time for use in subsequent measurement. The period of time in minutes from the addition of the enzyme to the sampling is defined as Y. 50 μl of a reaction mixture for total CPM or for blank was prepared by adding 1 μl of the enzyme dilution solution instead of an enzyme solution. 100 μl of 100 mM sodium pyrophosphate, 50 μl of the heat-denatured calf thymus DNA solution and 300 μl of 10% trichloroacetic acid (300 μl of ultrapure water for measuring total CPM) were added to the sample. The mixture was incubated at 0°C for 5 minutes, and then centrifuged at 10000 rpm for 10 minutes. After centrifugation, 250 μl of the resulting supernatant was placed in a vial. 10 ml of Aquasol-2 (NEN Life Science Products) was added thereto. CPM was measured in a liquid scintillation counter.

(3) Calculation of units

Unit value for each enzyme was calculated according to the following equation.

$$\text{Unit/ml} = \{(\text{measured CPM} - \text{blank CPM}) \times 1.2^{*} \times 20 \times 1000 \times \text{dilution rate}\}\ 200\ (\mu l) / (\text{total CPM} \times Y\ (\text{min.}) \times 50\ (\mu l) \times 9^{**})$$

1.2*: Amount in nmol of poly[8-$^3$H]rA-poly-dT contained in total CPM per 50 μl.
9**: Correction coefficient.

Example 1

(1) Synthesis of template DNA and primers

A single-stranded DNA of 99 bases as a template and primers used in this Example were synthesized using a DNA synthesizer (Applied Biosystems). The nucleotide sequence of the single-stranded DNA of 99 bases is shown in SEQ ID NO:1 of the Sequence Listing. Basic nucleotide sequences of an upstream primer and a downstream primer are shown in SEQ ID NOS:2 and 3 of the Sequence Listing, respectively. The structures of the primers used in this Example are described below in detail:

Primer Pair 1: A combination of primers having a nucleotide sequence as shown in SEQ ID NO:2 or 3 of the Sequence Listing and wholly composed of deoxyribonucleotides;
Primer Pair 2: A combination of primers in which the first and second deoxyribonucleotides from the 3'-terminus are replaced by ribonucleotides and the phosphate bond on the 5'-terminal side of the second ribonucleotide from the 3'-terminus is replaced by a phosphorothioate bond in each of the primers of the Primer Pair 1;
Primer Pair 3: A combination of primers in which the deoxyribonucleotide at the 3'-terminus is replaced by a ribonucleotide and the phosphate bond on the 5'-terminal side of the ribonucleotide is replaced by a phosphorothioate bond in each of the primers of the Primer Pair 1;
Primer Pair 4: A combination of primers in which the first and second deoxyribonucleotides from the 3'-terminus are replaced by ribonucleotides in each of the primers of the Primer Pair 1; and
Primer Pair 5: A combination of primers in which the third and fourth deoxyribonucleotides from the 3'-terminus are replaced by ribonucleotides and the phosphate bond on the 5'-terminal side of the fourth ribonucleotide from the 3'-terminus is replaced by a phosphorothioate bond in each of the primers of the Primer Pair 1.

(2) Amplification reaction

Bca BEST DNA polymerase (Takara Shuzo), which is a DNA polymerase lacking a 5'→3' exonuclease activity from Bacillus caldotenax, and cloned ribonuclease H (Takara Shuzo), which is RNase H from E. coli, were used to examine the reaction systems of Models 1 to 7 as described below.

A reaction mixture was prepared as follows.

35 mM tris-hydrochloride buffer (pH 7.5), 0.1 mg/ml bovine serum albumin (BSA), 2.7% glycerol, 5% dimethyl sulfoxide, 1.4 mM each of dNTPs, 10 mM magnesium chloride, 20 pmol of one or both of the primers of one of the primer pairs as described above in (1), 0.6 ng of the synthetic single-stranded DNA as the template, 5 U of Bca BEST DNA polymerase and 60 U of cloned ribonuclease H, the final reaction volume being 50 μl. The reaction mixture was mixed to homogeneity, incubated at 55°C for 60 minutes, and then heated at 90°C for 2 minutes to inactivate the enzymes. 8 μl of the reaction mixture was subjected to electrophoresis on 3% NuSieve 3:1 agarose (Takara Shuzo) gel. The primers used in the respective Models are described below:

Models 1-5: One of the Primer Pairs 1-5 was used;
Model 6: Only the downstream primer of the Primer Pair 2 was used; and
Model 7: The Primer Pair 4 was used without the addition of RNase H.

As a result, amplified fragments having a size of interest ranging from about 40 base pairs (bp) to about 90 bp were observed when the reaction mixtures of the Models 2 to 5 were used, indicating that DNAs are amplified using these reaction systems. An amplified fragment having an expected size of about 70 bases (b) (a single-stranded DNA fragment) was observed for the Model 6 in which only one of the two primers was used. No DNA amplification was observed for the reaction of the Model 1 or 7.

(3) Confirmation of amplification products

The reaction mixture obtained by the reaction of the Model 4 as described in (2) was filtrated using Microcon-100 (Takara Shuzo) to recover an amplified DNA fragment entrapped on the filter. The nucleotide sequence of the DNA fragment was determined by the dideoxy method. As a result, the fragment amplified by the above-mentioned reaction was confirmed to be a DNA having the same nucleotide sequence as that of the DNA as the template.

(4) Examination of reaction time

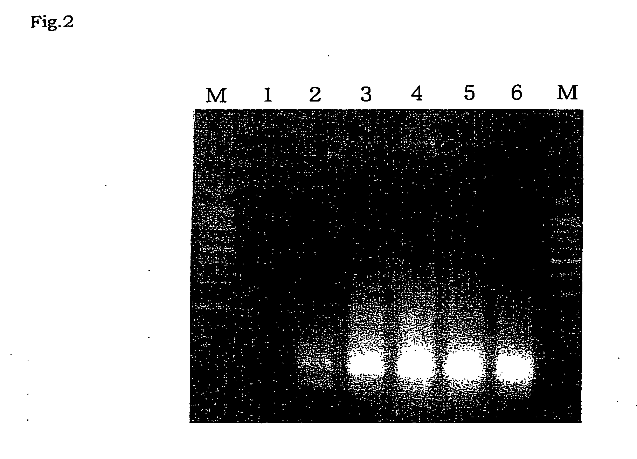

The reaction mixture of the Model 2 as described above in (2) was prepared to study the change in the amount of the amplification product when it was reacted for a varying time. The reaction mixture was incubated for 0, 15, 30, 60, 90 or 120 minutes at 55°C. The mixture was then treated at 90°C for 2 minutes to inactivate the enzymes. 8 μl of the reaction mixture was analyzed by electrophoresis on 3% NuSieve 3:1 agarose gel. The results of the electrophoresis are shown in Figure 2. Numbers 1 to 6 in the figure represent lanes to which the reaction mixture reacted for 0, 15, 30, 60, 90 or 120 minutes was applied, respectively. M represents a lane to which 100 bp DNA ladder marker (Takara Shuzo) was applied as a molecular weight marker.

As shown in Figure 2, no amplification product was observed for a reaction time of 0 minute. It was confirmed that the amount of the amplification product increased as the reaction time became longer from 15 minutes to 30 or 60 minutes. However, the amount of the amplification product as observed by electrophoresis was almost unchanged for a reaction time of 60 minutes or longer, indicating that the amplification in the reaction system used reached the plateau at about 60 minutes.

Example 2

(1) Preparation of RNA

An RNA used as a template in this Example was prepared from human cultured cell HT29 (ATCC HTB-38) (Dainippon Pharmaceutical) using TRIzol reagent (Life Technologies). The concentration of the resulting total RNA was adjusted to 1 μg/μl. The OD260/OD280 value was 1.8, which indicates the spectrophotometric purity of the RNA.

(2) Amplification reaction

Bca BEST DNA polymerase, which has a reverse transcription activity and a DNA polymerase activity, as well as RNase H endonuclease were used to determine if a cDNA is amplified from an RNA.

A reaction mixture having the composition as described in Example 2 was prepared with the addition of 1 μg of the above-mentioned total RNA. A targeted region encoding human transferrin receptor (GenBank accession no. X01060) was amplified using the Primer Pair 2 in Example 1 as primers.

The reaction mixture was incubated at 55°C for 60 minutes, and then heated at 90°C for 2 minutes to inactivate the enzymes. When 8 μl of the reaction mixture was subjected to electrophoresis on 3% NuSieve 3:1 agarose gel, an amplified fragment having an expected size of 56 bp was observed. Furthermore, Southern hybridization was carried out using a probe having the targeted nucleotide sequence. A DNA probe having a nucleotide sequence as shown in SEQ ID NO:4 of the Sequence Listing labeled with biotin at the 5'-terminus was used to conduct Southern hybridization. As a result, the probe hybridized with the above-mentioned amplified fragment, confirming that the targeted region was correctly amplified by the method of the present invention.

Example 3

(1) Synthesis of primers

The amplification method of the present invention was examined using a double-stranded DNA as a template. Primers used were synthesized using a DNA synthesizer (Applied Biosystems). Basic nucleotide sequences of primers are shown in SEQ ID NOS:5-13 of the Sequence Listing. The structures of the primers used in this Example are described below in detail. The pUC19 DNA (Takara Shuzo) was used as a template for the Primer Pairs A-F. The nucleotide sequence of pUC19 is available from a database (GenBank accession no. L09137). An amplified double-stranded DNA fragment was used as a template for the Primer Pair G. The fragment was prepared from the human total RNA obtained in Example 2 using primers having a sequence as shown in SEQ ID NOS:14 or 15 of the Sequence Listing and TaKaRa RNA PCR Kit (AMV) Ver. 2.1 (Takara Shuzo) according to the attached standard protocol.

Primer Pair A (amplified fragment length: about 450 bp): A combination of primers having a nucleotide sequence as shown in SEQ ID NO:5 or 6 of the Sequence Listing in which the first and second bases from the 3'-terminus are replaced by ribonucleotides;

Primer Pair B (amplified fragment length: about 250 bp): A combination of primers having a nucleotide sequence as shown in SEQ ID NO:5 or 7 of the Sequence Listing in which the first and second bases from the 3'-terminus are replaced by ribonucleotides;

Primer Pair C (amplified fragment length: about 520 bp): A combination of primers having a nucleotide sequence as shown in SEQ ID NO:5 or 8 of the Sequence Listing in which the first and second bases from the 3'-terminus are replaced by ribonucleotides;

Primer Pair D (amplified fragment length: about 890 bp): A combination of primers having a nucleotide sequence as shown in SEQ ID NO:5 or 9 of the Sequence Listing in which the first and second bases from the 3'-terminus are replaced by ribonucleotides;

Primer Pair E (amplified fragment length: about 130 bp): A combination of primers having a nucleotide sequence as shown in SEQ ID NO:10 or 6 of the Sequence Listing in which the first to third bases from the 3'-terminus are replaced by ribonucleotides;

Primer Pair F (amplified fragment length: about 220 bp): A combination of primers having a nucleotide sequence as shown in SEQ ID NO:11 or 6 of the Sequence Listing in which the first to third bases from the 3'-terminus are replaced by ribonucleotides; and

Primer Pair G (amplified fragment length: about 320 bp): A combination of primers having a nucleotide sequence as shown in SEQ ID NO:12 or 13 of the Sequence Listing in which the first to third bases from the 3'-terminus are replaced by ribonucleotides.

(2) Amplification reaction

A reaction mixture was prepared as follows.

35 mM potassium phosphate buffer (pH 7.5), 0.1 mg/ml bovine serum albumin (BSA), 5% dimethyl sulfoxide, 1.4 mM each of dNTPs, 10 mM magnesium chloride, 60 pmol each of the primers of one of the primer pairs as described above in (1), 100 ng of the pUC19 DNA as the template, 5.5 U of Bca BEST DNA polymerase and 60 U of RNase H to a final reaction volume of 50 μl.

The reaction conditions were as follows. The reaction mixture without the DNA polymerase or RNase H was heat-denatured at 98°C for 1 minute, and then cooled to 55°C. The DNA polymerase and RNase H were then added thereto and the mixture was incubated at 55°C for 60 minutes. After the completion of the reaction, the mixture was heated at 90°C for 2 minutes to inactivate the enzymes. 8 μl of the reaction mixture was then subjected to electrophoresis on 3% NuSieve 3:1 agarose gel.

As a result, it was confirmed that an amplified fragment of interest was obtained using either of the Primer Pairs. Thus, it was confirmed that a double-stranded DNA can be used as a template to conduct an amplification reaction in the amplification method of the present invention.

(3) Digestion of amplification product with restriction enzyme

Digestion of an amplified fragment obtained using the amplification method of the present invention with a restriction enzyme was examined. The pUC19 plasmid DNA was used as a template DNA. pUC19 upper (2) NN primer and pUC19 lower NN primer as shown in SEQ ID NOS:5 and 6 of the Sequence Listing, respectively, were used. In the primers, the first and second bases from the 3'-terminus are replaced by ribonucleotides. The composition of the reaction mixture was as follows.

Reaction Mixture A: 35 mM potassium phosphate buffer (pH 7.5), 10 mM magnesium chloride, 1.4 mM each of dNTPs, 0.01% BSA, 5% DMSO, 2.7% glycerol, 100 pmol each of the pUC19 upper (2) NN primer and the pUC19 lower NN primer, 500 ng of the pUC19 DNA and sterile distilled water to a reaction volume of 48 μl.

The reaction mixture was heat-denatured at 98°C for 1 minute, and then cooled to 55°C. 60 U of E. coli RNase H and 5.5 U of Bca BEST were then added thereto to make the reaction volume to 50 μl. The reaction mixture was incubated at 55°C for 1 hour. After the completion of the reaction, the mixture was heated at 90°C for 2 minutes to inactivate the enzymes. The reaction mixture was subjected to electrophoresis on 3% agarose gel to purify the resulting amplification product. The recovered amplification product was resupended in 100 μl of sterile distilled water.

The thus obtained DNA solution was used for restriction enzyme digestion. Restriction enzymes used were AccII (Takara Shuzo) and BcnI (Takara Shuzo). The composition of the reaction mixture was as follows.

3 μl of the DNA solution, 1 μl of 10 x AccII buffer or 10 x BcnI buffer attached to each of the enzymes, 1 μl of the restriction enzyme AccII or BcnI and sterile distilled water to a reaction volume of 10 μl. The reaction mixture was reacted at 37°C. for 30 minutes. 1.5 μl of 10 x loading buffer was added thereto. 6 μl of the mixture was subjected to electrophoresis on 3% NuSieve agarose gel.

As a result, restriction enzyme-digested DNA fragments of interest were obtained using both of the restriction enzymes AccII and BcnI.

(4) Detection of mutation

Detection of a mutation using the amplification method of the present invention was examined. pUC19 was used as a template. Basic nucleotide sequences of pUC19 upper (2) NN primer and pUC19 lower NN primer are shown in SEQ ID NOS:5 and 6 of the Sequence Listing, respectively. Both of these primers are chimeric oligonucleotide primers in which the first and second bases from the 3'-terminus are replaced by ribonucleic acids. Additionally, four primers in which the base at the 3'-terminus of the pUC19 upper (2) NN primer is replaced by U (which is complementary to the corresponding base in the template), or A, C or G (which is a mismatched base) designated as pUC19 upper (2) NN-U, pUC19 upper (2) NN-A, pUC19 upper (2) NN-C or pUC19 upper (2) NN-G, respectively, were made. The combinations of these primers were as follows.

Primer Pair 1: pUC19 upper (2) NN-U and pUC19 lower NN;
Primer Pair 2: pUC19 upper (2) NN-A and pUC19 lower NN;
Primer Pair 3: pUC19 upper (2) NN-C and pUC19 lower NN; and
Primer Pair 4: pUC19 upper (2) NN-G and pUC19 lower NN.

A reaction mixture was prepared as follows.

30 mM potassium phosphate buffer (pH 7.3), 0.01% bovine serum albumin (BSA), 5% DMSO, 1 mM each of dNTPs, 8 mM magnesium acetate, 60 pmol each of the primers, 50 ng of the DNA as the template and sterile distilled water to a reaction volume of 48 μl.

The reaction mixture was heat-denatured at 98°C for 1 minute, and then cooled to 55°C. 5.5 U of Bca BEST DNA polymerase and 60 U of E. coli RNase H were then added thereto and the reaction mixture was incubated at 55°C for 60 minutes. The mixture was then heated at 90°C for 2 minutes to inactivate the enzymes. 8 μl of the reaction mixture was subjected to electrophoresis on 4% NuSieve 3:1 agarose (Takara Shuzo) gel. As a result, an amplified fragment of about 450 bp of interest was detected only when the combination of the primers that included the primer having a complementary base at the 3'-terminus of pUC19 upper (2) NN was used. On the other hand, no amplified fragment was observed for the combinations including the primer having a mismatched base at the 3'-terminus of pUC19 upper (2) NN.

Example 4

(1) Reaction in microtube

Reaction volume for the amplification method of the present invention was examined. A region encoding human transferrin receptor was selected as a region to be amplified. Primers having a sequence as shown in SEQ ID NO:12 or 13 of the Sequence Listing were used. In the primers, the first and second bases from the 3'-terminus are replaced by ribonucleotides. A fragment of about 750 bp amplified by RT-PCR was used as a template DNA. The reaction volume was adjusted to 50, 100, 300 or 500 μl. The composition of the reaction mixture was as follows.

Reaction Mixture A: 10 μl of 5 x specialized buffer (135 mM potassium phosphate buffer (pH 7.5), 0.5 mg/ml BSA, 2.5% DMSO), 4 μl of 100 mM magnesium acetate, 5 μl of 10 mM dNTPs, 10 μl of 10 μM ATP, 1 μl of Bca BEST DNA polymerase (22 U/μl), 1 μl of RNase H (60 U/μl) and sterile distilled water to 39 μl.

Reaction Mixture B: 3 μl each of 20 μM human transferrin receptor S primer (SEQ ID NO:12) and 20 μM human transferrin receptor primer (SEQ ID NO:13), about 100 ng of the DNA as the template and sterile distilled water to 11 μl. If the volume became 50 μl or more, it was scaled up to have the above-mentioned composition.

For an amplification reaction, the Reaction Mixture B was treated at 98°C for 2 minutes, and then incubated at 55°C for 3 minutes. The Reaction Mixture B was added to the Reaction Mixture A which had been preincubated in a 1500-μl microtube at 55°C. After mixing, the reaction mixture was incubated at 55°C for 1 hour. After the completion of the reaction, the mixture was transferred to an ice bath. 8 μl of the reaction mixture was subjected to electrophoresis on 3% agarose gel.

As a result, a fragment of about 300 bp of interest was efficiently amplified using each of the reaction volumes. In addition, it was confirmed that an amplified fragment of interest can be obtained without a problem using a PCR-amplified fragment as a template DNA.

(2) Reaction in Petri dish

Use of a Petri dish for preventing the heterogeneous temperature in a reaction mixture due to increased reaction volume was examined. A region encoding human transferrin receptor was selected as a region to be amplified. Primers having a sequence as shown in SEQ ID NO:12 or 13 of the Sequence Listing were used. In the primers, the first and second bases from the 3'-terminus are replaced by ribonucleotides. A fragment of about 750 bp amplified by RT-PCR was used as a template DNA. The reaction volume was adjusted to 10 ml. The composition of the reaction mixture was as follows.

Reaction Mixture A: 2000 μl of 5 x specialized buffer (135 mM potassium phosphate buffer (pH 7.5), 0.5 mg/ml BSA, 2.5% DMSO), 800 μl of 100 mM magnesium acetate, 1000 μl of 10 mM dNTPs and sterile distilled water to 9.1 ml.

Reaction Mixture B: 200 μl each of 60 μM human transferrin receptor S primer (SEQ ID NO:12) and 60 μM human transferrin receptor primer (SEQ ID NO:13), about 10 μg of the DNA as the template and sterile distilled water to 500 μl.

Reaction Mixture C: 200 μl of Bca BEST DNA polymerase (22 U/μl) and 200 μl of RNase H (60 U/μl).

For an amplification reaction, the Reaction Mixture B was treated at 98°C for 1 minute, and then incubated at 55°C for 3 minutes. The Reaction Mixture B was added to the Reaction Mixture A which had been preincubated in a 60-mm (diameter) plastic Petri dish at 55°C. The Reaction Mixture C was further added thereto. After mixing, the reaction mixture was incubated at 55°C for 1 hour. After the completion of the reaction, the reaction mixture was transferred to an ice bath. 8 μl of the reaction mixture was then subjected to electrophoresis on 3% agarose gel.

As a result, a fragment of about 300 bp of interest was efficiently amplified even if the reaction volume of 10 ml was used. In addition, it was confirmed that an amplified fragment of interest can be obtained without a problem using a PCR-amplified fragment as a template DNA. Thus, it was confirmed that the method of the present invention can be more preferably used for making a DNA chip, which requires a large amount of a DNA fragment, as compared to the conventional PCR method.

Example 5

(1) Relationship between type of buffer and amount of RNase H used

The relationship between the type of buffer and the amount of RNase H used was examined. Plasmid DNAs, in which a fragment of 249 bp or 911 bp was cloned into the pUC19 vector (designated as pUC19-249 and pUC19-911) were used as templates. Chimeric oligonucleotide primers, in which the first to third bases from the 3'-terminus of MF2N3 (24) primer or MR1N3 (24) primer having a sequence as shown in SEQ ID NO:16 or 17 of the Sequence Listing are replaced by ribonucleotides, were used as primers. By using the combination of these primers, amplified fragments of about 450 bp and about 1100 bp are obtained for pUC19-249 and pUC19-911, respectively.

A tris-hydrochloride buffer, a potassium phosphate buffer and Tricine buffer were selected as buffer systems to be examined. The amounts of RNase H examined were no addition and a final concentration ranging from 0.3 to 1.2 U/μl. The tris-hydrochloride buffer system was prepared as described in Example 1 (2), except that 10 ng of pUC19-249 or 200 ng of pUC19-911, 60 pmol each of the primers and 11 U/50 μl reaction volume of Bca BEST DNA polymerase were used. The potassium phosphate buffer system was prepared to have a similar composition. The Tricine buffer system was prepared to contain the following at the indicated final concentration: 34 mM Tricine buffer (pH 8.7), 10 mM potassium chloride, 10 mM ammonium sulfate, 0.01% BSA, 1% DMSO, 4 mM magnesium acetate and 0.5 mM each of dNTPs. 10 ng/50 μl reaction volume of the pUC19-249 plasmid or 200 ng/50 μl reaction volume of the pUC19-911 plasmid, 60 pmol/50 μl reaction volume each of primers, RNase H at a predetermined concentration and 11 U/50 μl reaction volume of Bca BEST DNA polymerase were added to the buffer system.

For an amplification reaction, a mixture of pUC19-249 or pUC19-911 as a template and the respective primers was heat-denatured at 98°C for 1 minute, and then cooled to 55°C. A mixture of the remaining reaction components was added thereto. The mixture was reacted at 55°C for 60 minutes. After the completion of the reaction, the mixture was cooled to 4°C and 1/10 volume of 0.5 M EDTA was added thereto to terminate the reaction. 3 μl of the reaction mixture was subjected to electrophoresis on 3% NuSieve 3:1 agarose (Takara Shuzo) gel.

As a result, when pUC19-249 was used as a template, increase in amplification efficiency was observed depending on the buffer system used in the following order: tris-hydrochloride < potassium phosphate < Tricine. When pUC19-911 was used as a template, increase in amplification efficiency was observed depending on the buffer system used in the following order: tris-hydrochloride < Tricine < potassium phosphate. The use of RNase H at a final concentration ranging from 0.3 to 1.2 U/μl resulted in the amplified fragment of interest, although no amplified fragment of interest was observed for no addition.

(2) Examination of amount of primer

The effect of the amount of a primer used on the amplification method of the present invention was examined. A reaction mixture system having a composition in which pUC19-249 was used as a template among the compositions as described above in (1) was used. 60 U/50 μl reaction volume of RNase H was used for the potassium phosphate buffer system, whereas 30 U/50 μl reaction volume of RNase H was used for the tris-hydrochloride or Tricine buffer system. The examined concentration of the primer ranged from 10 to 100 pmol/50 μl. Reaction conditions and confirmation of amplification were as described above in (1).

As a result, an amplified fragment of interest was observed using each of the reaction buffer systems containing the primer at a concentration ranging from 10 to 100 pmol/50 μl.

(3) Effect of pH of reaction buffer

The effect of the pH of a reaction mixture on the amplification method of the present invention was examined. The composition of the reaction mixture was as described above in (2). The pH examined were 7.0-8.0 for the potassium phosphate buffer system, 7.5-9.2 for the Tricine buffer system, and 7.5-9.0 for the tris-hydrochloride buffer system. Reaction conditions and confirmation of amplification were as described above in (1).

As a result, an amplified fragment of interest was observed at pH within the range used for the respective buffer systems.

(4) Effect of additive

The effect of addition of dimethyl sulfoxide (DMSO) was examined using the reaction mixture composition of the phosphate buffer system (pH 7.5) as described above in (3). Additionally, the effect of addition of a polyamine was also examined. The examined amount of added DMSO ranged from no addition to 10%. On the other hand, spermine tetrahydrochloride (Sigma), spermidine trihydrochloride (Sigma), acetylputrescine (Nacalai Tesque), putrescine dihydrochloride (Nacalai Tesque), trimethylene diamine (Nacalai Tesque), propylenediamine (Nacalai Tesque) and diaminomethane dihydrochloride (Nacalai Tesque) were used as a polyamine. The amounts of propylenediamine and trimethylene diamine added were within the range between no addition and 2%. Other polyaminies were used within the range between no addition and 5 mM. Reaction conditions and confirmation of amplification were as described above in (1).

As a result, a DNA fragment of interest was efficiently amplified using the additive at a concentration within the indicated range: no addition to 5% of DMSO; no addition to 200 μM of spermine tetrahydrochloride or spermidine; 40 μM to 40 mM of acetylputrescine or putrescine dihydrochloride; 0.002% to 0.02% of trimethylene diamine; 0.0001% to 0.01% of propylenediamine; and 0.1 μM to 10 μM of diaminomethane dihydrochloride.

(5) Examination of type of magnesium salt

The effect of the type of a magnesium salt on the amplification method of the present invention was examined. The pUC19 DNA was used as a template. pUC19 upper NN 249 primer and pUC19 lower NN primer having sequences as shown in SEQ ID NOS:11 and 6 of the Sequence Listing, respectively, were used as primers. An amplified fragment of about 225 bp is obtained using a pair of these primers. Magnesium chloride, magnesium acetate and magnesium sulfate were used as magnesium salts. The composition of the reaction mixture was as follows.

35 mM potassium phosphate buffer (pH 7.3), 8 mM (final concentration) magnesium chloride, magnesium acetate or magnesium sulfate, 1.0 mM (final concentration) each of dNTPs, 50 ng of the pUC19 DNA, 60 pmol each of the primers, 60 U of RNase H, 5.5 U of Bca BEST DNA polymerase and sterile distilled water to a reaction volume of 50 μl. Reaction conditions and confirmation of amplification were as described above in (3).

As a result, an amplified fragment of interest was observed using each of the magnesium salts.

(6) Examination of concentrations of magnesium and dNTPs

The effects of the concentrations of magnesium and dNTPs on the amplification method of the present invention were examined. The composition of the reaction mixture was as described above in (5), except that 25 ng of the pUC19 DNA, and magnesium and dNTPs at various concentrations were used. Reaction conditions and confirmation of amplification were as described above in (1).

In a reaction system in which the final concentration of each of dNTPs was fixed at 1 mM, an amplified fragment of interest was obtained when a final magnesium concentration ranging from 6 mM to 10 mM was used. In a reaction system in which the final magnesium concentration of was fixed at 8 mM, an amplified fragment of interest was obtained when a final concentration of each of dNTPs ranging from 0.6 mM to 1.2 mM was used. Furthermore, in a reaction system in which the final concentration of each of dNTPs was fixed at 0.5 mM, an amplified fragment of interest was obtained when a final magnesium concentration ranging from 2 mM to 6 mM was used. In a reaction system in which the final magnesium concentration was fixed at 4 mM, an amplified fragment of interest was obtained when a final concentration of each of dNTPs ranging from 0.2 mM to 0.8 mM was used.

(7) Examination of change in concentration of potassium phosphate buffer or Tricine buffer and reactivity

The effect of the concentration of the potassium phosphate buffer or the Tricine buffer on the amplification method of the present invention was examined. The composition of the reaction mixture was as described above in (1) for a case where pUC19-249 was used as a template, except that a potassium phosphate buffer at a final concentration of 20-50 mM or a Tricine buffer at a final concentration of 22-46 mM was used. Reaction conditions and confirmation of amplification were as described above in (1).

As a result, an amplified fragment of interest was obtained when the potassium phosphate buffer at a final concentration ranging from 20 to 50 mM or the Tricine buffer at a final concentration ranging from 22 to 46 mM was used.

(8) Examination of concentration of Bca BEST DNA polymerase

The effect of the concentration of Bca BEST DNA polymerase on the amplification method of the present invention was examined. The composition of the reaction mixture was as described above in (1) for a case where pUC19-249 was used as a template, except that a potassium phosphate buffer system or a Tricine buffer system and Bca BEST DNA polymerase at a concentration within a range of 1-22 U/50 μl reaction volume was used. Reaction conditions and confirmation of amplification were as described above in (1).

As a result, an amplified fragment of interest was obtained when Bca BEST DNA polymerase was used at a concentration within a range of 1-22 U/50 μl.

Example 6

Comparison with the PCR method

The amplification method of the present invention was compared with the PCR method. Ones in which a DNA fragment of about 150 bp or about 250 bp is inserted into a multi-cloning site in the pUC19 plasmid DNA were used as templates. The templates were prepared as follows.

pUC19 upper 150 primer, pUC19 upper 249 primer and pUC19 lower NN primer, which have sequences as shown in SEQ ID NOS:10, 11 and 6 of the Sequence Listing, respectively, were used to conduct a PCR reaction using 100 pg of the pUC19 plasmid DNA as a template. An amplified fragment of about 150 bp was obtained by using a combination of the pUC19 upper 150 primer and the pUC19 lower NN primer. An amplified fragment of about 250 bp was obtained by using a combination of the pUC19 upper 249 primer and the pUC19 lower NN primer. Each of these amplified fragments was purified using Microcon-100, blunt-ended using DNA blunting kit (Takara Shuzo), and then subcloned into a HincII site in the pUC19 plasmid. Plasmids into which one of the amplified fragments is inserted were used to transform E. coli JM109. The resulting transformants were cultivated and plasmids with inserted DNA were purified from the cells using QIAGEN plasmid mini kit (Qiagen). The plasmids with inserted DNA were used as templates.

The sequences of the primers used in this Example are shown in SEQ ID NOS:18 and 19 of the Sequence Listing. Primers in which the first to third bases from the 3'-terminus are replaced by ribonucleotides were used for the amplification method of the present invention. The composition of the reaction mixture was as follows.

27 mM phosphate buffer (pH 7.3), 0.01% bovine serum albumin (BSA), 5% DMSO, 1 mM each of dNTPs, 8 mM magnesium acetate, 60 pmol each of the primers, 1 ng of the DNA as the template and sterile distilled water to a reaction volume of 48 μl.

The reaction mixture was heat-denatured at 98°C for 1 minute, and then cooled to 55°C. 5.5 U of Bca BEST DNA polymerase and 60 U of E. coli RNase H were added thereto, and the mixture was incubated at 55°C for 60 minutes. Thereafter, the mixture was heated at 90°C for 2 minutes to inactivate the enzymes. 3 μl of the reaction mixture was subjected to electrophoresis on 4% NuSieve 3:1 agarose (Takara Shuzo) gel.

On the other hand, amplification using the PCR method was conducted as a control. PCR Amplification kit (Takara Shuzo), 10 pmol each of primers having a sequence as shown in SEQ ID NO:18 or 19 of the Sequence Listing without a ribonucleotide, 1 ng of the DNA as the template and sterile distilled water to a reaction volume of 50 μl were used for the reaction. The reaction conditions were 25 cycles of 94°C for 30 seconds, 55°C for 30 seconds and 72°C for 40 seconds. After the completion of the reaction, 3 μl of the reaction mixture was subjected to electrophoresis on 4% NuSieve 3:1 agarose (Takara Shuzo) gel.

As a result, more amount of a fragment of interest was amplified from each of the plasmids having an insert of 150 bp or 249 bp as a template in the amplification method of the present invention as compared with the PCR method. 20 μl of the reaction mixture was purified using Microcon-100, and the amount of the amplification product was quantified using Beckman DU-640 spectrophotometer (Beckman) in order to express numerically the amount of the amplification product. The amount of the fragment amplified from the plasmid having an insert of 150 bp as the template in the amplification method of the present invention was confirmed to be about 60-fold higher than that in the PCR method. The amount of the fragment amplified from the plasmid having an insert of 250 bp as the template in the amplification method of the present invention was confirmed to be about 40-fold higher than that in the PCR methods. Based on these results, it was confirmed that the method of the present invention can be more preferably used for making a DNA chip, for which a large amount of a DNA fragment is required, as compared with the conventional PCR method.

Example 7

(1) Preparation of RNA probe

A method for detecting an amplified fragment obtained by the amplification method of the present invention was examined. A probe for detection composed of ribonucleotides in which two different fluorescent substances are attached to the ribonucleotides on both ends of the probe was prepared. The RNA probe for detection was synthesized using a DNA synthesizer (Applied Biosystems). The nucleotide sequence of the probe is shown in SEQ ID NO:20 of the Sequence Listing. 6-FAM (Glen Research) and TAMRA (Glen Research) were used as fluorescent substances to label the probe at the 5'-terminus and the 3'-terminus, respectively.

(2) Amplification reaction and detection

0.1 or 1 ng of the pUC19 DNA was used as a template. pUC19 upper 150 primer and pUC19 lower 542 primer having sequences as shown in SEQ ID NOS:10 and 8 of the Sequence Listing, respectively, in which the first and second bases from the 3'-terminus of the primer are replaced by ribonucleotides were used as primers.

The composition of the reaction mixture was as follows.

27 mM phosphate buffer (pH 7.3), 0.01% BSA, 5% DMSO, 1 mM each of dNTPs, 8 mM magnesium acetate, 60 pmol each of the primers, 0.1 or 1 ng of the DNA as the template, 0.1 μg of the RNA probe and sterile distilled water to a reaction volume of 48 μl. One without the DNA as the template was also prepared as a control.

The reaction mixture was heat-denatured at 98°C for 1 minute, and then cooled to 55°C. 22 U of Bca BEST DNA polymerase or sterile water, and 60 U of E. coli RNase H were added thereto, and the mixture was incubated at 55°C for 60 minutes. Thereafter, 5 μl of 10% sodium dodecyl sulfate (SDS; Nacalai Tesque) was added to the mixture to inactivate the enzymes. 50 μl of the reaction mixture was diluted with an equal volume of sterile water and transferred to a microplate. An image analyzer FM BIO II Multi-View (Takara Shuzo) was used for detection at an excitation wavelength of 505 nm.

As a result, no fluorescent signal was detected using either of the templates when Bca BEST DNA polymerase was not added. Also, no fluorescent signal was detected for the reaction mixture containing Bca BEST DNA polymerase when the DNA as the template was not added. On the other hand, a fluorescent signal was detected when either 0.1 or 1 ng of the DNA as the template was added. An amplified fragment of about 190 bp of interest was also observed by electrophoresis on 3% agarose gel containing 0.00003% ethiduim bromide only when 0.1 or 1 ng of the DNA as the template was added in the presence of Bca BEST DNA polymerase. That is, the same results were obtained by the detection method using an RNA probe and the conventional electrophoretic detection method. Thus, a method for detecting an amplified fragment obtained by the amplification method of the present invention using an RNA probe was established.

Example 8

Use of a primer composed of deoxyribonucleotides as one of the two primers in the method of the present invention was examined. MR1N3 (30) having a sequence as shown in SEQ ID NO:19 of the Sequence Listing and M4 primer (Takara Shuzo) having a sequence as shown in SEQ ID NO:58 of the Sequence Listing were used as primers. In the MR1N3 primer, the first to third bases from the 3'-terminus are replaced by ribonucleotides. The composition of the reaction mixture was as follows.

27 mM phosphate buffer (pH 7.3), 0.01% bovine serum albumin (BSA), 5% DMSO, 1 mM each of dNTPs, 8 mM magnesium acetate, 30 pmol each of the primers, 1 ng of the DNA as the template and sterile distilled water to a reaction volume of 24 μl.

The reaction mixture was heat-denatured at 98°C for 2 minutes, and then cooled to 55°C. 11 U of Bca BEST DNA polymerase and 30 U of E. coli RNase H were added thereto to make the reaction volume to 25 μl. The reaction mixture was incubated at 55°C for 60 minutes. Thereafter, the mixture was heated at 90°C for 2 minutes to inactivate the enzymes. 5 μl of the reaction mixture was subjected to electrophoresis on 4% NuSieve 3:1 agarose gel. As a result, an amplified fragment of interest was observed.

Example 9

The method of the present invention was used to detect hemorrhagic E. coli O-157.

Sequences of the primers used in this Example are shown in SEQ ID NOS:21-24 of the Sequence Listing. A combination of primers having a sequence of SEQ ID NO:21 or 22, and a combination of primers having a sequence of SEQ ID NO:23 or 24 were constructed for detecting a sequence encoding vero toxin 1 or vero toxin 2 of O-157 according to the description of Rinsho To Biseibutsu (Clinical Microbiology), 18(4):507-513 (1991). Primers in which the first to third bases from the 3'-terminus are replaced by ribonucleotides were used for the amplification method of the present invention. A heat-extract prepared by harvesting a culture of hemorrhagic E. coli O-157 (ATCC accession no. 43895), suspending it in sterile water at an appropriate cell density and treating it at 98°C for 10 minutes was used as a template. The composition of the reaction mixture was as follows.

27 mM phosphate buffer (pH 7.3), 0.01% bovine serum albumin (BSA), 5% DMSO, 1 mM each of dNTPs, 8 mM magnesium acetate, 60 pmol each of the primers, the DNA as the template (the heat-extract) corresponding to $10^4$-$10^6$ cells and sterile distilled water to a reaction volume of 48 μl.

The reaction mixture was heat-denatured at 98°C for 1 minute, and then cooled to 55°C. 5.5 U of Bca BEST DNA polymerase and 60 U of E. coli RNase H were added thereto. The reaction mixture was incubated at 55°C for 60 minutes. Thereafter, the mixture was heated at 90°C for 2 minutes to inactivate the enzymes. 3 μl of the reaction mixture was subjected to electrophoresis on 4% NuSieve 3:1 agarose (Takara Shuzo) gel.

As a result, O-157 vero toxin 1 and 2 could be detected using either one of the primer pairs and the DNA as the template corresponding to $10^4$ cells, confirming that the method of the present invention can be utilized as a method for detecting a virulent bacterium.

Example 10

Amplification of a long-chain DNA fragment by the method of the present invention was examined. A double-stranded DNA as a template was prepared as follows. First, a library was constructed from mRNA derived from a normal gastric tissue using Uni-ZAP XR vector (Stratagene) according to a conventional method. The library was screened to select clones having an insert of about 2.1 kbp or about 4.3 kbp. The clones were used to obtain pBluescript SK (-) phage vectors by in vitro excision. Amplified fragments of about 2.2 kbp and about 4.4 kbp were obtained using the plasmids as templates, MCR-F primer and MCR-R primer having sequences as shown in SEQ ID NOS:25 and 26 of the Sequence Listing, respectively, and PCR Amplification kit (Takara Shuzo). These PCR fragments were used as templates for the amplification method of the present invention. MF2N3 (24) primer and MR1N3 (24) primer having sequences as shown in SEQ ID NOS:27 and 28 of the Sequence Listing, respectively, in which the first to third bases from the 3'-terminus are replaced by ribonucleotides were used as primers. The composition of the reaction mixture was as follows.

28 mM phosphate buffer (pH 7.5), 0.01% bovine serum albumin (BSA), 1% DMSO, 0.5 mM each of dNTPs, 4 mM magnesium acetate, 30 pmol each of the primers, 0.2 mM putrescine and sterile distilled water to 24.25 μl. The reaction mixture was treated at 92°C for 2 minutes, and then cooled to 55°C. 30 U of RNase H and 5.5 U of Bca BEST DNA polymerase were added thereto to make the reaction volume to 25 μl. The reaction mixture was incubated for 1 hour. After the completion of the reaction, the mixture was cooled at 4°C, and 2.5 μl of a 0.5 M EDTA solution added thereto to terminate the reaction. 5 μl of the mixture was subjected to electrophoresis on 1% agarose gel.

As a result, an amplified fragment of about 2.2 kbp or about 4.4 kbp was obtained by the method of the present invention, confirming that the method of the present invention can be used to amplify a long-chain DNA fragment.

Example 11

A DNA microarray onto which a λ DNA fragment of about 400 bp amplified by the amplification method of the present invention and λ DNA fragments of 300 bp and 1000 bp amplified by PCR were spotted was produced. The nucleotide sequence of the λ DNA is available from GenBank accession nos. V00636, J02459, M17233 and X00906. The sequences of primers used in this Example are shown in SEQ ID NOS:25-26 and 29-35 of the Sequence Listing. A reaction mixture for the amplification method of the present invention was prepared as follows.

34 mM Tricine-hydrochloride buffer (pH 8.7), 10 mM potassium chloride, 10 mM ammonium sulfate, 0.01% bovine serum albumin (BSA), 1% dimethyl sulfoxide, 4 mM magnesium acetate, 0.5 mM each of dNTPs, 500 pmol each of the primers, 100 ng of the PCR amplification product as the template, 110 U of Bca BEST DNA polymerase and 300 U of cloned RNase H in a final reaction volume of 500 μl. The reaction mixture was mixed to homogeneity, incubated at 55°C for 60 minutes, and then heated at 90°C for 2 minutes to inactivate the enzymes. This solution was used in the subsequent steps. The spotted DNA fragments were as follows.

1. Sample: A PCR amplification product (300 bp) obtained by using a λ DNA as a template and a combination of primers having a sequence as shown in SEQ ID NO:29 or 30 of the Sequence Listing was subcloned into the pUC19 vector. The subcloned product was then PCR-amplified using primers having a sequence as shown in SEQ ID NO: 25 or 26 of the Sequence Listing. The thus obtained product as a template and chimeric oligonucleotide primers having a sequence as shown in SEQ ID NO:31 or 32 of the Sequence Listing, in which the first and second bases from the 3'-terminus of the primer are replaced by ribonucleotides, were used to amplify a product of about 400 bp by the amplification method of the present invention to obtain the Sample. Five DNA solutions, i.e., the reaction mixture at its original concentration or 2-, 4-, 8- or 16-fold dilutions of the reaction mixture with a carbonate buffer (a carbonate buffer at a concentration of 50 mM were used for dilution in each case) were used for spotting.
2. Sample: The DNA fragment amplified in 1 above was treated with Microcon-100 (Takara Shuzo). Then, five DNA solutions were prepared by adjusting the concentrations to 0.125 μg/μl, 0.25 μg/μl, 0.5 μg/μl, 1.0 μg/μl and 2.0 μg/μl with the 50 mM carbonate buffer.
3. Positive Control: A PCR amplification product (300 bp) obtained by using the λ DNA as a template and a combination of primers having a sequence as shown in SEQ ID NO:29 or 30 of the Sequence Listing was treated with Microcon-100. Then, five DNA solutions were prepared by adjusting the concentrations to 0.125 μg/μl, 0.25 μg/μl, 0.5 μg/μl, 1.0 μg/μl and 2.0 μg/μl with the 50 mM carbonate buffer.
4. Positive Control: A PCR amplification product (1000 bp) obtained by using the λ DNA as a template and a combination of primers having a sequences as shown in SEQ ID NO:33 or 34 of the Sequence Listing was treated with Microcon-100. Then, four DNA solutions were prepared by adjusting the concentrations to 0.125 μg/μl, 0.25 μg/μl, 0.5 μg/μl and 1.0 μg/μl with the 50 mM carbonate buffer.
5. Negative Control: A PCR amplification product (300 bp) obtained by using the λ DNA as a template and a combination of primers having a sequence as shown in SEQ ID NO:33 or 35 of the Sequence Listing was subcloned into the pUC19 vector. The subcloned product was then PCR-amplified using primers having a sequence as shown in SEQ ID NO:25 or 26 of the Sequence Listing. The thus obtained product as a template and primers having a sequence as shown in SEQ ID NO:31 or 32 of the Sequence Listing as primers were used to amplify a product of about 400 bp by the amplification method of the present invention to obtain the Negative Control. Five DNA solutions, i.e., the reaction mixture at its original concentration or 2-, 4-, 8- or 16-fold dilutions of the reaction mixture with a carbonate buffer (a carbonate buffer at a concentration of 50 mM were used for dilution in each case) were used for spotting.
6. Negative Control: The DNA fragment obtained in 5 above was treated with Microcon-100. Then, five DNA solutions were prepared by adjusting the concentrations to 0.125 μg/μl, 0.25 μg/μl, 0.5 μg/μl, 1.0 μg/μl and 2.0 μg/μl with the 50 mM carbonate buffer.

The respective DNA solutions thus prepared were spotted onto a slide glass to which amino groups had been introduced (Matsunami Glass) using an equipment for making DNA chips (Genetic Microsystems (GMS)), and were immobilized using UV irradiation. The slide was washed with 0.2% SDS followed by distilled water, dried, and then used as a DNA array.

A PCR amplification product (300 bp) obtained by using a combination of primers having a sequence as shown in SEQ ID NO:29 or 30 of the Sequence Listing was labeled with Cy5 using Label IT Cy5R Labeling Kit (Takara Shuzo) for use as a probe. Hybridization was carried out using a prehybridization solution and a hybridization solution as described in the instructions attached to IntelliGene (Takara Shuzo). First, the DNA array was subjected to prehybridization at room temperature for 2 hours. The hybridization solution containing the denatured Cy5-labeled probe was dripped onto the DNA array. A cover glass was mounted thereon. The sides of the cover glass were sealed with a film. The sealed DNA array was incubated at 65°C for 13 hours. After the cover glass was removed, the DNA array was washed in 2 x SSC at 65°C for 5 minutes, in a solution containing 0.2 x SSC and 0.1% SDS at 65°C for 5 minutes, and finally in 0.2 x SSC at room temperature for 5 minutes, and air-dried. The DNA array was then subjected to a microarray scanner (GMS) to analyze the fluorescent signals from the respective spots.

As a result, a fluorescent signal was observed at each of the positions onto which fragments amplified by the PCR method (the Positive Controls as described above in 3 and 4) and the method of the present invention (the Samples as described above in 1 and 2) were spotted. The intensities of the signals were as follows: the Sample 2 > the Positive Control 4 > the Sample 1 > the Positive Control 3. On the other hand, no signal was observed at all for the Negative Controls 5 and 6. From these results, it was confirmed that an unpurified or purified DNA fragment amplified by the method of the present invention can be preferably used as a DNA fragment to be immobilized to make a DNA chip.

Example 12

(1) The designing of a primer used in the method of the present invention in which a PCR-amplified fragment was used as a template was examined. First, primers having one of sequences as shown in SEQ ID NOS:36-41 of the Sequence Listing were synthesized according to a conventional method. The structures of the respective primers are as follows.

(i) R1-S1 primer: From the 5'-terminus, 7 bases of a spacer sequence, 17 bases of a M13RV sequence (or RV sequence; the nucleotide sequence of M13RV primer (Takara Shuzo)) and 20 bases of a sense primer sequence for λ DNA-specific PCR;

(ii) R1-A3 primer: From the 5'-terminus, 7 bases of a spacer sequence, 17 bases of the M13RV sequence and 20 bases of an antisense primer sequence for λ DNA-specific PCR;

(iii) R2-S1 primer: From the 5'-terminus, 25 bases of a spacer sequence, 17 bases of the M13RV sequence and 20 bases of a sense primer sequence for λ DNA-specific PCR;

(iv) R2-A3 primer: From the 5'-terminus, 25 bases of a spacer sequence, 17 bases of the M13RV sequence and 20 bases of an antisense primer sequence for λ DNA-specific PCR;

(v) R3-S1 primer: From the 5'-terminus, 58 bases of a spacer sequence, 17 bases of the M13RV sequence and 20 bases of a sense primer sequence for λ DNA-specific PCR; and

(vi) R3-A3 primer: From the 5'-terminus, 58 bases of a spacer sequence, 17 bases of the M13RV sequence and 20 bases of an antisense primer sequence for λ DNA-specific PCR.

M13RV 20mer has a sequence of a total of 20 bases consisting of 17 bases of the M13RV sequence and 3 bases at the 5'-terminus. Therefore, when M13RV 20 mer is used in the method of the present invention, the lengths of the spacer sequences in the above-mentioned primers become 4 bases, 22 bases and 55 bases, respectively. Primers without a spacer sequence were also made as controls for the above-mentioned primers.

For example, when the primer pair, R1-S1 primer/R1-A3 primer, is used, an amplified fragment of 348 bp is obtained. 7 bases on both ends of the amplified fragment correspond to the spacer portions. The RV sequences are located inside the spacer portions. The λ DNA sequences are located inside the RV sequences.

Similarly, when the primer pair, R2-S1 primer/R2-A3 primer, is used, an amplified fragment of 384 bp in which 25 bases on both ends of the amplified fragment correspond to the spacer portions is obtained. In addition, when the primer pair, R3-S1 primer/R3-A3 primer, is used, an amplified fragment of 450 bp in which 58 bases on both ends of the amplified fragment correspond to the spacer portions is obtained. On the other hand, a fragment amplified using control primers has no spacer portion. These PCR-amplified fragments were used as templates for the following examination.

One of two primers, M13RV-2N 17mer primer or M13RV-2N 20mer having a sequence as shown in SEQ ID NOS:42 or 43 of the Sequence Listing was used in this Example. In the primers, the first and second bases from the 3'-terminus are replaced by ribonucleotides. The reaction was carried out as follows. 5 μl of a mixture of 20 μM of the primer, about 20 ng of the template and 0.01% propylenediamine was denatured at 98°C for 2 minutes, and then cooled to 55°C. Thereafter, 34 mM Tricine buffer (pH 8.7), 10 mM potassium chloride, 10 mM ammonium sulfate, 0.01% BSA, 1% DMSO, 4 mM magnesium acetate, 0.5 mM each of dNTPs, 1 U of Bca BEST DNA polymerase and 15 U of RNase H were added thereto to make the final reaction volume to 25 μl. The reaction mixture was incubated at 55°C for 1 hour. After the completion of the reaction, the mixture was cooled to 4°C, and then 2.5 μl of a 0.5 M EDTA solution was added thereto to terminate the reaction. 3 μl of the reaction mixture was subjected to electrophoresis on 3% NuSieve 3:1 agarose (Takara Shuzo) gel. As a result, when M13RV-2N 17mer was used, increase in amplification efficiency was observed depending on the length of the spacer sequence in the following order: 25mer > 7mer > 58mer > no spacer sequence. When M13RV-2N 20mer was used, increase in amplification efficiency was observed depending on the length of the spacer sequence in the following order: 22mer > 4mer > 55mer > no spacer sequence. Furthermore, when the M13RV sequences in the primers described above in (i) to (vi) were replaced by M13M4 sequences, a similar tendency was observed for the relationship between the spacer sequence and the amplification efficiency. Thus, it was confirmed that, when a linear DNA fragment such as a PCR-amplified fragment is used as a template, the designing of primers used in the method of the present invention to generate a spacer sequence (portion) leads to an increased amplification efficiency.
(2) Amplification of a template having a high GC content in the method for amplifying a nucleotide sequence using an elevated reaction temperature was examined. First, primers having a sequence as shown in SEQ ID NO:44 or 45 of the Sequence Listing for PCR amplification of a 307-bp region (GC content: 62.5%) of CDC2-related protein kinase PISS LRE gene (GenBank accession no. AA789328) were produced. In addition, primers having a sequence as shown in SEQ ID NO:46 or 47 of the Sequence Listing for PCR amplification of a 284-bp region (GC content: 61.3%) of Type II cytoskeltal 1 keratin gene (GenBank accession no. AA706022) were produced. PCR amplification was carried out using these primers and commercially available DNA fragments (Research Genetics) as templates. The respective PCR-amplified fragments obtained by using the above-mentioned primer pairs have spacer sequences and the M13RV sequences on both ends. The fragments were used as templates for the present invention.
M13RV-2N 17mer primer having a sequence as shown in SEQ ID NO:42 of the Sequence Listing or M13RV-2N 20mer primer having a sequence as shown in SEQ ID NO:43 of the Sequence Listing was used in this Example. In the primers, the first and second bases from the 3'-terminus are replaced by ribonucleotides. The reaction was carried out as follows. 10 μl of a mixture of 100 pmol of the primer, 20 ng of the template and 0.01% propylenediamine was denatured at 98°C for 2 minutes, and then cooled to 55°C or 60°C. Thereafter, 34 mM Tricine buffer (pH 8.7), 10 mM KCl, 10 mM ammonium sulfate, 0.01% BSA, 1% DMSO, 4 mM magnesium acetate, 0.5 mM each of dNTPs, 11 U of Bca BEST DNA polymerase and 30 U of RNase H were added thereto to make the final reaction volume to 50 μl. The reaction mixture was incubated at 55°C or 60°C for 1 hour. After the completion of the reaction, the mixture was cooled to 4°C. 3 μl of the reaction mixture was subjected to electrophoresis on 3% agarose gel. The results are shown in Table 1 below.

Table 1

| | | Gene and results of amplification | |
|---|---|---|---|
| Reaction temperature | Primers used | CDC2-related | Type II cytoskeltal |
| 55°C | M13RV-2N 17mer | ++ | ++ |
| | M13RV-2N 20mer | ++ | ++ |
| 60°C | M13RV-2N 17mer | + | + |
| | M13RV-2N 20mer | ++++ | ++++ |

+ to ++++: The degree of amplification was scored in four grades.
-: No amplification was observed.

As shown in Table 1, the region of interest was efficiently amplified even if a template having a high GC content was used. This amplification was accomplished by elevating the reaction temperature (from 55°C to 60°C) and by using a primer having a higher Tm value as compared with an optimal primer for a reaction at 55°C when the reaction was carried at 60°C.
(3) The relationship between the length of an amplified fragment and the amount of the amplification product in the method for amplifying a nucleotide sequence under high reaction temperature conditions was examined. First, a pair of primers having a sequence as shown in SEQ ID NO:48 or 49 of the Sequence Listing for amplifying a 800-bp region of the lambda DNA (Takara Shuzo) and a pair of primers having a sequence as shown in SEQ ID NO:50 or 51 of the Sequence Listing for amplifying a 400-bp region of the lambda DNA were synthesized according to a conventional method. PCR was conducted using one of these primer pairs and the λ DNA as a template to obtain an amplified fragment. An amplified fragment of about 1.1 kbp was also prepared using the pUC19-911 plasmid as described in Example 5 (1) as a template and MF2 (24) primer and MR1 (24) primer, which have sequences as shown in SEQ ID NOS:16 and 17 of the Sequence Listing, respectively. The PCR-amplified fragments obtained by using the above-mentioned primer pairs have spacer sequences and the M13RV or M4 sequences on both ends. These fragments were used as templates for the present invention.
M13RV-2N 17mer primer having a sequence as shown in SEQ ID NO:42 of the Sequence Listing or M13RV-2N 20mer primer having a sequence as shown in SEQ ID NO:43 of the Sequence Listing was used as a primer in this Example. In the primers, the first and second bases from the 3'-terminus are replaced by ribonucleotides. A combination of M13M4-3N 20mer primer having a sequence as shown in SEQ ID NO:55 of the Sequence Listing and M13RV-3N 20mer primer having a sequence as shown in SEQ ID NO:43 of the Sequence Listing, and a combination of M13M4-3N 24mer primer and M13RV-3N 24mer primer having sequences as shown in SEQ ID NO:56 and 57 of the Sequence Listing, respectively, were used for amplifying a region of about 1 kbp. In the primers, the first to third bases from the 3'-terminus are replaced by ribonucleotides. The reaction was carried out as follows. 10 μl of a mixture of 10 pmol of the primer, about 20 ng of the template and 0.01% propylenediamine was denatured at 98°C for 2 minutes, and then cooled to 55°C or 60°C. Thereafter, 34 mM Tricine buffer (pH 8.7), 10 mM potassium chloride, 10 mM ammonium sulfate, 0.01% BSA, 1% DMSO, 4 mM magnesium acetate, 0.5 mM each of dNTPs, 11 U of Bca BEST DNA polymerase and 30 U of RNase H were added thereto to make the final reaction volume to 50 μl. The reaction mixture was incubated at 55°C or 60°C for 1 hour. After the completion of the reaction, the mixture was cooled to 4°C, and then 5 μl of a 0.5 M EDTA solution was added thereto to terminate the reaction. 3 μl of the reaction mixture was subjected to electrophoresis on 3% NuSieve 3:1 agarose (Takara Shuzo) gel. The results are shown in Tables 2 and 3 below.

Table 2

| | | Length of amplified fragment and results | |
|---|---|---|---|
| Reaction temperature | Primers used | 400 bp | 800 bp |
| 55°C | M13RV-2N 17mer | ++ | ++ |
| | M13RV-2N 20mer | ++ | ++ |
| 60°C | M13RV-2N 17mer | + | + |
| | M13RV-2N 20mer | ++++ | ++++ |

+ to ++++: The degree of amplification was scored in four grades.
-: No amplification was observed.

As shown in Table 2, fragments for regions of 400 bp and 800 bp were efficiently amplified by making the length of the primer for amplification from 17mer to 20mer and by elevating the reaction temperature from 55°C to 60°C.

Table 3

| Reaction temperature | Primers used | Length of amplified fragment and results 1034bp |
|---|---|---|
| 55°C | M13RV-3N 20mer & M13M4-3N 20mer | ++ |
| | M13RV-3N 24mer & M13M4-3N 24mer | ++ |
| 65°C | M13RV-3N 20mer & M13M4-3N 20mer | + |
| | M13RV-3N 24mer & M13M4-3N 24mer | ++++ |

+ to ++++: The degree of amplification was scored in four grades.
-: No amplification was observed.

Furthermore, as shown in Table3, a fragment for a region of about 1 kbp was efficiently amplified by making the length of the primer for amplification from 20mer to 24mer and by elevating the reaction temperature from 55°C to 65°C. Additionally, similar results were obtained for amplification of a long-chain DNA fragment as described in Example 10 by using longer primers and an elevated reaction temperature. Increase in amplification efficiency was observed when a region of about 2 kbp or longer was amplified.

Example 13

(1) Use of a heat-resistant DNA polymerase other than Bca BEST DNA polymerase in the method of the present invention was examined. Bst DNA polymerase (New England Biolabs) was used as a heat-resistant DNA polymerase. A pair of primers, 5'-ID primer and 3'-ID primer having sequences as shown in SEQ ID NOS:52 and 53 of the Sequence Listing, respectively, were synthesized according to a conventional method. PCR was carried out using the primer pair and a commercially available DNA fragment for cyclin A gene (Research Genetics) as a template, resulting in an amplified fragment of about 300 bp. The PCR-amplified fragment obtained by using the primer pair has the M13RV sequences on both ends. The fragment was used as a template for the present invention.
M13RV-2N 17mer primer having a sequence as shown in SEQ ID NO:42 of the Sequence Listing was used as a primer in this Example. In the primer, the first and second bases from the 3'-terminus are replaced by ribonucleotides. The reaction was carried out as follows. 10 μl of a mixture of 20 μM of the primer, about 20 ng of the template and 0.01% propylenediamine was denatured at 98°C for 2 minutes, and then cooled to 55°C. Thereafter, 34 mM Tricine buffer (pH 8.7), 10 mM potassium chloride, 10 mM ammonium sulfate, 0.01% BSA, 1% DMSO, 4 mM magnesium acetate, 0.5 mM each of dNTPs, 4, 8, 12 or 16 U of Bst DNA polymerase and 30 U of RNase H were added thereto to make the final reaction volume to 50 μl. As a control, a reaction mixture having a composition identical with the above-mentioned one was prepared, except that 11 U of Bca BEST DNA polymerase was used. The reaction mixture was incubated at 55°C for 1 hour. After the completion of the reaction, the mixture was cooled to 4°C, and then 5 μl of a 0.5 M EDTA solution was added thereto to terminate the reaction. 3 μl of the reaction mixture was subjected to electrophoresis on 3% NuSieve 3:1 agarose (Takara Shuzo) gel. As a result, an amplified fragment of interest was obtained using each of the various units of Bst DNA polymerase. Thus, it was confirmed that heat-resistant DNA polymerases can be preferably used in the method of the present invention.
(2) Use of a mesophilic DNA polymerase in the method of the present invention was examined. 5'→3'exo activity (-) Klenow fragment (Takara Shuzo) was used as a mesophilic DNA polymerase. The DNA prepared in (1) above was used a template DNA for the method of the present invention.

M13RV-2N 16mer primer having a sequence as shown in SEQ ID NO:54 of the Sequence Listing was used as a primer in this Example. In the primer, the first and second bases from the 3'-terminus are replaced by ribonucleotides. The reaction was carried out as follows. 10 μl of a mixture of 20 μM of the primer, about 20 ng of the template and 0.01% propylenediamine was denatured at 98°C for 2 minutes, and then cooled to 40°C. Thereafter, 34 mM Tricine buffer (pH 8.7), 10 mM potassium chloride, 10 mM ammonium sulfate, 0.01% BSA, 1% DMSO, 4 mM magnesium acetate, 0.5 mM each of dNTPs, 0, 2, 4, 6 or 8 U of Klenow fragment and 30 U of RNase H were added thereto to make the final reaction volume to 50 μl. The reaction mixture was incubated at 40°C for 1 hour. After the completion of the reaction, the mixture was cooled to 4°C, and then 5 μl of a 0.5 M EDTA solution was added thereto to terminate the reaction. 3 μl of the reaction mixture was subjected to electrophoresis on 3% NuSieve 3:1 agarose (Takara Shuzo) gel. As a result, an amplified fragment of interest was obtained in cases where the various units of Klenow fragment were used excluding the case where no Klenow fragment was added. Thus, it was confirmed that mesophilic DNA polymerases can be preferably used in the method of the present invention.

Example 14

Chimeric oligonucleotide primers to be used in the method of the present invention were examined. A DNA as a template and primers were synthesized as described in Example 1 (1). The structures of the primers used in this Examples are described below in detail:

Primer Pair 1: A combination of primers having a nucleotide sequence as shown in SEQ ID NO:2 or 3 of the Sequence Listing and wholly composed of deoxyribonucleotides;
Primer Pair 2: A combination of primers having a nucleotide sequence as shown in SEQ ID NO:59 or 60 of the Sequence Listing in which the sixth and seventh deoxyribonucleotides from the 3'-terminus are replaced by ribonucleotides;
Primer Pair 3: A combination of primers having a nucleotide sequence as shown in SEQ ID NO:61 or 62 of the Sequence Listing in which the fifth and sixth deoxyribonucleotides from the 3'-terminus are replaced by ribonucleotides;
Primer Pair 4: A combination of primers having a nucleotide sequence as shown in SEQ ID NO:63 or 64 of the Sequence Listing in which the fourth and fifth deoxyribonucleotides from the 3'-terminus are replaced by ribonucleotides;
Primer Pair 5: A combination of primers having a nucleotide sequence as shown in SEQ ID NO:65 or 66 of the Sequence Listing in which the third and fourth deoxyribonucleotides from the 3'-terminus are replaced by ribonucleotides;
Primer Pair 6: A combination of primers having a nucleotide sequence as shown in SEQ ID NO:67 or 68 of the Sequence Listing in which the second and third deoxyribonucleotides from the 3'-terminus are replaced by ribonucleotides;
Primer Pair 7: A combination of primers having a nucleotide sequence as shown in SEQ ID NO:2 or 3 of the Sequence Listing in which the first and second deoxyribonucleotides from the 3'-terminus are replaced by ribonucleotides; and
Primer Pair 8: A combination of primers having a nucleotide sequence as shown in SEQ ID NO:67 or 68 of the Sequence Listing in which the second and third deoxyribonucleotides from the 3'-terminus are replaced by ribonucleotides and the phosphate bond on the 5'-terminal side of the third ribonucleotide from the 3'-terminus is replaced by a phosphorothioate bond.

Amplification conditions and detection method were as described in Example 1 (2) and (3). As a result, an amplified fragment having a length of interest was observed for each of the Primer Pairs 2-8. For the Primer Pairs 2-7, the amount of the amplification product increased as the number of deoxyribonucleotides at the 3'-terminus decreased. Particularly, the most abundant amplification product was observed for the Primer Pair 7 having no deoxyribonucleotide at the 3'-terminus. On the other hand, no amplified fragment was observed for the Primer Pair 1. Furthermore, the fact that amplified fragments of interest were observed for both of the Primer Pairs 6 and 8 confirms that both of a modified ribonucleotide and a unmodified ribonucleotide can be preferably used as a ribonucleotide contained in a primer in the method of the present invention.

Industrial Applicability

The present invention provides a convenient and efficient method for amplifying a nucleotide sequence characterized in that a DNA synthesis reaction is carried out in the presence of a chimeric oligonucleotide primer. Th present invention provides a method for supplying an amplified DNA fragment in large quantities. An efficient method for amplifying a nucleotide sequence is also provided by combining the method for amplifying a nucleotide sequence of the present invention with another nucleic acid amplification method. Furthermore, the present invention provides a method for detecting a nucleotide sequence for detecting or quantifying a microorganism such as a virus, a bacterium, a fungus and a yeast, as well as a method for real-time detection of the amplified DNA fragment obtained by the method of the present invention. In addition, present invention provides a large-scale gene sequencing method.

Sequence Listing Free Text

SEQ ID NO:1: Synthetic DNA corresponding to a portion of human transferrin receptor-encoding sequence used as a template.
SEQ ID NO:2: Designed oligonucleotide primer to amplify a portion of human transferrin receptor-encoding sequence.
SEQ ID NO:3: Designed oligonucleotide primer to amplify a portion of human transferrin receptor-encoding sequence.
SEQ ID NO:4: Designed oligonucleotide used as a probe for detecting an amplified portion of human transferrin receptor-encoding sequence.
SEQ ID NO:5: Designed oligonucleotide primer designated as pUC19 upper (2) NN to amplify a portion of plasmid pUC19.
SEQ ID NO:6: Designed oligonucleotide primer designated as pUC19 lower NN to amplify a portion of plasmid pUC19.
SEQ ID NO:7: Designed oligonucleotide primer to amplify a portion of plasmid pUC19.
SEQ ID NO:8: Designed oligonucleotide primer designated as pUC19 lower 542 to amplify a portion of plasmid pUC19.
SEQ ID NO:9: Designed oligonucleotide primer to amplify a portion of plasmid pUC19.
SEQ ID NO:10: Designed oligonucleotide primer designated as pUC19 upper 150 to amplify a portion of plasmid pUC19.
SEQ ID NO:11: Designed oligonucleotide primer designated as pUC19 upper 249 to amplify a portion of plasmid pUC19.
SEQ ID NO:12: Designed oligonucleotide primer to amplify a portion of human transferrin receptor-encoding sequence.
SEQ ID NO:13: Designed oligonucleotide primer to amplify a portion of human transferrin receptor-encoding sequence.
SEQ ID NO:14: Designed oligonucleotide primer to amplify a portion of human transferrin receptor-encoding sequence.
SEQ ID NO:15: Designed oligonucleotide primer to amplify a portion of human transferrin receptor-encoding sequence.
SEQ ID NO:16: Designed oligonucleotide primer designated as MF2N3 (24) to amplify a portion of plasmid pUC19-249 or plasmid pUC19-911.
SEQ ID NO:17: Designed oligonucleotide primer designated as MR1N3 (24) to amplify a portion of plasmid pUC19-249 or plasmid pUC19-911.
SEQ ID NO:18: Designed oligonucleotide primer to amplify a portion of plasmid pUC19.
SEQ ID NO:19: Designed oligonucleotide primer designated as MR1N3 to amplify a portion of plasmid pUC19.

SEQ ID NO:20: Synthetic RNA used as a probe for detecting an amplified portion of plasmid pUC19.
SEQ ID NO:21: Designed oligonucleotide primer to amplify a portion of vero toxin 1-encoding sequence from hemorrhagic Escherichia coli O-157.
SEQ ID NO:22: Designed oligonucleotide primer to amplify a portion of vero toxin 1-encoding sequence from hemorrhagic Escherichia coli O-157.
SEQ ID NO:23: Designed oligonucleotide primer to amplify a portion of vero toxin 2-encoding sequence from hemorrhagic Escherichia coli O-157.
SEQ ID NO:24: Designed oligonucleotide primer to amplify a portion of vero toxin 2-encoding sequence from hemorrhagic Escherichia coli O-157.
SEQ ID NO:25: Designed oligonucleotide primer designated as MCR-F to amplify a long DNA fragment.
SEQ ID NO:26: Designed oligonucleotide primer designated as MCR-R to amplify a long DNA fragment.
SEQ ID NO:27: Designed oligonucleotide primer designated as MF2N3 (24) to amplify a long DNA fragment.
SEQ ID NO:28: Designed oligonucleotide primer designated as MR1N3 (24) to amplify a long DNA fragment.
SEQ ID NO:29: Designed oligonucleotide primer to amplify a portion of bacteriophage lambda DNA.
SEQ ID NO:30: Designed oligonucleotide primer to amplify a portion of bacteriophage lambda DNA.
SEQ ID NO:31: Designed oligonucleotide primer to amplify a portion of bacteriophage lambda DNA.
SEQ ID NO:32: Designed oligonucleotide primer to amplify a portion of bacteriophage lambda DNA.
SEQ ID NO:33: Designed oligonucleotide primer to amplify a portion of bacteriophage lambda DNA.
SEQ ID NO:34: Designed oligonucleotide primer to amplify a portion of bacteriophage lambda DNA.
SEQ ID NO:35: Designed oligonucleotide primer to amplify a portion of bacteriophage lambda DNA.
SEQ ID NO:36: Designed oligonucleotide primer designated as R1-S1 to amplify a portion of bacteriophage lambda DNA.
SEQ ID NO:37: Designed oligonucleotide primer designated as R1-A3 to amplify a portion of bacteriophage lambda DNA.
SEQ ID NO:38: Designed oligonucleotide primer designated as R2-S1 to amplify a portion of bacteriophage lambda DNA.
SEQ ID NO:39: Designed oligonucleotide primer designated as R2-A3 to amplify a portion of bacteriophage lambda DNA.
SEQ ID NO:40: Designed oligonucleotide primer designated as R3-S1 to amplify a portion of bacteriophage lambda DNA.
SEQ ID NO:41: Designed oligonucleotide primer designated as R3-A3 to amplify a portion of bacteriophage lambda DNA.
SEQ ID NO:42: Designed oligonucleotide primer designated as M13RV-2N 17mer.
SEQ ID NO:43: Designed oligonucleotide primer designated as M13RV-2N 20mer.
SEQ ID NO:44: Designed oligonucleotide primer to amplify a portion of CDC2-related protein kinase PISSLRE gene.
SEQ ID NO:45: Designed oligonucleotide primer to amplify a portion of CDC2-related protein kinase PISSLRE gene.
SEQ ID NO:46: Designed oligonucleotide primer to amplify a portion of Type II cytoskeltal 11 keratin gene.
SEQ ID NO:47: Designed oligonucleotide primer to amplify a portion of Type II cytoskeltal 11 keratin gene.
SEQ ID NO:48: Designed oligonucleotide primer to amplify a portion of bacteriophage lambda DNA.
SEQ ID NO:49: Designed oligonucleotide primer to amplify a portion of bacteriophage lambda DNA.
SEQ ID NO:50: Designed oligonucleotide primer to amplify a portion of bacteriophage lambda DNA.
SEQ ID NO:51: Designed oligonucleotide primer to amplify a portion of bacteriophage lambda DNA.
SEQ ID NO:52: Designed oligonucleotide primer designated as 5'ID to amplify a portion of cyclin A DNA.
SEQ ID NO:53: Designed oligonucleotide primer designated as 3'ID to amplify a portion of cyclin A DNA.
SEQ ID NO:54: Designed oligonucleotide primer designated as M13RV-2N 16mer.
SEQ ID NO:55: Designed oligonucleotide primer designated as M13M4-3N 16mer.
SEQ ID NO:56: Designed oligonucleotide primer designated as M13M4-3N 24mer.
SEQ ID NO:57: Designed oligonucleotide primer designated as M13RV-3N 24mer.
SEQ ID NO:58: Designed oligonucleotide primer designated as M13M4 17mer.
SEQ ID NO:59: Designed oligonucleotide primer to amplify a portion of human transferrin receptor-encoding sequence.
SEQ ID NO:60: Designed oligonucleotide primer to amplify a portion of human transferrin receptor-encoding sequence.
SEQ ID NO:61: Designed oligonucleotide primer to amplify a portion of human transferrin receptor-encoding sequence.
SEQ ID NO:62: Designed oligonucleotide primer to amplify a portion of human transferrin receptor-encoding sequence.
SEQ ID NO:63: Designed oligonucleotide primer to amplify a portion of human transferrin receptor-encoding sequence.
SEQ ID NO:64: Designed oligonucleotide primer to amplify a portion of human transferrin receptor-encoding sequence.
SEQ ID NO:65: Designed oligonucleotide primer to amplify a portion of human transferrin receptor-encoding sequence.
SEQ ID NO:66: Designed oligonucleotide primer to amplify a portion of human transferrin receptor-encoding sequence.
SEQ ID NO:67: Designed oligonucleotide primer to amplify a portion of human transferrin receptor-encoding sequence.
SEQ ID NO:68: Designed oligonucleotide primer to amplify a portion of human transferrin receptor-encoding sequence.

## Claims

1. A method for amplifying a nucleotide sequence or for producing a nucleic acid in large quantities, **characterized in that** the method comprises:

   (a) preparing a reaction mixture by mixing a nucleic acid as a template, a deoxyribonucleotide triphosphate, a DNA polymerase having a strand displacement activity, at least one primer and an endonuclease that cleaves an extended strand generated from the primer, wherein the primer is a chimeric oligonucleotide primer that is substantially complementary to the base sequence of the nucleic acid as the template and contains deoxyribonucleotides and one or more ribonucleotide(s), the ribonucleotide(s) being positioned at the 3'-terminus or on the 3'-terminal side of the primer wherein the endonuclease cleaves at a site that contains the ribonucleotide, and
   (b) incubating the reaction mixture for a sufficient time to generate a reaction product under conditions where specific annealing of the primer to the nucleic acid as a template, an extended strand synthesis reaction and a strand displacement reaction by the DNA polymerase, and a reaction of cleaving the extended strand by the endonuclease take place.

2. The method according to claim 1, wherein the reaction mixture is incubated isothermally.

3. The method according to claim 1 or 2, wherein the reaction mixture further contains a chimeric oligonucleotide primer having a sequence substantially homologous to the base sequence of the template.

4. The method according to claim 1, **characterized in that** the method comprises:

   (a) treating a nucleic acid as a template with at least one primer that is substantially complementary to the base sequence of the nucleic acid and a DNA polymerase to synthesize a primer-extended strand that is complementary to the template, wherein the primer is a chimeric oligonucleotide primer containing deoxyribonucleotides and one or more ribonucleotide(s), the ribonucleotide(s) being positioned at the 3'-terminus or on the 3'-terminal side of the primer; wherein the endonuclease cleaves at a site that contains the ribonucleotide;
(b) cleaving the primer-extended strand of a double-stranded nucleic acid obtained in step (a) with the endonuclease at a site that contains the ribonucleotide; and
(c) extending a nucleotide sequence that is complementary to the template using a DNA polymerase having a strand displacement activity from the 3'-terminus of the primer portion of the double-stranded nucleic acid cleaved in step (b) to effect a strand displacement.

**5.** The method according to claim 4, wherein step (b) and step (c) are sequentially repeated.

**6.** A method of claim 4, wherein a double-stranded nucleic acid containing a regenerated primer-extended strand is reused in step (b); and the method further comprises the steps of:

(d) treating a released displaced strand obtained in step (c) as a template with at least one primer that is different from that used in step (a) and a DNA polymerase to synthesize a primer-extended strand that is complementary to the displaced strand, wherein the primer that is different from that used in step (a) is a chimeric oligonucleotide primer that is substantially complementary to the base sequence of the displaced strand and contains a deoxyribonucleotide and a ribonucleotide, the ribonucleotide being positioned at the 3'-terminus or on the 3'-terminal side of the primer, wherein the endonuclease cleaves at a site that contains the ribonucleotide;
(e) cleaving the primer-extended strand of a double-stranded nucleic acid obtained in step (d) with the endonuclease at a site that contains the ribonucleotide; and
(f) extending a nucleotide sequence that is complementary to the template using a DNA polymerase having a strand displacement activity from the 3'-terminus of the primer portion of the double-stranded nucleic acid cleaved in step (e) to effect a strand displacement, wherein a double-stranded nucleic acid containing a regenerated primer-extended strand is reused in step (e).

**7.** The method according to any one of claims 4 to 6, wherein the respective steps are conducted isothermally.

**8.** The method according to any one of claims 1 to 7, wherein the endonuclease is an endoribonuclease.

**9.** The method according to claim 8, wherein the endoribonuclease is RNase H.

**10.** The method according to any one of claims 1 to 9, wherein the chimeric oligonucleotide primer contains two or more successive ribonucleotide residues.

**11.** The method according to any one of claims 1 to 10, wherein the chimeric oligonucleotide primer contains one or more modified ribonucleotide.

**12.** The method according to claim 11, wherein the chimeric oligonucleotide primer contains an ($\alpha$-S) ribonucleotide in which the oxygen atom bound to the phosphorous atom at the $\alpha$-position of the ribonucleotide is replaced by a sulfur atom.

**13.** The method according to any one of claims 1 to 12, wherein the nucleic acid used as the template is a single-stranded DNA or a double-stranded DNA.

**14.** The method according to claim 13, which is conducted after converting a double-stranded DNA as the template into single-stranded DNAs.

**15.** The method according to claim 13 or 14, wherein the nucleic acid used as the template is cDNA obtained from an RNA by a reverse transcription reaction.

**16.** The method according to claim 15, which is conducted after synthesizing cDNA by a reverse transcription reaction using RNA as a template.

**17.** The method according to claim 16, wherein a primer selected from the group consisting of an oligo-dT primer, a random primer and a specific primer is used as a primer for the reverse transcription reaction.

**18.** The method according to claim 15 or 16, wherein a chimeric oligonucleotide primer is used as a primer for the reverse transcription reaction.

**19.** The method according to any one of claims 15 to 18, wherein a DNA polymerase having a reverse transcriptase activity is used as a reverse transcriptase.

**20.** The method according to any one of claims 15 to 19, wherein the reverse transcription reaction and the synthesis of the extended strand that is complementary to the template are conducted using one DNA polymerase having a reverse transcriptase activity and a strand displacement activity.

**21.** The method according to any one of claims 1 to 19, wherein the DNA polymerase is selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax.

**22.** The method according to claim 20, wherein the DNA polymerase is Bst DNA polymerase lacking 5'→3' exonuclease from Bacillus stearothermophilus or Bca DNA polymerase lacking 5'→3' exonuclease from Bacillus caldotenax.

**23.** The method according to any one of claims 16 to 22, wherein the RNA as the template in the reverse transcription reaction is an RNA amplified by a nucleic acid amplification reaction.

**24.** The method according to claim 23, which is conducted after synthesizing an amplified RNA fragment by a nucleic acid amplification reaction using an RNA as a template.

**25.** The method according to claim 23 or 24, wherein the nucleic acid amplification reaction is selected from the group consisting of the transcription-based amplification system (TAS) method, the self-sustained sequence replication (3SR) method, the nucleic acid sequence-based amplification (NASBA) method, the transcription-mediated amplification (TMA) method and the Qβ replicase method.

**26.** The method according to claim 13 or 14 wherein the nucleic acid as the template is a DNA obtained by a nucleic acid amplification reaction.

**27.** The method according to claim 26, which is conducted after synthesizing an amplified DNA fragment by a nucleic acid amplification reaction using a DNA as a template.

**28.** The method according to claim 27, wherein the nucleic acid amplification reaction is selected from the group consisting of the polymerase chain reaction (PCR) method, the ligase chain reaction (LCR) method and the strand displacement amplification (SDA) method.

**29.** The method according to any one of claims 23 to 28, wherein a random primer or a degenerate primer is used for the nucleic acid amplification reaction.

**30.** The method according to claim 29, wherein the random primer or the degenerate primer is a primer having a random sequence or a degenerate sequence at least at the 3'-terminus or on the 3'-terminal side.

**31.** A method for detecting a target nucleic acid in a sample, **characterized in that** the method comprises:

(a) amplifying a target nucleic acid by the method for amplifying a nucleotide sequence according to any one of claims 1 to 30; and
(b) detecting the target nucleic acid amplified in step (a).

**32.** The method according to claim 31, wherein the amplified target nucleic acid is detected using a ribonucleotide (RNA) probe labeled with two or more fluorescent substances positioned at a distance that results in a quenching state.

**33.** A kit for the method according to any one of claims 1 to 32, which contains:

(a) a DNA polymerase having a strand displacement activity;
(b) an endonuclease; and

(c) a chimeric oligonucleotide primer, which contains a deoxyribonucleotide and a ribonucleotide, the ribonucleotide being positioned at the 3'-terminus.

**34.** The kit according to claim 33, which contains a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax as the DNA polymerase.

**35.** The kit according to claim 33 which contains RNase H as the endonuclease.

**36.** A method for producing a material having an immobilized nucleic acid in which the nucleic acid is arrayed in a predefined region, **characterized in that** the method comprises:

(a) amplifying a nucleic acid to be immobilized by the method for amplifying a nucleotide sequence or for producing a nucleic acid in large quantities according to any one of claims 1 to 32; and
(b) arraying and immobilizing the nucleic acid amplified in step (a) in a predefined region on a substrate.

**37.** The method according to claim 36, wherein a single-stranded nucleic acid substantially free of a complementary strand thereto is amplified, and arrayed and immobilized in the predefined region on the substrate.

**38.** A method for determining a base sequence of a nucleic acid, **characterized in that** the method comprises amplifying a nucleotide sequence according to the method according to any one of claims 1 to 32.

**39.** A method for detecting a mutation of a target nucleic acid, **characterized in that** the method comprises amplifying a nucleotide sequence according to the method according to any one of claims 1 to 32.

## Patentansprüche

**1.** Verfahren zum Amplifizieren einer Nukleotidsequenz oder zum Erzeugen einer Nukleinsäure in großen Mengen, **dadurch** charakterisiert, dass das Verfahren umfasst:

(a) Herstellen eines Reaktionsgemisches durch Mischen einer Nukleinsäure als einer Matrize, eines Desoxyribonukleotidtriphosphats, einer DNA-Polymerase mit einer Strangverdrängungsaktivität, mindestens eines Primers und einer Endonuklease, die einen aus dem Primer erzeugten verlängerten Strang spaltet, wobei der Primer ein chimärer Oligonukleotidprimer ist, der im Wesentlichen komplementär zu der Basensequenz der Nukleinsäure als der Matrize ist und Desoxyribonukleotide und ein oder mehrere Ribonukleotid(e) enthält, wobei das/die Ribonukleotid(e) an dem 3'-Terminus oder an der 3'-terminalen Seite des Primers gelegen ist/sind, wobei die Endonuklease bei einer Stelle spaltet, die das Ribonukleotid enthält, und
(b) Inkubieren des Reaktionsgemisches für eine Zeitspanne, die ausreicht, um ein Reaktionsprodukt unter Bedingungen zu erzeugen, bei denen ein spezifisches Anlagern des Primers an die Nukleinsäure als eine Matrize, eine Synthesereaktion eines verlängerten Strangs und eine Strangverdrängungsreaktion durch die DNA-Polymerase und eine Reaktion eines Spaltens des verlängerten Strangs durch die Endonuklease stattfinden.

**2.** Verfahren gemäß Anspruch 1, wobei das Reaktionsgemisch isotherm inkubiert wird.

**3.** Verfahren gemäß Anspruch 1 oder 2, wobei das Reaktionsgemisch ferner einen chimären Oligonukleotidprimer mit einer Sequenz enthält, die im Wesentlichen homolog zu der Basensequenz der Matrize ist.

**4.** Verfahren gemäß Anspruch 1, **dadurch** charakterisiert, dass das Verfahren umfasst:

(a) Behandeln einer Nukleinsäure als einer Matrize mit mindestens einem Primer, der im Wesentlichen komplementär zu der Basensequenz der Nukleinsäure ist, und einer DNA-Polymerase, um einen Primer-verlängerten Strang zu synthetisieren, der komplementär zu der Matrize ist, wobei der Primer ein chimärer Oligonukleotidprimer mit Desoxyribonukleotiden und einem oder mehreren Ribonukleotid(en) ist, wobei das/die Ribonukleotid(e) an dem 3'-Terminus oder an der 3'-terminalen Seite des Primers gelegen ist/sind, wobei die Endonuklease bei einer Stelle spaltet, die das Ribonukleotid enthält,

(b) Spalten des Primer-verlängerten Strangs einer doppelsträngigen Nukleinsäure, die in Schritt (a) erhalten wurde, mit der Endonuklease bei einer Stelle, die das Ribonukleotid enthält, und

(c) Verlängern einer Nukleotidsequenz, die komplementär zu der Matrize ist, unter Verwendung einer DNA-Polymerase mit einer Strangverdrängungsaktivität von dem 3'-Terminus des Primeranteils der doppelsträngigen Nukleinsäure, die in Schritt (b) gespalten wurde, um eine Strangverdrängung zu bewirken.

**5.** Verfahren gemäß Anspruch 4, wobei der Schritt (b) und der Schritt (c) sequenziell wiederholt werden.

**6.** Verfahren nach Anspruch 4, wobei eine doppelsträngige Nukleinsäure mit einem regenerierten Primer-verlängerten Strang in Schritt (b) wieder verwendet wird und das Verfahren ferner die Schritte umfasst:

(d) Behandeln eines freigesetzten verdrängten Strangs, der in Schritt (c) erhalten wurde, als einer Matrize mit mindestens einem Primer, der zu demjenigen, der in Schritt (a) verwendet wurde, unterschiedlich ist, und einer DNA-Polymerase, um einen Primer-verlängerten Strang zu synthetisieren, der komplementär zu dem verdrängten Strang ist, wobei der Primer, der zu dem in Schritt (a) verwendeten unterschiedlich ist, ein chimärer Oligonukleotidprimer ist, der im Wesentlichen komplementär zu der Basensequenz des verdrängten Strangs ist und ein Desoxyribonukleotid und ein Ribonukleotid enthält, wobei das Ribonukleotid an dem 3'-Terminus oder an der 3'-terminalen Seite des Primers gelegen ist, wobei die Endonuklease bei einer Stelle spaltet, die das Ribonukleotid enthält,

(e) Spalten des Primer-verlängerten Strangs einer doppelsträngigen Nukleinsäure, die in Schritt (d) erhalten wurde, mit der Endonuklease bei einer Stelle, die das Ribonukleotid enthält, und

(f) Verlängern einer Nukleotidsequenz, die komplementär zu der Matrize ist, unter Verwendung einer DNA-Polymerase mit einer Strangverdrängungsaktivität von dem 3'-Terminus des Primeranteils der doppelsträngigen Nukleinsäure, die in Schritt (e) gespalten wurde, um eine Strangverdrängung zu bewirken, wobei eine doppelsträngige Nukleinsäure mit einem regenerierten Primer-verlängerten Strang in Schritt (e) wieder verwendet wird.

**7.** Verfahren gemäß einem der Ansprüche 4 bis 6, wobei die entsprechenden Schritte isotherm durchgeführt werden.

**8.** Verfahren gemäß einem der Ansprüche 1 bis 7, wobei die Endonuklease eine Endoribonuklease ist.

**9.** Verfahren gemäß Anspruch 8, wobei die Endoribonuklease RNase H ist.

**10.** Verfahren gemäß einem der Ansprüche 1 bis 9, wobei der chimäre Oligonukleotidprimer zwei oder mehrere aufeinander folgende Ribonukleotidreste aufweist.

**11.** Verfahren gemäß einem der Ansprüche 1 bis 10, wobei der chimäre Oligonukleotidprimer ein oder mehrere modifizierte Ribonukleotide enthält.

**12.** Verfahren gemäß Anspruch 11, wobei der chimäre Oligonukleotidprimer ein ($\alpha$-S)-Ribonukleotid enthält, worin das Sauerstoffatom, das an das Phosphoratom an der $\alpha$-Position des Ribonukleotids gebunden ist, durch ein Schwefelatom ersetzt ist.

**13.** Verfahren gemäß einem der Ansprüche 1 bis 12, wobei die Nukleinsäure, die als die Matrize verwendet wird, eine einzelsträngige DNA oder eine doppelsträngige DNA ist.

**14.** Verfahren gemäß Anspruch 13, das nach Umwandeln einer doppelsträngigen DNA als der Matrize in einzelsträngige DNAs durchgeführt wird.

**15.** Verfahren gemäß Anspruch 13 oder 14, wobei die Nukleinsäure, die als die Matrize verwendet wird, cDNA ist, die aus einer RNA durch eine reverse Transkriptionsreaktion erhalten wurde.

**16.** Verfahren gemäß Anspruch 15, das nach Synthetisieren der cDNA durch eine reverse Transkriptionsreaktion unter Verwendung von RNA als einer Matrize durchgeführt wird.

**17.** Verfahren gemäß Anspruch 16, wobei ein Primer, ausgewählt aus der Gruppe, bestehend aus einem Oligo-dT-Primer, einem zufälligen Primer und einem spezifischen Primer, als ein Primer für die reverse Transkriptionsreaktion verwendet wird.

**18.** Verfahren gemäß Anspruch 15 oder 16, wobei ein chimärer Oligonukleotidprimer als ein Primer für die reverse Transkriptionsreaktion verwendet wird.

**19.** Verfahren gemäß einem der Ansprüche 15 bis 18, wobei eine DNA-Polymerase mit einer reversen Transkriptaseaktivität als eine reverse Transkriptase verwendet wird.

**20.** Verfahren gemäß einem der Ansprüche 15 bis 19, wobei die reverse Transkriptionsreaktion und die Synthese des verlängerten Strangs, der komplementär zu der Matrize ist, unter Verwendung einer DNA-Polymerase erfolgen, die eine reverse Transkriptaseaktivität und eine Strangverdrängungsaktivität aufweist.

**21.** Verfahren gemäß einem der Ansprüche 1 bis 19, wobei die DNA-Polymerase ausgewählt ist aus der Gruppe, bestehend aus Klenow-Fragment einer DNA-Polymerase I aus Escherichia coli, Bst-DNA-Polymerase, der die 5'→3'-Exonuklease fehlt, aus Bacillus stearothermophilus und Bca-DNA-Polymerase, der die 5'→3'-Exonuklease fehlt, aus Bacillus caldotenax.

**22.** Verfahren gemäß Anspruch 20, wobei die DNA-Polymerase Bst-DNA-Polymerase, der die 5'→3'-Exonuklease fehlt, aus Bacillus stearothermophilus oder Bca-DNA-Polymerase, der die 5'→3'-Exonuklease fehlt, aus Bacillus caldotenax ist.

**23.** Verfahren gemäß einem der Ansprüche 16 bis 22, wobei die RNA als die Matrize in der reversen Transkriptionsreaktion eine RNA ist, die durch eine Nukleinsäureamplifizierungsreaktion amplifiziert wurde.

**24.** Verfahren gemäß Anspruch 23, das nach Synthetisieren eines amplifizierten RNA-Fragments durch eine Nukleinsäureamplifikationsreaktion unter Verwendung einer RNA als einer Matrize durchgeführt wird.

**25.** Verfahren gemäß Anspruch 23 oder 24, wobei die Nukleinsäureamplifikationsreaktion ausgewählt ist aus der Gruppe, bestehend aus dem Verfahren eines Transkriptions-basierenden Amplifikationssystems (TAS), dem Verfahren einer selbst erhaltenden Sequenzreplikation (3SR), dem Verfahren einer Nukleinsäuresequenz-basierenden Amplifikation (NASBA), dem Verfahren einer Transkriptions-vermittelten Amplifikation (TMA) und dem Qβ-Replikase-Verfahren.

**26.** Verfahren gemäß Anspruch 13 oder 14, wobei die Nukleinsäure als die Matrize eine DNA ist, die durch eine Nukleinsäureamplifikationsreaktion erhalten wurde.

**27.** Verfahren gemäß Anspruch 26, das nach Synthetisieren eines amplifizierten DNA-Fragments durch eine Nukleinsäureamplifikationsreaktion unter Verwendung einer DNA als einer Matrize durchgeführt wird.

**28.** Verfahren gemäß Anspruch 27, wobei die Nukleinsäureamplifikationsreaktion ausgewählt ist aus der Gruppe, bestehend aus dem Verfahren einer Polymerase-Kettenreaktion (PCR), dem Verfahren einer Ligase-Kettenreaktion (LCR) und dem Verfahren einer Strangverdrängungsamplifikation (SDA).

**29.** Verfahren gemäß einem der Ansprüche 23 bis 28, wobei ein zufälliger Primer oder ein degenerierter Primer für die Nukleinsäureamplifikationsreaktion verwendet wird.

**30.** Verfahren gemäß Anspruch 29, wobei der zufällige Primer oder der degenerierte Primer ein Primer ist, der eine zufällige Sequenz oder eine degenerierte Sequenz mindestens an dem 3'-Terminus oder an der 3'-terminalen Seite aufweist.

**31.** Verfahren zum Nachweisen einer Zielnukleinsäure in einer Probe, **dadurch** charakterisiert, dass das Verfahren umfasst:

(a) Amplifizieren einer Zielnukleinsäure durch das Verfahren zum Amplifizieren einer Nukleinsäuresequenz gemäß einem der Ansprüche 1 bis 30 und
(b) Nachweisen der in Schritt (a) amplifizierten Zielnukleinsäure.

**32.** Verfahren gemäß Anspruch 31, wobei die amplifizierte Zielnukleinsäure unter Verwendung einer Ribonukleotid (RNA)-Sonde nachgewiesen wird, die mit zwei oder mehreren fluoreszierenden Substanzen markiert ist, die bei einem Abstand positioniert sind, der zu einem Löschungszustand führt.

**33.** Kit für das Verfahren gemäß einem der Ansprüche 1 bis 32, der enthält:

(a) eine DNA-Polymerase mit einer Strangverdrängungsaktivität,
(b) eine Endonuklease und
(c) einen chimären Oligonukleotidprimer, der ein Desoxyribonukleotid und ein Ribonukleotid enthält, wobei das Ribonukleotid an dem 3'-Terminus positioniert ist.

**34.** Kit gemäß Anspruch 33, der eine DNA-Polymerase, ausgewählt aus der Gruppe, bestehend aus Klenow-Fragment der DNA-Polymerase I aus Escherichia coli, Bst-DNA-Polymerase, der die 5'→3'-Exonuklease fehlt, aus Bacillus stearothermophilus und Bca-DNA-Polymerase, der die 5'→3'-Exonuklease fehlt, aus Bacillus caldotenax, als die DNA-Polymerase, enthält.

**35.** Kit gemäß Anspruch 33, der RNase H als die Endonuklease enthält.

**36.** Verfahren zum Herstellen eines Materials mit einer immobilisierten Nukleinsäure, worin die Nukleinsäure in einem vordefinierten Bereich angeordnet ist, **dadurch** charakterisiert, dass das Verfahren umfasst:

(a) Amplifizieren einer zu immobilisierenden Nukleinsäure durch das Verfahren zum Amplifizieren einer Nukleotidsequenz oder zum Herstellen einer Nukleinsäure in großen Mengen gemäß einem der Ansprüche 1 bis 32 und
(b) Anordnen und Immobilisieren der in Schritt (a) amplifizierten Nukleinsäure in einem vorbestimmten Bereich auf einem Substrat.

**37.** Verfahren gemäß Anspruch 36, wobei eine einzelsträngige Nukleinsäure, die im Wesentlichen frei von einem dazu komplementären Strang ist, amplifiziert wird und in dem vorbestimmten Bereich auf dem Substrat angeordnet und immobilisiert wird.

**38.** Verfahren zum Bestimmen einer Basensequenz einer Nukleinsäure, **dadurch** charakterisiert, dass das Verfahren ein Amplifizieren einer Nukleotidsequenz gemäß dem Verfahren gemäß einem der Ansprüche 1 bis 32 umfasst.

**39.** Verfahren zum Nachweisen einer Mutation einer Zielnukleinsäure, **dadurch** charakterisiert, dass das Verfahren ein Amplifizieren einer Nukleotidsequenz gemäß dem Verfahren gemäß einem der Ansprüche 1 bis 32 umfasst.

## Revendications

**1.** Méthode pour amplifier une séquence nucléotidique ou pour produire un acide nucléique en grandes quantités, **caractérisée en ce que** la méthode comprend :

(a) la préparation d'un mélange réactionnel en mélangeant un acide nucléique matrice, un triphosphate de désoxyribonucléotide, une ADN polymérase ayant une activité de déplacement de brin, au moins une amorce et une endonucléase qui clive un brin allongé généré à partir de l'amorce, dans laquelle l'amorce est une amorce oligonucléotidique chimère qui est sensiblement complémentaire de la séquence de bases de l'acide nucléique matrice et contient des désoxyribonucléotides et un ou plusieurs ribonucléotide(s), le(s) ribonucléotide(s) étant positionné(s) à l'extrémité 3' ou du côté 3' terminal de l'amorce dans laquelle l'endonucléase clive en un site qui contient le ribonucléotide, et
(b) l'incubation du mélange réactionnel pendant un temps suffisant pour générer un produit de réaction dans des conditions dans lesquelles une hybridation spécifique de l'amorce avec l'acide nucléique matrice, une réaction de synthèse d'un brin allongé et une réaction de déplacement de brin par l'ADN polymérase et une réaction de clivage du brin allongé par l'endonucléase ont lieu.

**2.** Méthode selon la revendication 1, dans laquelle le mélange réactionnel est incubé de manière isotherme.

**3.** Méthode selon la revendication 1 ou 2, dans laquelle le mélange réactionnel contient en outre une amorce oligonucléotidique chimère ayant une séquence sensiblement homologue à la séquence de bases de la matrice.

**4.** Méthode selon la revendication 1, **caractérisée en ce que** la méthode comprend :

(a) le traitement d'un acide nucléique matrice avec au moins une amorce qui est sensiblement complémentaire de la séquence de bases de l'acide nucléique et une ADN polymérase pour synthétiser un brin allongé à partir de l'amorce qui est complémentaire de la matrice, dans laquelle l'amorce est une amorce oligonucléotidique chimère contenant des désoxyribonucléotides et un ou plusieurs ribonucléotide(s), le(s) ribonucléotide(s) étant positionné(s) à l'extrémité 3' ou du côté 3' terminal de l'amorce ; dans laquelle l'endonucléase clive en un site qui contient le ribonucléotide ;
(b) le clivage du brin allongé à partir de l'amorce d'un acide nucléique double brin obtenu à l'étape (a) avec l'endonucléase en un site qui contient le ribonucléotide ; et
(c) l'extension d'une séquence nucléotidique qui est complémentaire de la matrice en utilisant une ADN polymérase ayant une activité de déplacement de brin de l'extrémité 3' de la portion amorce de l'acide nucléique double brin clivé à l'étape (b) pour effectuer un déplacement de brin.

5. Méthode selon la revendication 4, dans laquelle l'étape (b) et l'étape (c) sont séquentiellement répétées.

6. Méthode de la revendication 4, dans laquelle un acide nucléique double brin contenant un brin régénéré allongé à partir d'une amorce est réutilisé à l'étape (b) ; et la méthode comprend en outre les étapes de :

(d) traitement d'un brin déplacé libéré obtenu à l'étape (c) comme matrice avec au moins une amorce qui est différente de celle utilisée à l'étape (a) et une ADN polymérase pour synthétiser un brin allongé à partir d'une amorce qui est complémentaire du brin déplacé, dans laquelle l'amorce qui est différente de celle utilisée à l'étape (a) est une amorce oligonucléotidique chimère qui est sensiblement complémentaire de la séquence de bases du brin déplacé et contient un désoxyribonucléotide et un ribonucléotide, le ribonucléotide étant positionné à l'extrémité 3' ou du côté 3' terminal de l'amorce, dans laquelle l'endonucléase clive en un site qui contient le ribonucléotide ;
(e) clivage du brin allongé à partir d'une amorce d'un acide nucléique double brin obtenu à l'étape (d) avec l'endonucléase en un site qui contient le ribonucléotide ; et
(f) extension d'une séquence d'acide nucléique qui est complémentaire de la matrice utilisant une ADN polymérase ayant une activité de déplacement de brin de l'extrémité 3' de la portion amorce de l'acide nucléique double brin clivé à l'étape (e) pour produire un déplacement de brin, dans laquelle un acide nucléique contenant un brin régénéré allongé à partir d'une amorce est réutilisé à l'étape (e).

7. Méthode selon l'une quelconque des revendications 4 à 6, dans laquelle les différentes étapes sont réalisées de manière isotherme.

8. Méthode selon l'une quelconque des revendications 1 à 7, dans laquelle l'endonucléase est une endoribonucléase.

9. Méthode selon la revendication 8, dans laquelle l'endoribonucléase est la RNase H.

10. Méthode selon l'une quelconque des revendications 1 à 9, dans laquelle l'amorce oligonucléotidique chimère contient deux ou plusieurs résidus successifs de ribonucléotide.

11. Méthode selon l'une quelconque des revendications 1 à 10, dans laquelle l'amorce oligonucléotidique chimère contient un ou plusieurs ribonucléotides modifiés.

12. Méthode selon la revendication 11, dans laquelle l'amorce oligonucléotidique chimère contient un ($\alpha$-S) ribonucléotide dans lequel l'atome d'oxygène lié à l'atome de phosphore en position $\alpha$ du ribonucléotide est remplacé par un atome de soufre.

13. Méthode selon l'une quelconque des revendications 1 à 12, dans laquelle l'acide nucléique utilisé comme matrice est un ADN simple brin ou un ADN double brin.

14. Méthode selon la revendication 13, qui est réalisée après conversion d'un ADN double brin comme matrice en ADN simple brin.

15. Méthode selon la revendication 13 ou 14 dans laquelle l'acide nucléique utilisé comme matrice est un ADNc obtenu à partir d'un ARN par une réaction de transcription inverse.

16. Méthode selon la revendication 15, qui est réalisée après synthèse d'ADNc par une réaction de transcription inverse en utilisant un ARN comme matrice.

**17.** Méthode selon la revendication 16, dans laquelle une amorce choisie dans le groupe constitué d'une amorce oligo-dT, une amorce aléatoire et une amorce spécifique est utilisée comme amorce pour la réaction de transcription inverse.

**18.** Méthode selon la revendication 15 ou 16, dans laquelle une amorce oligonucléotidique chimère est utilisée comme amorce pour la réaction de transcription inverse.

**19.** Méthode selon l'une quelconque des revendications 15 à 18, dans laquelle une ADN polymérase ayant une activité de transcriptase inverse est utilisée comme transcriptase inverse.

**20.** Méthode selon l'une quelconque des revendications 15 à 19, dans laquelle la réaction de transcription inverse et la synthèse du brin allongé qui est complémentaire de la matrice sont réalisées en utilisant une ADN polymérase ayant une activité de transcriptase inverse et une activité de déplacement de brin.

**21.** Méthode selon l'une quelconque des revendications 1 à 19, dans laquelle l'ADN polymérase est choisie dans le groupe constitué du fragment de Klenow de l'ADN polymérase I d'*Escherichia coli,* l'ADN polymérase Bst dépourvue d'exonucléase 5'→3' de Bacillus stearothermophilus et l'ADN polymérase Bca dépourvue d'exonucléase 5'→3' de Bacillus caldotenax.

**22.** Méthode selon la revendication 20, dans laquelle l'ADN polymérase est l'ADN polymérase Bst dépourvue d'exonucléase 5'→3' de Bacillus stearothermophilus ou l'ADN polymérase Bca dépourvue d'exonucléase 5'→3' de Bacillus caldotenax.

**23.** Méthode selon l'une quelconque des revendications 16 à 22, dans laquelle l'ARN matrice dans la réaction de transcription inverse est un ARN amplifié par une réaction d'amplification d'acide nucléique.

**24.** Méthode selon la revendication 23, qui est réalisée après synthèse d'un fragment d'ARN amplifié par une réaction d'amplification d'acide nucléique en utilisant un ARN comme matrice.

**25.** Méthode selon la revendication 23 ou 24, dans laquelle la réaction d'amplification d'acide nucléique est choisie dans le groupe constitué de la méthode du système d'amplification basé sur la transcription (TAS), la méthode de réplication de séquence auto-entretenue (3SR), la méthode d'amplification basée sur la séquence d'acide nucléique (NASBA), la méthode d'amplification médiée par la transcription (TMA) et la méthode de la réplicase Qβ.

**26.** Méthode selon la revendication 13 ou 14, dans laquelle l'acide nucléique en tant que matrice est un ADN obtenu par une réaction d'amplification d'acide nucléique.

**27.** Méthode selon la revendication 26, qui est réalisée après synthèse d'un fragment d'ADN amplifié par une réaction d'amplification d'acide nucléique en utilisant un ADN comme matrice.

**28.** Méthode selon la revendication 27, dans laquelle la réaction d'amplification d'acide nucléique est choisie parmi le groupe constitué de la méthode de réaction de polymérisation en chaîne (PCR), la méthode de réaction de ligature en chaîne (LCR) et la méthode d'amplification de déplacement de brin (SDA).

**29.** Méthode selon l'une quelconque des revendications 23 à 28, dans laquelle une amorce aléatoire ou une amorce dégénérée est utilisée pour la réaction d'amplification d'acide nucléique.

**30.** Méthode selon la revendication 29, dans laquelle l'amorce aléatoire ou l'amorce dégénérée est une amorce ayant une séquence aléatoire ou une séquence dégénérée au moins à l'extrémité 3'-terminale ou du côté 3'-terminal.

**31.** Méthode pour la détection d'un acide nucléique cible dans un échantillon, **caractérisée en ce que** la méthode comprend :

(a) l'amplification d'un acide nucléique cible par la méthode pour amplifier une séquence nucléotidique selon l'une quelconque des revendications 1 à 30 ; et (b) la détection de l'acide nucléique cible amplifié à l'étape (a).

**32.** Méthode selon la revendication 31, dans laquelle l'acide nucléique cible amplifié est détecté en utilisant une sonde ribonucléotidique (ARN) marquée avec deux ou plusieurs substances fluorescentes à une distance qui entraîne un état de quenching.

**33.** Kit pour la méthode selon une quelconque des revendications 1 à 32, qui contient :

(a) une ADN polymérase ayant une activité de déplacement de brin ;
(b) une endonucléase ; et
(c) une amorce oligonucléotidique chimère, qui contient un désoxyribonucléotide et un ribonucléotide, le ribonucléotide étant positionné à l'extrémité 3'.

**34.** Kit selon la revendication 33, qui contient une ADN polymérase choisie dans le groupe constitué du fragment de Klenow de l'ADN polymérase I d'Escherichia coli, l'ADN polymérase Bst dépourvue d'exonucléase 5'→3' de Bacillus stearothermophilus et l'ADN polymérase Bca dépourvue d'exonucléase 5'→3' de Bacillus caldotenax en tant qu'ADN polymérase.

**35.** Kit selon la revendication 33 qui contient la RNAse H comme endonucléase.

**36.** Méthode pour produire un matériel ayant un acide nucléique immobilisé dans laquelle l'acide nucléique est disposé dans une région prédéfinie, **caractérisée en ce que** la méthode comprend :

(a) l'amplification d'un acide nucléique à immobiliser par la méthode pour amplifier une séquence nucléotidique ou pour produire un acide nucléique en grandes quantités selon l'une quelconque des revendications 1 à 32 ; et
(b) la disposition et l'immobilisation de l'acide nucléique amplifié à l'étape (a) dans une région prédéfinie sur un substrat.

**37.** Méthode selon la revendication 36, dans laquelle un acide nucléique simple brin sensiblement dépourvu d'un brin complémentaire de celui-ci est amplifié, et disposé et immobilisé dans la région prédéfinie sur le substrat.

**38.** Méthode pour déterminer une séquence de bases d'un acide nucléique, **caractérisée en ce que** la méthode comprend l'amplification d'une séquence nucléotidique selon la méthode selon l'une quelconque des revendications 1 à 32.

**39.** Méthode pour détecter une mutation d'un acide nucléique cible, **caractérisée en ce que** la méthode comprend l'amplification d'une séquence nucléotidique selon la méthode selon l'une quelconque des revendications 1 à 32.

Fig. 1

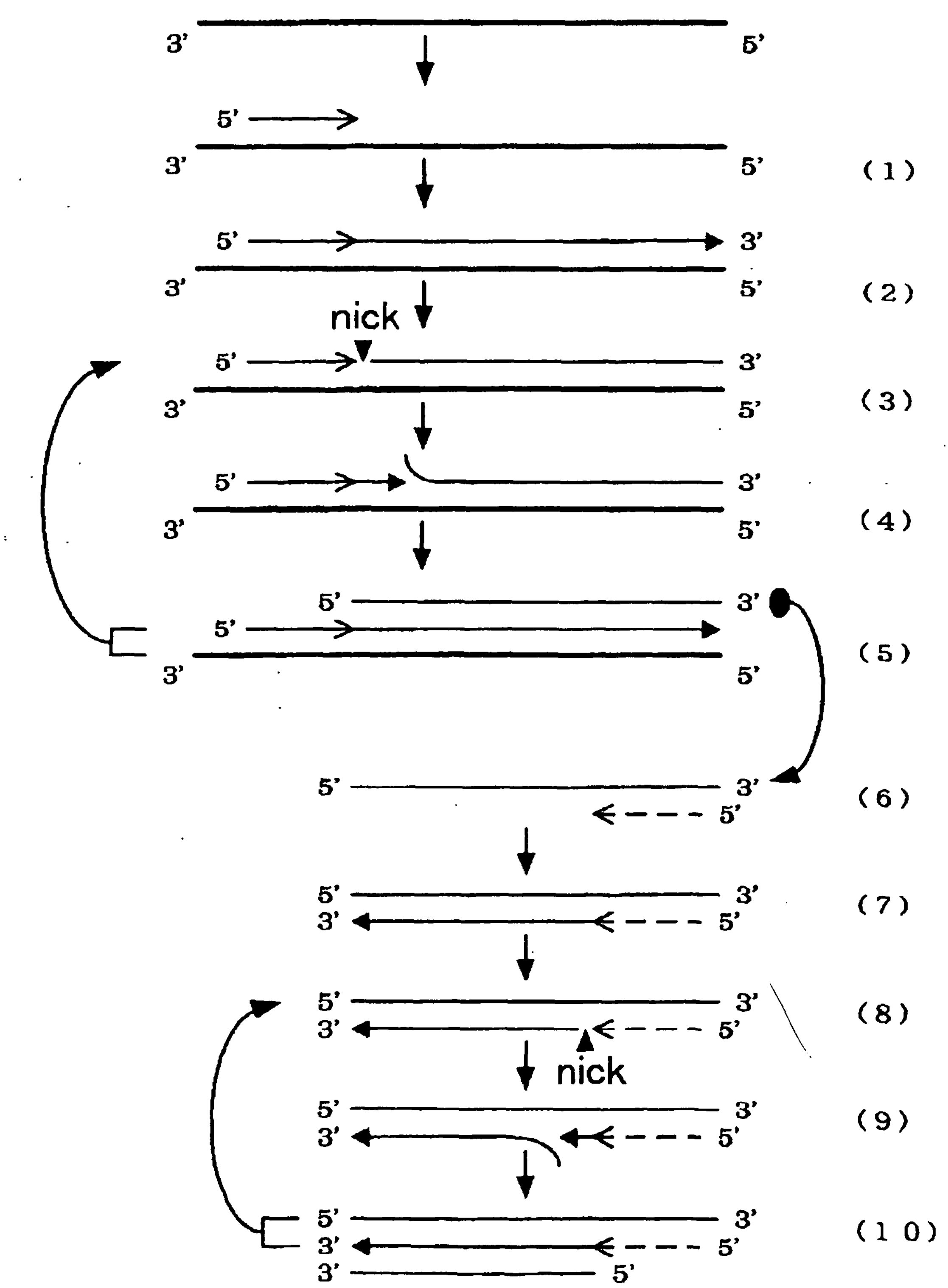
3'
5'
5'
3'
5'
(1)
5'
3'
3'
5'
(2)
nick
5'
3'
3'
5'
(3)
5'
3'
3'
5'
(4)
5'
3'
5'
3'
5'
(5)
5'
3'
5'
(6)
5'
3'
3'
5'
(7)
5'
3'
3'
5'
(8)
nick
5'
3'
3'
5'
(9)
5'
3'
3'
5'
3'
5'
(10)

Fig.2